(19) Europäisches Patentamt
European Patent Office
Office européen des brevets

(11) EP 3 919 514 A1

(12) EUROPEAN PATENT APPLICATION
published in accordance with Art. 153(4) EPC

(43) Date of publication:
**08.12.2021 Bulletin 2021/49**

(21) Application number: **20738344.9**

(22) Date of filing: **10.01.2020**

(51) Int Cl.:
***C07K 16/28*** *(2006.01)* ***C12N 15/13*** *(2006.01)*
***A61K 39/395*** *(2006.01)* ***A61P 35/00*** *(2006.01)*

(86) International application number:
**PCT/CN2020/071353**

(87) International publication number:
**WO 2020/143749 (16.07.2020 Gazette 2020/29)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **10.01.2019 CN 201910022548**

(71) Applicant: **Mabwell (Shanghai) Bioscience Co., Ltd.**
**Shanghai 215400 (CN)**

(72) Inventors:
- **ZHANG, Jinchao**
**Beijing 100096 (CN)**
- **WANG, Rongjuan**
**Beijing 100096 (CN)**
- **JIAO, Shasha**
**Beijing 100096 (CN)**
- **WANG, Shuang**
**Beijing 100096 (CN)**

(74) Representative: **Schiweck Weinzierl Koch Patentanwälte Partnerschaft mbB**
**Ganghoferstraße 68 B**
**80339 München (DE)**

# (54) RECOMBINANT ANTI-HUMAN PD-1 ANTIBODY AND APPLICATION THEREOF

(57) The present invention provides an antibody binding to programmed cell death protein 1 (PD-1) or a fragment thereof and a use of the antibody or a fragment thereof in preventing or treating a tumor or cancer. The antibody or a fragment thereof of the present invention can effectively block the interactions of PD-1/PD-L1 and PD-1/PD-L2 and has a unique antigen binding epitope and unique properties in terms of efficacy and safety.

EP 3 919 514 A1

Sequence Listing

<110> MABWELL (SHANGHAI) BIOSCIENCE CO., LTD.

<120> RECOMBINANT ANTI-HUMAN PD-1 ANTIBODY AND APPLICATION THEREOF

<130> LC19210015P

<150> CN201910022548.9
<151> 2019-01-10

<160> 100

<170> PatentIn version 3.3

<210> 1
<211> 11
<212> PRT
<213> Artificial

<220>
<223> LCDR1

<220>
<221> MISC_FEATURE
<222> (1)..(1)
<223> X= K or R

<220>
<221> MISC_FEATURE
<222> (7)..(7)
<223> X= E or S

<220>
<221> MISC_FEATURE
<222> (9)..(9)
<223> X= A, V or Y

<400> 1

Xaa Ala Ser Gln Asp Val Xaa Thr Xaa Val Ala
1               5                   10

<210> 2
<211> 7
<212> PRT
<213> Artificial

<220>
<223> LCDR2

<220>
<221> MISC_FEATURE
<222> (1)..(1)
<223> X= A, G or W

<220>
<221> MISC_FEATURE
<222> (6)..(6)
<223> X= A, H or Q

<220>
<221> MISC_FEATURE
<222> (7)..(7)
<223> X= S or T

<400> 2

Xaa Ala Ser Thr Arg Xaa Xaa
1 5

<210> 3
<211> 8
<212> PRT
<213> Artificial

<220>
<223> LCDR3

<220>
<221> MISC_FEATURE
<222> (3)..(3)
<223> X= A, F or Y

<220>
<221> MISC_FEATURE
<222> (4)..(4)
<223> X= D, N or S

<220>
<221> MISC_FEATURE
<222> (5)..(5)
<223> X= R, N or S

<220>
<221> MISC_FEATURE
<222> (6)..(6)
<223> X= F or Y

<220>
<221> MISC_FEATURE
<222> (8)..(8)
<223> X= G, W or Y

<400> 3

Gln Gln Xaa Xaa Xaa Xaa Pro Xaa
1 5

<210> 4
<211> 5
<212> PRT
<213> Artificial

<220>
<223> HCDR1

<220>
<221> MISC_FEATURE

```
<222>  (1)..(1)
<223>  X= D  or  S

<220>
<221>  MISC_FEATURE
<222>  (2)..(2)
<223>  X= A, N  or  Y

<220>
<221>  MISC_FEATURE
<222>  (3)..(3)
<223>  X= D, S  or  Y

<400>  4

Xaa Xaa Xaa Met Ser
1               5


<210>  5
<211>  17
<212>  PRT
<213>  Artificial

<220>
<223>  HCDR2


<220>
<221>  MISC_FEATURE
<222>  (3)..(3)
<223>  X= K, S  or  W

<220>
<221>  MISC_FEATURE
<222>  (4)..(4)
<223>  X= G, S  or  Y

<220>
<221>  MISC_FEATURE
<222>  (5)..(5)
<223>  X= D, G  or  S

<220>
<221>  MISC_FEATURE
<222>  (6)..(6)
<223>  X= G  or  S

<220>
<221>  MISC_FEATURE
<222>  (7)..(7)
<223>  X= G  or  S

<220>
<221>  MISC_FEATURE
<222>  (8)..(8)
<223>  X= T  or  Y

<220>
<221>  MISC_FEATURE
<222>  (10)..(10)
<223>  X= A, T  or  Y
```

<400>  5

Thr Ile Xaa Xaa Xaa Xaa Xaa Xaa Thr Xaa Tyr Pro Asp Ser Val Lys
1               5                   10                  15

Gly

<210>  6
<211>  8
<212>  PRT
<213>  Artificial

<220>
<223>  HCDR3

<220>
<221>  MISC_FEATURE
<222>  (2)..(2)
<223>  X= D, E  or  Y

<220>
<221>  MISC_FEATURE
<222>  (3)..(3)
<223>  X= A  or  S

<220>
<221>  MISC_FEATURE
<222>  (8)..(8)
<223>  X= T  or  Y

<400>  6

Pro Xaa Xaa Ser Gly Val Ala Xaa
1               5

<210>  7
<211>  11
<212>  PRT
<213>  Artificial

<220>
<223>  LCDR1

<400>  7

Lys Ala Ser Gln Asp Val Glu Thr Val Val Ala
1               5                   10

<210>  8
<211>  11
<212>  PRT
<213>  Artificial

<220>
<223>  LCDR1

<400>  8

Arg Ala Ser Gln Asp Val Glu Thr Val Val Ala
1 5 10

<210> 9
<211> 11
<212> PRT
<213> Artificial

<220>
<223> LCDR1

<400> 9

Lys Ala Ser Gln Asp Val Ser Thr Val Val Ala
1 5 10

<210> 10
<211> 11
<212> PRT
<213> Artificial

<220>
<223> LCDR1

<400> 10

Lys Ala Ser Gln Asp Val Glu Thr Ala Val Ala
1 5 10

<210> 11
<211> 11
<212> PRT
<213> Artificial

<220>
<223> LCDR1

<400> 11

Lys Ala Ser Gln Asp Val Glu Thr Tyr Val Ala
1 5 10

<210> 12
<211> 7
<212> PRT
<213> Artificial

<220>
<223> LCDR2

<400> 12

Trp Ala Ser Thr Arg His Thr
1 5

<210> 13
<211> 7

<212> PRT
<213> Artificial

<220>
<223> LCDR2

<400> 13

Ala Ala Ser Thr Arg His Thr
1 5

<210> 14
<211> 7
<212> PRT
<213> Artificial

<220>
<223> LCDR2

<400> 14

Gly Ala Ser Thr Arg His Thr
1 5

<210> 15
<211> 7
<212> PRT
<213> Artificial

<220>
<223> LCDR2

<400> 15

Trp Ala Ser Thr Arg Ala Thr
1 5

<210> 16
<211> 7
<212> PRT
<213> Artificial

<220>
<223> LCDR2

<400> 16

Trp Ala Ser Thr Arg Gln Thr
1 5

<210> 17
<211> 7
<212> PRT
<213> Artificial

<220>
<223> LCDR2

<400> 17

Trp Ala Ser Thr Arg His Ser
1               5

<210> 18
<211> 8
<212> PRT
<213> Artificial

<220>
<223> LCDR3

<400> 18

Gln Gln Tyr Ser Arg Tyr Pro Trp
1               5

<210> 19
<211> 8
<212> PRT
<213> Artificial

<220>
<223> LCDR3

<400> 19

Gln Gln Ala Ser Arg Tyr Pro Trp
1               5

<210> 20
<211> 8
<212> PRT
<213> Artificial

<220>
<223> LCDR3

<400> 20

Gln Gln Phe Ser Arg Tyr Pro Trp
1                   5

<210> 21
<211> 8
<212> PRT
<213> Artificial

<220>
<223> LCDR3

<400> 21

Gln Gln Tyr Asp Arg Tyr Pro Trp
1               5

<210> 22
<211> 8

<212> PRT
<213> Artificial

<220>
<223> LCDR3

<400> 22

Gln Gln Tyr Asn Arg Tyr Pro Trp
1 5

<210> 23
<211> 8
<212> PRT
<213> Artificial

<220>
<223> LCDR3

<400> 23

Gln Gln Tyr Ser Asn Tyr Pro Trp
1 5

<210> 24
<211> 8
<212> PRT
<213> Artificial

<220>
<223> LCDR3

<400> 24

Gln Gln Tyr Ser Ser Tyr Pro Trp
1 5

<210> 25
<211> 8
<212> PRT
<213> Artificial

<220>
<223> LCDR3

<400> 25

Gln Gln Tyr Ser Arg Phe Pro Trp
1 5

<210> 26
<211> 8
<212> PRT
<213> Artificial

<220>
<223> LCDR3

<400> 26

Gln Gln Tyr Ser Arg Tyr Pro Gly
1               5

<210> 27
<211> 8
<212> PRT
<213> Artificial

<220>
<223> LCDR3

<400> 27

Gln Gln Tyr Ser Arg Tyr Pro Tyr
1               5

<210> 28
<211> 5
<212> PRT
<213> Artificial

<220>
<223> HCDR1

<400> 28

Ser Tyr Asp Met Ser
1               5

<210> 29
<211> 5
<212> PRT
<213> Artificial

<220>
<223> HCDR1

<400> 29

Asp Tyr Asp Met Ser
1               5

<210> 30
<211> 5
<212> PRT
<213> Artificial

<220>
<223> HCDR1

<400> 30

Ser Ala Asp Met Ser
1               5

<210> 31
<211> 5

<212> PRT
<213> Artificial

<220>
<223> HCDR1

<400> 31

Ser Asn Asp Met Ser
1               5

<210> 32
<211> 5
<212> PRT
<213> Artificial

<220>
<223> HCDR1

<400> 32

Ser Tyr Ser Met Ser
1               5

<210> 33
<211> 5
<212> PRT
<213> Artificial

<220>
<223> HCDR1

<400> 33

Ser Tyr Tyr Met Ser
1               5

<210> 34
<211> 17
<212> PRT
<213> Artificial

<220>
<223> HCDR2

<400> 34

Thr Ile Ser Gly Gly Gly Ser Tyr Thr Tyr Tyr Pro Asp Ser Val Lys
1               5                   10                  15

Gly

<210> 35
<211> 17
<212> PRT
<213> Artificial

<220>
<223> HCDR2

<400> 35

Thr Ile Lys Gly Gly Gly Ser Tyr Thr Tyr Tyr Pro Asp Ser Val Lys
1 5 10 15

Gly

<210> 36
<211> 17
<212> PRT
<213> Artificial

<220>
<223> HCDR2

<400> 36

Thr Ile Trp Gly Gly Gly Ser Tyr Thr Tyr Tyr Pro Asp Ser Val Lys
1 5 10 15

Gly

<210> 37
<211> 17
<212> PRT
<213> Artificial

<220>
<223> HCDR2

<400> 37

Thr Ile Ser Ser Gly Gly Ser Tyr Thr Tyr Tyr Pro Asp Ser Val Lys
1 5 10 15

Gly

<210> 38
<211> 17
<212> PRT
<213> Artificial

<220>
<223> HCDR2

<400> 38

Thr Ile Ser Tyr Gly Gly Ser Tyr Thr Tyr Tyr Pro Asp Ser Val Lys
1 5 10 15

Gly

<210> 39
<211> 17
<212> PRT
<213> Artificial

<220>
<223> HCDR2

<400> 39

Thr Ile Ser Gly Asp Gly Ser Tyr Thr Tyr Tyr Pro Asp Ser Val Lys
1 5 10 15

Gly

<210> 40
<211> 17
<212> PRT
<213> Artificial

<220>
<223> HCDR2

<400> 40

Thr Ile Ser Gly Ser Gly Ser Tyr Thr Tyr Tyr Pro Asp Ser Val Lys
1 5 10 15

Gly

<210> 41
<211> 17
<212> PRT
<213> Artificial

<220>
<223> HCDR2

<400> 41

Thr Ile Ser Gly Gly Ser Ser Tyr Thr Tyr Tyr Pro Asp Ser Val Lys
1 5 10 15

Gly

<210> 42
<211> 17
<212> PRT
<213> Artificial

<220>
<223>  HCDR2

<400>  42

Thr Ile Ser Gly Gly Gly Gly Tyr Thr Tyr Tyr Pro Asp Ser Val Lys
1               5                   10                  15

Gly

<210>  43
<211>  17
<212>  PRT
<213>  Artificial

<220>
<223>  HCDR2

<400>  43

Thr Ile Ser Gly Gly Gly Ser Thr Thr Tyr Tyr Pro Asp Ser Val Lys
1               5                   10                  15

Gly

<210>  44
<211>  17
<212>  PRT
<213>  Artificial

<220>
<223>  HCDR2

<400>  44

Thr Ile Ser Gly Gly Gly Ser Tyr Thr Ala Tyr Pro Asp Ser Val Lys
1               5                   10                  15

Gly

<210>  45
<211>  17
<212>  PRT
<213>  Artificial

<220>
<223>  HCDR2

<400>  45

Thr Ile Ser Gly Gly Gly Ser Tyr Thr Thr Tyr Pro Asp Ser Val Lys
1               5                   10                  15

Gly

<210> 46
<211> 17
<212> PRT
<213> Artificial

<220>
<223> HCDR2

<400> 46

Thr Ile Ser Gly Ser Gly Ser Tyr Thr Ala Tyr Pro Asp Ser Val Lys
1 5 10 15

Gly

<210> 47
<211> 8
<212> PRT
<213> Artificial

<220>
<223> HCDR3

<400> 47

Pro Asp Ser Ser Gly Val Ala Tyr
1 5

<210> 48
<211> 8
<212> PRT
<213> Artificial

<220>
<223> HCDR3

<400> 48

Pro Glu Ser Ser Gly Val Ala Tyr
1 5

<210> 49
<211> 8
<212> PRT
<213> Artificial

<220>
<223> HCDR3

<400> 49

Pro Tyr Ser Ser Gly Val Ala Tyr
1 5

<210> 50
<211> 8
<212> PRT
<213> Artificial

<220>
<223> HCDR3

<400> 50

Pro Asp Ala Ser Gly Val Ala Tyr
1 5

<210> 51
<211> 8
<212> PRT
<213> Artificial

<220>
<223> HCDR3

<400> 51

Pro Asp Ser Ser Gly Val Ala Thr
1 5

<210> 52
<211> 104
<212> PRT
<213> Artificial

<220>
<223> VL

<400> 52

Asp Ile Val Met Thr Gln Ser His Lys Phe Met Ser Thr Ser Val Gly
1 5 10 15

Asp Arg Val Ser Ile Thr Cys Lys Ala Ser Gln Asp Val Glu Thr Val
20 25 30

Val Ala Trp Tyr Gln Gln Lys Pro Gly Gln Ser Pro Lys Leu Leu Ile
35 40 45

Tyr Trp Ala Ser Thr Arg His Thr Gly Val Pro Asp Arg Phe Thr Gly
50 55 60

Ser Gly Ser Gly Thr Asp Phe Thr Leu Thr Ile Ser Asn Val Gln Ser
65 70 75 80

Glu Asp Leu Ala Asp Tyr Phe Cys Gln Gln Tyr Ser Arg Tyr Pro Trp
85 90 95

Thr Phe Gly Gly Gly Thr Lys Leu
100

<210> 53
<211> 312
<212> DNA
<213> Artificial

<220>
<223> VL

<400> 53
gacattgtga tgacccagtc tcacaaattc atgtccacat cagtaggaga cagggtcagc 60

atcacctgca aggccagtca ggatgtggaa actgttgtag cctggtatca acagaaacca 120

gggcaatctc ctaaactact gatttactgg gcatccaccc ggcacactgg agtccctgat 180

cgcttcacag gcagtggatc tgggacagat ttcactctca ccattagcaa tgtgcagtct 240

gaagacttgg cagattattt ctgtcagcag tatagcaggt atccgtggac gttcggtgga 300

ggcaccaagc tg 312

<210> 54
<211> 117
<212> PRT
<213> Artificial

<220>
<223> VH

<400> 54

Glu Val Lys Leu Val Glu Ser Gly Gly Gly Leu Met Lys Pro Gly Gly
1 5 10 15

Ser Leu Lys Leu Ser Cys Ala Ala Ser Gly Phe Thr Phe Ser Ser Tyr
20 25 30

Asp Met Ser Trp Val Arg Gln Thr Pro Glu Lys Arg Leu Glu Trp Val
35 40 45

Ala Thr Ile Ser Gly Gly Gly Ser Tyr Thr Tyr Tyr Pro Asp Ser Val
50 55 60

Lys Gly Arg Phe Thr Ile Ser Arg Asp Asn Ala Lys Asn Asn Leu Tyr
65 70 75 80

Leu Gln Met Ser Ser Leu Arg Ser Glu Asp Thr Ala Leu Tyr Tyr Cys
85 90 95

Ala Ser Pro Asp Ser Ser Gly Val Ala Tyr Trp Gly Gln Gly Thr Leu
100 105 110

```
Val Thr Val Ser Ala
        115


<210>  55
<211>  351
<212>  DNA
<213>  Artificial

<220>
<223>  VH

<400>  55
gaagtgaagc tggtggagtc tgggggaggc ttaatgaagc ctggagggtc cctgaaactc      60

tcctgtgcag cctctggatt cactttcagt agttatgaca tgtcttgggt tcgccagact     120

ccggagaaga ggctggagtg ggtcgcaacc attagtggtg gtggaagtta cacctactat     180

ccagacagtg tgaaggggcg attcaccatc tccagagaca atgccaagaa caacctgtat     240

ctgcaaatga gcagtctgag gtctgaggac acggccttgt attactgtgc aagtccagac     300

agctcgggcg ttgcttactg gggccaaggg actctggtca ctgtctctgc a              351


<210>  56
<211>  110
<212>  PRT
<213>  Artificial

<220>
<223>  CL

<400>  56

Glu Ile Lys Arg Ala Asp Ala Ala Pro Thr Val Ser Ile Phe Pro Pro
1               5                   10                  15


Ser Ser Glu Gln Leu Thr Ser Gly Gly Ala Ser Val Val Cys Phe Leu
            20                  25                  30


Asn Asn Phe Tyr Pro Lys Asp Ile Asn Val Lys Trp Lys Ile Asp Gly
        35                  40                  45


Ser Glu Arg Gln Asn Gly Val Leu Asn Ser Trp Thr Asp Gln Asp Ser
    50                  55                  60


Lys Asp Ser Thr Tyr Ser Met Ser Ser Thr Leu Thr Leu Thr Lys Asp
65                  70                  75                  80


Glu Tyr Glu Arg His Asn Ser Tyr Thr Cys Glu Ala Thr His Lys Thr
                85                  90                  95


Ser Thr Ser Pro Ile Val Lys Ser Phe Asn Arg Asn Glu Cys
            100                 105                 110
```

<210> 57
<211> 330
<212> DNA
<213> Artificial

<220>
<223> CL

<400> 57
gaaatcaaac gggctgatgc tgcaccaact gtatccatct tcccaccatc cagtgagcag 60

ttaacatctg gaggtgcctc agtcgtgtgc ttcttgaaca acttctaccc caaagacatc 120

aatgtcaagt ggaagattga tggcagtgaa cgacaaaatg gcgtcctgaa cagttggact 180

gatcaggaca gcaaagacag cacctacagc atgagcagca ccctcacgtt gaccaaggac 240

gagtatgaac gacataacag ctatacctgt gaggccactc acaagacatc aacttcaccc 300

attgtcaaga gcttcaacag gaatgagtgt 330

<210> 58
<211> 102
<212> PRT
<213> Artificial

<220>
<223> CH

<400> 58

Ala Lys Thr Thr Pro Pro Ser Val Tyr Pro Leu Ala Pro Gly Ser Ala
1 5 10 15

Ala Gln Thr Asn Ser Met Val Thr Leu Gly Cys Leu Val Lys Gly Tyr
20 25 30

Phe Pro Glu Pro Val Thr Val Thr Trp Asn Ser Gly Ser Leu Ser Ser
35 40 45

Gly Val His Thr Phe Pro Ala Val Leu Gln Ser Asp Leu Tyr Thr Leu
50 55 60

Ser Ser Ser Val Thr Val Pro Ser Ser Thr Trp Pro Ser Glu Thr Val
65 70 75 80

Thr Cys Asn Val Ala His Pro Ala Ser Ser Thr Lys Val Asp Lys Lys
85 90 95

Ile Val Pro Arg Asp Cys
100

<210> 59

<211> 306
<212> DNA
<213> Artificial

<220>
<223> CH

<400> 59
gccaaaacga cacccccatc tgtctatcca ctggcccctg gatctgctgc ccaaactaac 60

tccatggtga ccctgggatg cctggtcaag ggctatttcc ctgagccagt gacagtgacc 120

tggaactctg gatccctgtc cagcggtgtg cacaccttcc cagctgtcct gcagtctgac 180

ctctacactc tgagcagctc agtgactgtc ccctccagca cctggcccag cgagaccgtc 240

acctgcaacg ttgcccaccc ggccagcagc accaaggtgg acaagaaaat tgtgcccagg 300

gattgt 306

<210> 60
<211> 104
<212> PRT
<213> Artificial

<220>
<223> VL

<400> 60

Asp Ile Val Met Thr Gln Ser Pro Leu Ser Leu Pro Val Thr Pro Gly
1 5 10 15

Glu Pro Ala Ser Ile Thr Cys Lys Ala Ser Gln Asp Val Glu Thr Val
20 25 30

Val Ala Trp Tyr Leu Gln Lys Pro Gly Gln Ser Pro Arg Leu Leu Ile
35 40 45

Tyr Trp Ala Ser Thr Arg His Thr Gly Val Pro Asp Arg Phe Ser Gly
50 55 60

Ser Gly Ser Gly Thr Asp Phe Thr Leu Lys Ile Ser Arg Val Glu Ala
65 70 75 80

Glu Asp Val Gly Val Tyr Tyr Cys Gln Gln Tyr Ser Arg Tyr Pro Trp
85 90 95

Thr Phe Gly Gln Gly Thr Lys Leu
100

<210> 61
<211> 312
<212> DNA
<213> Artificial

<220>
<223> VL

<400> 61
gacatcgtga tgacccagtc tcctctgtcc ctgcccgtga cacctggtga gcctgcctcc 60

atcacctgca aggcctccca ggacgtggag accgtggtgg cctggtacct gcagaagcct 120

ggccagagtc ccagactgct gatctactgg gcttccacca gacacaccgg cgtgcctgac 180

agattctccg gctctggctc cggcaccgac ttcaccctga agatctcccg ggtggaggcc 240

gaggacgtgg gcgtgtacta ctgccagcag tactccagat acccctggac cttcggccag 300

ggcaccaagc tg 312

<210> 62
<211> 117
<212> PRT
<213> Artificial

<220>
<223> VH

<400> 62

Glu Val Gln Leu Val Glu Ser Gly Gly Gly Leu Val Lys Pro Gly Gly
1 5 10 15

Ser Leu Arg Leu Ser Cys Ala Ala Ser Gly Phe Thr Phe Ser Ser Tyr
20 25 30

Asp Met Ser Trp Val Arg Gln Ala Pro Gly Lys Gly Leu Glu Trp Val
35 40 45

Ala Thr Ile Ser Gly Gly Gly Ser Tyr Thr Tyr Tyr Pro Asp Ser Val
50 55 60

Lys Gly Arg Phe Thr Ile Ser Arg Asp Asn Ala Lys Asn Ser Leu Tyr
65 70 75 80

Leu Gln Met Asn Ser Leu Arg Ala Glu Asp Thr Ala Val Tyr Tyr Cys
85 90 95

Ala Ser Pro Asp Ser Ser Gly Val Ala Tyr Trp Gly Gln Gly Thr Leu
100 105 110

Val Thr Val Ser Ser
115

<210> 63
<211> 351
<212> DNA

<213> Artificial

<220>
<223> VH

<400> 63
gaggtgcagc tggtggagtc tggcggaggc ctggtgaagc ctggcggttc cctgagactg 60

tcctgcgctg cctctggctt caccttctcc tcctacgaca tgtcctgggt gagacaggct 120

cccggaaagg gactggagtg ggtggccacc atctctggcg gaggctccta cacctactac 180

cctgactccg tgaagggccg gttcaccatc tccagagaca acgccaagaa ctccctgtac 240

ctgcagatga actccctgag agccgaggac accgccgtgt actactgcgc ctctcccgac 300

tcttccggag tggcctactg gggccaggga accctggtga ccgtgtcttc c 351

<210> 64
<211> 110
<212> PRT
<213> Artificial

<220>
<223> CL

<400> 64

Glu Ile Lys Arg Thr Val Ala Ala Pro Ser Val Phe Ile Phe Pro Pro
1 5 10 15

Ser Asp Glu Gln Leu Lys Ser Gly Thr Ala Ser Val Val Cys Leu Leu
20 25 30

Asn Asn Phe Tyr Pro Arg Glu Ala Lys Val Gln Trp Lys Val Asp Asn
35 40 45

Ala Leu Gln Ser Gly Asn Ser Gln Glu Ser Val Thr Glu Gln Asp Ser
50 55 60

Lys Asp Ser Thr Tyr Ser Leu Ser Ser Thr Leu Thr Leu Ser Lys Ala
65 70 75 80

Asp Tyr Glu Lys His Lys Val Tyr Ala Cys Glu Val Thr His Gln Gly
85 90 95

Leu Ser Ser Pro Val Thr Lys Ser Phe Asn Arg Gly Glu Cys
100 105 110

<210> 65
<211> 330
<212> DNA
<213> Artificial

<220>

<223> CL

<400> 65
gagatcaagc ggaccgtggc ggcgccatct gtcttcatct tcccgccatc tgatgagcag 60

ttgaaatctg gtaccgctag cgttgtgtgc ctgctgaata acttctatcc cagagaggcc 120

aaagtacagt ggaaggtgga taacgccctc caatcgggta actcccagga gagtgtcaca 180

gagcaggaca gcaaggacag cacctacagc ctcagcagca ccctgacgct gagcaaagca 240

gactacgaga aacacaaagt ctacgcctgc gaagtcaccc atcagggcct gagctcgccc 300

gtcacaaaga gcttcaacag gggagagtgt 330

<210> 66
<211> 327
<212> PRT
<213> Artificial

<220>
<223> CH

<400> 66

Ala Ser Thr Lys Gly Pro Ser Val Phe Pro Leu Ala Pro Cys Ser Arg
1 5 10 15

Ser Thr Ser Glu Ser Thr Ala Ala Leu Gly Cys Leu Val Lys Asp Tyr
20 25 30

Phe Pro Glu Pro Val Thr Val Ser Trp Asn Ser Gly Ala Leu Thr Ser
35 40 45

Gly Val His Thr Phe Pro Ala Val Leu Gln Ser Ser Gly Leu Tyr Ser
50 55 60

Leu Ser Ser Val Val Thr Val Pro Ser Ser Ser Leu Gly Thr Lys Thr
65 70 75 80

Tyr Thr Cys Asn Val Asp His Lys Pro Ser Asn Thr Lys Val Asp Lys
85 90 95

Arg Val Glu Ser Lys Tyr Gly Pro Pro Cys Pro Pro Cys Pro Ala Pro
100 105 110

Glu Phe Leu Gly Gly Pro Ser Val Phe Leu Phe Pro Pro Lys Pro Lys
115 120 125

Asp Thr Leu Met Ile Ser Arg Thr Pro Glu Val Thr Cys Val Val Val
130 135 140

Asp Val Ser Gln Glu Asp Pro Glu Val Gln Phe Asn Trp Tyr Val Asp

145 150 155 160

Gly Val Glu Val His Asn Ala Lys Thr Lys Pro Arg Glu Glu Gln Phe
165 170 175

Asn Ser Thr Tyr Arg Val Val Ser Val Leu Thr Val Leu His Gln Asp
180 185 190

Trp Leu Asn Gly Lys Glu Tyr Lys Cys Lys Val Ser Asn Lys Gly Leu
195 200 205

Pro Ser Ser Ile Glu Lys Thr Ile Ser Lys Ala Lys Gly Gln Pro Arg
210 215 220

Glu Pro Gln Val Tyr Thr Leu Pro Pro Ser Gln Glu Glu Met Thr Lys
225 230 235 240

Asn Gln Val Ser Leu Thr Cys Leu Val Lys Gly Phe Tyr Pro Ser Asp
245 250 255

Ile Ala Val Glu Trp Glu Ser Asn Gly Gln Pro Glu Asn Asn Tyr Lys
260 265 270

Thr Thr Pro Pro Val Leu Asp Ser Asp Gly Ser Phe Phe Leu Tyr Ser
275 280 285

Arg Leu Thr Val Asp Lys Ser Arg Trp Gln Glu Gly Asn Val Phe Ser
290 295 300

Cys Ser Val Met His Glu Ala Leu His Asn His Tyr Thr Gln Lys Ser
305 310 315 320

Leu Ser Leu Ser Leu Gly Lys
325

<210> 67
<211> 981
<212> DNA
<213> Artificial

<220>
<223> CH

<400> 67
gctagcacca agggcccatc cgtcttcccc ctggcgccct gctccaggag cacctccgag 60

agcacagccg ccctgggctg cctggtcaag gactacttcc ccgaaccggt gacggtgtcg 120

tggaactcag gcgccctgac cagcggcgtg cacaccttcc cggctgtcct acagtcctca 180

ggactctact ccctcagcag cgtggtgacc gtgccctcca gcagcttggg cacgaagacc 240

tacacctgca acgtagatca caagcccagc aacaccaagg tggacaagag agttgagtcc 300

aaatatggtc ccccatgccc accatgccca gcacctgagt tcctgggggg accatcagtc 360

ttcctgttcc ccccaaaacc caaggacact ctcatgatct cccggacccc tgaggtcacg 420

tgcgtggtgg tggacgtgag ccaggaagac cccgaggtcc agttcaactg gtacgtggat 480

ggcgtggagg tgcataatgc caagacaaag ccgcgggagg agcagttcaa cagcacgtac 540

cgtgtggtca gcgtcctcac cgtcctgcac caggactggc tgaacggcaa ggagtacaag 600

tgcaaggtct ccaacaaagg cctcccgtcc tccatcgaga aaaccatctc caaagccaaa 660

gggcagcccc gagagccaca ggtgtacacc ctgcccccat cccaggagga gatgaccaag 720

aaccaggtca gcctgacctg cctggtcaaa ggcttctacc ccagcgacat cgccgtggag 780

tgggagagca atgggcagcc ggagaacaac tacaagacca cgcctcccgt gctggactcc 840

gacggctcct tcttcctcta cagcaggcta accgtggaca agagcaggtg gcaggagggg 900

aatgtcttct catgctccgt gatgcatgag gctctgcaca accactacac acagaagagc 960

ctctccctgt ctctgggtaa a 981

<210> 68
<211> 30
<212> DNA
<213> Artificial

<220>
<223> Primer

<400> 68
atgaagttgc ctgttaggct gttggtgctg 30

<210> 69
<211> 30
<212> DNA
<213> Artificial

<220>
<223> Primer

<400> 69
atggagwcag acacactcct gytatgggtg 30

<210> 70
<211> 30
<212> DNA
<213> Artificial

<220>
<223> Primer

<400> 70
atgagtgtgc tcactcaggt cctggsgttg 30

<210> 71
<211> 33
<212> DNA
<213> Artificial

<220>
<223> Primer

<400> 71
atgaggrccc ctgctcagwt tyttggmwtc ttg 33

<210> 72
<211> 30
<212> DNA
<213> Artificial

<220>
<223> Primer

<400> 72
atggatttwc aggtgcagat twtcagcttc 30

<210> 73
<211> 27
<212> DNA
<213> Artificial

<220>
<223> Primer

<400> 73
atgaggtkcy ytgytsagyt yctgrgg 27

<210> 74
<211> 31
<212> DNA
<213> Artificial

<220>
<223> Primer

<400> 74
atgggcwtca agatggagtc acakwyycwg g 31

<210> 75
<211> 31
<212> DNA
<213> Artificial

<220>
<223> Primer

<400> 75
atgtggggay ctktttycmm tttttcaatt g 31

<210> 76
<211> 25

<212>  DNA
<213>  Artificial

<220>
<223>  Primer

<400>  76
atggtrtccw casctcagtt ccttg                                             25

<210>  77
<211>  27
<212>  DNA
<213>  Artificial

<220>
<223>  Primer

<400>  77
atgtatatat gtttgttgtc tatttct                                           27

<210>  78
<211>  28
<212>  DNA
<213>  Artificial

<220>
<223>  Primer

<400>  78
atggaagccc cagctcagct tctcttcc                                          28

<210>  79
<211>  32
<212>  DNA
<213>  Artificial

<220>
<223>  Primer

<400>  79
tcatcaacac tcattcctgt tgaagctctt ga                                     32

<210>  80
<211>  27
<212>  DNA
<213>  Artificial

<220>
<223>  Primer

<400>  80
atgaaatgca gctggggcat sttcttc                                           27

<210>  81
<211>  26
<212>  DNA
<213>  Artificial

<220>
<223>  Primer

<400>  81
atgggatgga gctrtatcat sytctt                                           26

<210>  82
<211>  27
<212>  DNA
<213>  Artificial

<220>
<223>  Primer

<400>  82
atgaagwtgt ggttaaactg ggttttt                                          27

<210>  83
<211>  25
<212>  DNA
<213>  Artificial

<220>
<223>  Primer

<400>  83
atgractttg ggytcagctt grttt                                            25

<210>  84
<211>  30
<212>  DNA
<213>  Artificial

<220>
<223>  Primer

<400>  84
atggactcca ggctcaattt agttttcctt                                       30

<210>  85
<211>  28
<212>  DNA
<213>  Artificial

<220>
<223>  Primer

<400>  85
atggcttgtc ytrgsgctrc tcttctgc                                         28

<210>  86
<211>  26
<212>  DNA
<213>  Artificial

<220>
<223>  Primer

<400> 86
atggratgga gckggrtctt tmtctt 26

<210> 87
<211> 23
<212> DNA
<213> Artificial

<220>
<223> Primer

<400> 87
atgagagtgc tgattctttt gtg 23

<210> 88
<211> 30
<212> DNA
<213> Artificial

<220>
<223> Primer

<400> 88
atggmttggg tgtggamctt gctattcctg 30

<210> 89
<211> 27
<212> DNA
<213> Artificial

<220>
<223> Primer

<400> 89
atgggcagac ttacattctc attcctg 27

<210> 90
<211> 28
<212> DNA
<213> Artificial

<220>
<223> Primer

<400> 90
atggattttg ggctgatttt ttttattg 28

<210> 91
<211> 27
<212> DNA
<213> Artificial

<220>
<223> Primer

<400> 91
atgatggtgt taagtcttct gtacctg 27

<210> 92
<211> 34
<212> DNA
<213> Artificial

<220>
<223> Primer

<400> 92
ctaacaatcc ctgggcacaa ttttcttgtc cacc 34

<210> 93
<211> 25
<212> DNA
<213> Artificial

<220>
<223> Primer

<400> 93
ctcaagctta attgccgcca ccatg 25

<210> 94
<211> 30
<212> DNA
<213> Artificial

<220>
<223> Primer

<400> 94
cctggagcca tcggagacat tgtgatgacc 30

<210> 95
<211> 30
<212> DNA
<213> Artificial

<220>
<223> Primer

<400> 95
ggtcatcaca atgtctccga tggctccagg 30

<210> 96
<211> 29
<212> DNA
<213> Artificial

<220>
<223> Primer

<400> 96
gctggcgccg catcagcccg tttgatttc 29

<210> 97
<211> 25

<212> DNA
<213> Artificial

<220>
<223> Primer

<400> 97
ctcaagctta attgccgcca ccatg 25

<210> 98
<211> 31
<212> DNA
<213> Artificial

<220>
<223> Primer

<400> 98
gaagggcgtg cagtgcgaag tgaagctggt g 31

<210> 99
<211> 31
<212> DNA
<213> Artificial

<220>
<223> Primer

<400> 99
caccagcttc acttcgcact gcacgccctt c 31

<210> 100
<211> 28
<212> DNA
<213> Artificial

<220>
<223> Primer

<400> 100
gcgctagctg cagagacagt gaccagag 28

## Description

The present application claims the priority benefit of Chinese Patent Application No. 201910022548.9, filed on 10 January 2019, which is hereby incorporated by reference in its entirety.

## TECHNICAL FIELD

The present invention relates to the field of antibody-based drug. Particularly, the present invention relates to an antibody against human PD-1 and use of the antibody for manufacturing a medicament.

## BACKGROUND OF THE INVENTION

Programmed cell death protein 1, or PD-1 (also known as CD279) is a member of the CD28 family of T cell receptors and is expressed on the surface of various immune cells such as T cells, B cells, monocytes and the like. PD-1 is an important immune checkpoint molecule that inhibits the function of CD4+ and CD8+ T cells within tumor microenvironment. Key ligands for PD-1, including PD-L1 (B7-H1) and PD-L2 (B7-DC), are expressed by immune cells, and can also be induced on various issues. T cells receive inhibitory signals when PD-L1 or PD-L2 binds to PD-1 thereon, then the proliferation of the T cells and the production of cytokines by the T cells are inhibited, effectively reducing immune responses the T cells involved in. In addition, many human tumor cells can escape T cell immune surveillance due to the high expression levels of PD-L1 and PD-L2 on the tumor cells (Pardoll DM. The blockade of immune checkpoints in cancer immunotherapy. Nat. Rev. Cancer12(4), 252-264 (2012)).

Currently there are three anti-human PD-1 monoclonal antibodies have been approved globally. The first one is Nivolumab, a fully human antibody against human PD-1 which shows an activity of inhibiting the binding of PD-1 to PD-L1 and PD-L2 and is used in the treatment of advanced melanoma, non-small cell lung cancer, renal cell carcinoma, Hodgkin's lymphoma, head and neck squamous cell carcinoma, urothelial carcinoma, colorectal carcinoma and hepatocellular carcinoma clinically; the second one is Pembrolizumab, a humanized antibody against human PD-1 which shows an activity of inhibiting the binding of PD-1 to PD-L1 and PD-L2 and is used in the treatment of malignant melanoma, non-small cell lung cancer, classical Hodgkin's lymphoma, head and neck squamous cell carcinoma, malignant tumor with d-mmr mutation or MSI-H malignant tumor clinically (Alsaab et al. PD-1 and PD-L1 Checkpoint Signaling Inhibition for Cancer Immunotherapy: Mechanism, Combinations, and Clinical Outcome. Front. Pharmacol. 8:561). The third one is Cemiplimab (Libtayo), an anti-human PD-1 monoclonal antibody that binds to PD-1 and blocks the interaction of PD-1 with PD-L1 and PD-L2, releasing the inhibition of the immune response (including anti-tumor immune response) mediated by the PD-1 pathway. Cemiplimab is used clinically for the treatment of patients with metastatic cutaneous squamous cell carcinoma (CSCC) or locally advanced cutaneous squamous cell carcinoma (Markham, A. & Duggan, S. Drugs. 2018. doi: 10.1007/s40265 -018-1012-5). In China, there are more than 10 monoclonal antibodies against human PD-1 in clinical stage, with indications covering relapsed and refractory malignant lymphoma, Hodgkin's lymphoma, B-cell non-Hodgkin's lymphoma, alveolar soft part sarcoma, advanced or recurrent malignancy, esophagus cancer, gastric carcinoma and gastroesophageal junction adenocarcinoma, nasopharyngeal carcinoma, head and neck squamous cell carcinoma, lung cancer, metastatic colorectal carcinoma, localized renal cell carcinoma, urothelial carcinoma, hepatocellular carcinoma, melanoma, advanced triple negative breast cancer, pleural mesothelioma, advanced neuroendocrine tumor, solid tumor unresectable or with d-mmr mutation or MSI-H solid tumor. Among the monoclonal antibodies, marketing applications have been submitted for SHR-1210 developed by Hengrui Medicine, tislelizumab developed by Beigene, and IBI-308 developed by Innovent Biologics; and JS001, named Teriprolizumab Injection (trade name: Tuoyi) and developed by Junshi Biosciences, has been conditionally approved for marketing on December 17, 2018.

Internationally there are three marketed products of anti-PD-1 antibodies and a number of products are in clinical researches. Patents or patent applications related include WO2004004771, WO2006121168, WO2008156712, WO2010029435, WO2012145493, WO2015085847, US20170044260, CN104250302, CN105330740, CN105531288 and the like. An analysis of these patents or patent applications found that anti-PD-1 antibodies could be obtained through hybridoma screening, and humanization, or phage display or yeast surface display technology, or through transgenic mouse technology. The process for obtaining anti-human PD-1 antibodies disclosed in WO2015085847 is: generating murine antibodies by use of hybridoma technology and obtaining murine antibody candidates through antibody activity analysis (ELISA (binding, and blocking), affinity/kinetics); preparing chimeric antibodies by expressing in mammalian cells sequences obtained by cloning the light chain and heavy chain variable region sequences of murine antibody candidates to the upstream of the light chain and heavy chain constant region sequences of a human antibody; then determining lead antibodies through antibody activity analysis, blocking assay of binding of PD-1 to ligands thereof, binding assay of anti-PD-1 antibodies to other members of the CD28 family, binding assay of anti-PD-1 antibodies to PD-1 on cell surface, and in vitro cell binding assay; selecting humanized templates in Germline database and performing humanization design of antibody sequences; performing antibody activity analysis, blocking assay of binding of PD-1 to ligands thereof, binding assay of anti-PD-1 antibodies to other members of the CD28 family, binding assay of anti-PD-1 antibodies to PD-1 on cell surface, and in vitro cell binding assay on the obtained humanized antibodies again; evaluating the druggability of the humanized antibodies through tumor cell growth inhibition experiments in vitro or in vivo, and finally obtaining humanized anti-PD-1 antibodies with unchanged affinity and specificity.

What epitope of an antigen an antibody recognizes is one of the important characteristics of the antibody. Anti-PD-1 antibody plays role of activating T cells through blocking the binding of PD-1 to its ligand PD-L1/PD-L2. Although all the reported antibodies can block the interactions between PD-1 and its ligands, the epitopes targeted by them are not exactly the same (Tan S, Zhang H, Chai Y, et al. An unexpected N-terminal loop in PD-1 dominates binding by nivolumab. Nat Commun. 2017;8:14369). In fact, different epitopes may lead to antibodies having unique characteristics in pharmaceutical effect and safety.

## SUMMARY OF THE INVENTION

The problem to be solved by the present invention is to obtain an antibody capable of specifically binding to human PD-1 with high affinity through hybridoma screening and humanization. The antibody of the present invention can not only effectively block the interaction of PD-1/PD-L1 or PD-1/PD-L2, but also has a unique antigen binding epitope, and thus has unique properties in pharmaceutical effect and safety.

Regarding to the above technical problem, one purpose of the present invention is to provide an antibody specific for human PD-1 or a functional fragment thereof, and use thereof.

Technical solutions provided by the present invention are as follows.

In one aspect, the present invention provides an antibody capable of binding to programmed cell death protein 1 (PD-1) or a fragment thereof, the antibody or fragment thereof comprising a light chain variable region and/or a heavy chain variable region, wherein the light chain variable region comprises light chain CDR1 (LCDR1), CDR2 (LCDR2), CDR3 (LCDR3) and/or the heavy chain variable region comprises heavy chain CDR1 (HCDR1), CDR2 (HCDR2) and CDR3 (HCDR3) as set forth in following sequences respectively:

| LCDR1 | LCDR2 | LCDR3 |
|---|---|---|
| X1ASQDVX2TX3VA ( SEQ ID NO:1 ) | X1ASTRX2X3 ( SEQ ID NO:2 ) | QQX1X2X3X4PX5 ( SEQ ID NO:3 ) |
| X1= K or R;<br>X2= E or S;<br>X3= A, V or Y | X1= A, G or W;<br>X2= A , H or Q;<br>X3= S or T | X1= A, F or Y;<br>X2= D, N or S;<br>X3= R, N or S;<br>X4= F or Y;<br>X5= G, W or Y |
| **HCDR1** | **HCDR2** | **HCDR3** |
| X1X2X3MS (SEQ ID NO:4) | TIX1X2X3X4X5X6TX7YPDSVKG ( SEQ ID NO:5 ) | PX1X2SGVAX3 ( SEQ ID NO: 6 ) |
| X1= D or S;<br>X2= A, N or Y;<br>X3= D, S or Y | X1= K, S or W;<br>X2= G, S or Y;<br>X3= D, G or S;<br>X4= G or S;<br>X5= G or S;<br>X6= T or Y;<br>X7= A, T or Y | X1= D, E or Y;<br>X2= A or S;<br>X3= T or Y |

In the antibody or fragment thereof provided by the present invention, preferably, the light chain variable region and/or the heavy chain variable region comprises LCDR1, LCDR2, LCDR3 and/or HCDR1, HCDR2, HCDR3 as set forth in following sequences respectively:

| LCDR1 | LCDR2 | LCDR3 |
|---|---|---|
| X1ASQDVX2TX3VA (SEQ ID NO: 1 ) | X1ASTRX2X3 ( SEQ ID NO:2 ) | QQX1X2X3X4PX5 ( SEQ ID NO:3 ) |

(continued)

| LCDR1 | LCDR2 | LCDR3 |
|---|---|---|
| X1= K or R;<br>X2= E;<br>X3= A, V or Y | X1= W;<br>X2= H;<br>X3= S or T | X1= Y;<br>X2= D, N or S;<br>X3= R or S;<br>X4= F or Y;<br>X5= W |
| **HCDR1** | **HCDR2** | **HCDR3** |
| X1X2X3MS ( SEQ ID NO:4 ) | TIX1X2X3X4X5X6TX7YPDSVKG ( SEQ ID NO:5 ) | PX1X2SGVAX3 ( SEQ ID NO: 6 ) |
| X1= D or S;<br>X2= A or Y;<br>X3= D or S | X1= S;<br>X2= G or S;<br>X3= D, G or S;<br>X4= G or S;<br>X5= S;<br>X6= Y;<br>X7= A or Y | X1= D or Y;<br>X2= S;<br>X3= Y |

In the antibody or fragment thereof provided by the present invention, preferably, the light chain variable region and/or the heavy chain variable region comprises LCDR1, LCDR2, LCDR3 and/or HCDR1, HCDR2, HCDR3 as set forth in following sequences respectively:

LCDR1 selected from the group consisting of SEQ ID NO:7, SEQ ID NO:8, SEQ ID NO:9, SEQ ID NO:10 and SEQ ID NO:11;
LCDR2 selected from the group consisting of SEQ ID NO:12, SEQ ID NO:13, SEQ ID NO:14, SEQ ID NO:15, SEQ ID NO:16 and SEQ ID NO:17;
LCDR3 selected from the group consisting of SEQ ID NO:18, SEQ ID NO:19, SEQ ID NO:20, SEQ ID NO:21, SEQ ID NO:22, SEQ ID NO:23, SEQ ID NO:24, SEQ ID NO:25, SEQ ID NO:26 and SEQ ID NO:27; and/or
HCDR1 selected from the group consisting of SEQ ID NO:28, SEQ ID NO:29, SEQ ID NO:30, SEQ ID NO:31, SEQ ID NO:32 and SEQ ID NO:33;
HCDR2 selected from the group consisting of SEQ ID NO:34, SEQ ID NO:35, SEQ ID NO:36, SEQ ID NO:37, SEQ ID NO:38, SEQ ID NO:39, SEQ ID NO:40, SEQ ID NO:41, SEQ ID NO:42, SEQ ID NO:43, SEQ ID NO:44, SEQ ID NO:45 and SEQ ID NO:46;
HCDR3 selected from the group consisting of SEQ ID NO:47, SEQ ID NO:48, SEQ ID NO:49, SEQ ID NO:50, and SEQ ID NO:51.

In the antibody or fragment thereof provided by the present invention, preferably, the light chain variable region comprises LCDR1, LCDR2, LCDR3 selected from the group consisting of sequence combinations as follows:

| Combination | LCDR1 | LCDR2 | LCDR3 |
|---|---|---|---|
| 1 | SEQ ID NO:7 | SEQ ID NO:12 | SEQ ID NO:18 |
| 2 | SEQ ID NO:8 | SEQ ID NO:12 | SEQ ID NO:18 |
| 3 | SEQ ID NO:9 | SEQ ID NO:12 | SEQ ID NO:18 |
| 4 | SEQ ID NO:10 | SEQ ID NO:12 | SEQ ID NO:18 |
| 5 | SEQ ID NO:11 | SEQ ID NO:12 | SEQ ID NO:18 |
| 6 | SEQ ID NO:7 | SEQ ID NO:13 | SEQ ID NO:18 |
| 7 | SEQ ID NO:7 | SEQ ID NO:14 | SEQ ID NO:18 |

(continued)

| | | | |
|---|---|---|---|
| 8 | SEQ ID NO:7 | SEQ ID NO:15 | SEQ ID NO:18 |
| 9 | SEQ ID NO:7 | SEQ ID NO:16 | SEQ ID NO:18 |
| 10 | SEQ ID NO:7 | SEQ ID NO:17 | SEQ ID NO:18 |
| 11 | SEQ ID NO:7 | SEQ ID NO:12 | SEQ ID NO:19 |
| 12 | SEQ ID NO:7 | SEQ ID NO:12 | SEQ ID NO:20 |
| 13 | SEQ ID NO:7 | SEQ ID NO:12 | SEQ ID NO:21 |
| 14 | SEQ ID NO:7 | SEQ ID NO:12 | SEQ ID NO:22 |
| 15 | SEQ ID NO:7 | SEQ ID NO:12 | SEQ ID NO:23 |
| 16 | SEQ ID NO:7 | SEQ ID NO:12 | SEQ ID NO:24 |
| 17 | SEQ ID NO:7 | SEQ ID NO:12 | SEQ ID NO:25 |
| 18 | SEQ ID NO:7 | SEQ ID NO:12 | SEQ ID NO:26 |
| 19 | SEQ ID NO:7 | SEQ ID NO:12 | SEQ ID NO:27 |
| 20 | SEQ ID NO:8 | SEQ ID NO:17 | SEQ ID NO:18 |
| 21 | SEQ ID NO:8 | SEQ ID NO:12 | SEQ ID NO:22 |
| 22 | SEQ ID NO:8 | SEQ ID NO:12 | SEQ ID NO:25 |
| 23 | SEQ ID NO:7 | SEQ ID NO:17 | SEQ ID NO:22 |
| 24 | SEQ ID NO:8 | SEQ ID NO:17 | SEQ ID NO:22 |

and/or the heavy chain variable region comprises HCDR1, HCDR2, HCDR3 selected from the group consisting of sequence combinations as follows:

| Combination | HCDR1 | HCDR2 | HCDR3 |
|---|---|---|---|
| 1 | SEQ ID NO:28 | SEQ ID NO:34 | SEQ ID NO:47 |
| 2 | SEQ ID NO:29 | SEQ ID NO:34 | SEQ ID NO:47 |
| 3 | SEQ ID NO:30 | SEQ ID NO:34 | SEQ ID NO:47 |
| 4 | SEQ ID NO:31 | SEQ ID NO:34 | SEQ ID NO:47 |
| 5 | SEQ ID NO:32 | SEQ ID NO:34 | SEQ ID NO:47 |
| 6 | SEQ ID NO:33 | SEQ ID NO:34 | SEQ ID NO:47 |
| 7 | SEQ ID NO:28 | SEQ ID NO:35 | SEQ ID NO:47 |
| 8 | SEQ ID NO:28 | SEQ ID NO:36 | SEQ ID NO:47 |
| 9 | SEQ ID NO:28 | SEQ ID NO:37 | SEQ ID NO:47 |
| 10 | SEQ ID NO:28 | SEQ ID NO:38 | SEQ ID NO:47 |
| 11 | SEQ ID NO:28 | SEQ ID NO:39 | SEQ ID NO:47 |
| 12 | SEQ ID NO:28 | SEQ ID NO:40 | SEQ ID NO:47 |
| 13 | SEQ ID NO:28 | SEQ ID NO:41 | SEQ ID NO:47 |
| 14 | SEQ ID NO:28 | SEQ ID NO:42 | SEQ ID NO:47 |
| 15 | SEQ ID NO:28 | SEQ ID NO:43 | SEQ ID NO:47 |
| 16 | SEQ ID NO:28 | SEQ ID NO:44 | SEQ ID NO:47 |
| 17 | SEQ ID NO:28 | SEQ ID NO:45 | SEQ ID NO:47 |
| 18 | SEQ ID NO:28 | SEQ ID NO:34 | SEQ ID NO:48 |

(continued)

| Combination | HCDR1 | HCDR2 | HCDR3 |
|---|---|---|---|
| 19 | SEQ ID NO:28 | SEQ ID NO:34 | SEQ ID NO:49 |
| 20 | SEQ ID NO:28 | SEQ ID NO:34 | SEQ ID NO:50 |
| 21 | SEQ ID NO:28 | SEQ ID NO:34 | SEQ ID NO:51 |
| 22 | SEQ ID NO:32 | SEQ ID NO:40 | SEQ ID NO:47 |
| 23 | SEQ ID NO:32 | SEQ ID NO:44 | SEQ ID NO:47 |
| 24 | SEQ ID NO:28 | SEQ ID NO:46 | SEQ ID NO:47 |
| 25 | SEQ ID NO:32 | SEQ ID NO:40 | SEQ ID NO:47 |
| 26 | SEQ ID NO:28 | SEQ ID NO:46 | SEQ ID NO:47 |

Preferably, the light chain variable region and the heavy chain variable region comprise LCDR1, LCDR2, LCDR3 and HCDR1, HCDR2, HCDR3 selected from the group consisting of sequence combinations as follows:

| Combination | LCDR1 | LCDR2 | LCDR3 | HCDR1 | HCDR2 | HCDR3 |
|---|---|---|---|---|---|---|
| 1 | SEQ ID NO:7 | SEQ ID NO: 12 | SEQ ID NO: 18 | SEQ ID NO: 28 | SEQ ID NO: 34 | SEQ ID NO: 47 |
| 2 | SEQ ID NO:8 | SEQ ID NO: 12 | SEQ ID NO: 18 | SEQ ID NO: 28 | SEQ ID NO: 34 | SEQ ID NO: 47 |
| 3 | SEQ ID NO:9 | SEQ ID NO: 12 | SEQ ID NO: 18 | SEQ ID NO: 28 | SEQ ID NO: 34 | SEQ ID NO: 47 |
| 4 | SEQ ID NO: 10 | SEQ ID NO: 12 | SEQ ID NO: 18 | SEQ ID NO: 28 | SEQ ID NO: 34 | SEQ ID NO: 47 |
| 5 | SEQ ID NO: 11 | SEQ ID NO: 12 | SEQ ID NO: 18 | SEQ ID NO: 28 | SEQ ID NO: 34 | SEQ ID NO: 47 |
| 6 | SEQ ID NO:7 | SEQ ID NO: 13 | SEQ ID NO: 18 | SEQ ID NO: 28 | SEQ ID NO: 34 | SEQ ID NO: 47 |
| 7 | SEQ ID NO:7 | SEQ ID NO: 14 | SEQ ID NO: 18 | SEQ ID NO: 28 | SEQ ID NO: 34 | SEQ ID NO: 47 |
| 8 | SEQ ID NO:7 | SEQ ID NO: 15 | SEQ ID NO: 18 | SEQ ID NO: 28 | SEQ ID NO: 34 | SEQ ID NO: 47 |
| 9 | SEQ ID NO:7 | SEQ ID NO: 16 | SEQ ID NO: 18 | SEQ ID NO: 28 | SEQ ID NO: 34 | SEQ ID NO: 47 |
| 10 | SEQ ID NO:7 | SEQ ID NO: 17 | SEQ ID NO: 18 | SEQ ID NO: 28 | SEQ ID NO: 34 | SEQ ID NO: 47 |
| 11 | SEQ ID NO:7 | SEQ ID NO: 12 | SEQ ID NO: 19 | SEQ ID NO: 28 | SEQ ID NO: 34 | SEQ ID NO: 47 |
| 12 | SEQ ID NO:7 | SEQ ID NO: 12 | SEQ ID NO: 20 | SEQ ID NO: 28 | SEQ ID NO: 34 | SEQ ID NO: 47 |
| 13 | SEQ ID NO:7 | SEQ ID NO: 12 | SEQ ID NO: 21 | SEQ ID NO: 28 | SEQ ID NO: 34 | SEQ ID NO: 47 |
| 14 | SEQ ID NO:7 | SEQ ID NO: 12 | SEQ ID NO: 22 | SEQ ID NO: 28 | SEQ ID NO: 34 | SEQ ID NO: 47 |
| 15 | SEQ ID NO:7 | SEQ ID NO: 12 | SEQ ID NO: 23 | SEQ ID NO: 28 | SEQ ID NO: 34 | SEQ ID NO: 47 |

(continued)

| Combination | LCDR1 | LCDR2 | LCDR3 | HCDR1 | HCDR2 | HCDR3 |
|---|---|---|---|---|---|---|
| 16 | SEQ ID NO:7 | SEQ ID NO: 12 | SEQ ID NO: 24 | SEQ ID NO: 28 | SEQ ID NO: 34 | SEQ ID NO: 47 |
| 17 | SEQ ID NO:7 | SEQ ID NO: 12 | SEQ ID NO: 25 | SEQ ID NO: 28 | SEQ ID NO: 34 | SEQ ID NO: 47 |
| 18 | SEQ ID NO:7 | SEQ ID NO: 12 | SEQ ID NO: 26 | SEQ ID NO: 28 | SEQ ID NO: 34 | SEQ ID NO: 47 |
| 19 | SEQ ID NO:7 | SEQ ID NO: 12 | SEQ ID NO: 27 | SEQ ID NO: 28 | SEQ ID NO: 34 | SEQ ID NO: 47 |
| 20 | SEQ ID NO:8 | SEQ ID NO: 17 | SEQ ID NO: 18 | SEQ ID NO: 28 | SEQ ID NO: 34 | SEQ ID NO: 47 |
| 21 | SEQ ID NO:8 | SEQ ID NO: 12 | SEQ ID NO: 22 | SEQ ID NO: 28 | SEQ ID NO: 34 | SEQ ID NO: 47 |
| 22 | SEQ ID NO:8 | SEQ ID NO: 12 | SEQ ID NO: 25 | SEQ ID NO: 28 | SEQ ID NO: 34 | SEQ ID NO: 47 |
| 23 | SEQ ID NO:7 | SEQ ID NO: 17 | SEQ ID NO: 22 | SEQ ID NO: 28 | SEQ ID NO: 34 | SEQ ID NO: 47 |
| 24 | SEQ ID NO:8 | SEQ ID NO: 17 | SEQ ID NO: 22 | SEQ ID NO: 28 | SEQ ID NO: 34 | SEQ ID NO: 47 |
| 25 | SEQ ID NO:7 | SEQ ID NO: 12 | SEQ ID NO: 18 | SEQ ID NO: 29 | SEQ ID NO: 34 | SEQ ID NO: 47 |
| 26 | SEQ ID NO:7 | SEQ ID NO: 12 | SEQ ID NO: 18 | SEQ ID NO: 30 | SEQ ID NO: 34 | SEQ ID NO: 47 |
| 27 | SEQ ID NO:7 | SEQ ID NO: 12 | SEQ ID NO: 18 | SEQ ID NO: 31 | SEQ ID NO: 34 | SEQ ID NO: 47 |
| 28 | SEQ ID NO:7 | SEQ ID NO: 12 | SEQ ID NO: 18 | SEQ ID NO: 32 | SEQ ID NO: 34 | SEQ ID NO: 47 |
| 29 | SEQ ID NO:7 | SEQ ID NO: 12 | SEQ ID NO: 18 | SEQ ID NO: 33 | SEQ ID NO: 34 | SEQ ID NO: 47 |
| 30 | SEQ ID NO:7 | SEQ ID NO: 12 | SEQ ID NO: 18 | SEQ ID NO: 28 | SEQ ID NO: 35 | SEQ ID NO: 47 |
| 31 | SEQ ID NO:7 | SEQ ID NO: 12 | SEQ ID NO: 18 | SEQ ID NO: 28 | SEQ ID NO: 36 | SEQ ID NO: 47 |
| 32 | SEQ ID NO:7 | SEQ ID NO: 12 | SEQ ID NO: 18 | SEQ ID NO: 28 | SEQ ID NO: 37 | SEQ ID NO: 47 |
| 33 | SEQ ID NO:7 | SEQ ID NO: 12 | SEQ ID NO: 18 | SEQ ID NO: 28 | SEQ ID NO: 38 | SEQ ID NO: 47 |
| 34 | SEQ ID NO:7 | SEQ ID NO: 12 | SEQ ID NO: 18 | SEQ ID NO: 28 | SEQ ID NO: 39 | SEQ ID NO: 47 |
| 35 | SEQ ID NO:7 | SEQ ID NO: 12 | SEQ ID NO: 18 | SEQ ID NO: 28 | SEQ ID NO: 40 | SEQ ID NO: 47 |
| 36 | SEQ ID NO:7 | SEQ ID NO: 12 | SEQ ID NO: 18 | SEQ ID NO: 28 | SEQ ID NO: 41 | SEQ ID NO: 47 |
| 37 | SEQ ID NO:7 | SEQ ID NO: 12 | SEQ ID NO: 18 | SEQ ID NO: 28 | SEQ ID NO: 42 | SEQ ID NO: 47 |

(continued)

| Combination | LCDR1 | LCDR2 | LCDR3 | HCDR1 | HCDR2 | HCDR3 |
|---|---|---|---|---|---|---|
| 38 | SEQ ID NO:7 | SEQ ID NO: 12 | SEQ ID NO: 18 | SEQ ID NO: 28 | SEQ ID NO: 43 | SEQ ID NO: 47 |
| 39 | SEQ ID NO:7 | SEQ ID NO: 12 | SEQ ID NO: 18 | SEQ ID NO: 28 | SEQ ID NO: 44 | SEQ ID NO: 47 |
| 40 | SEQ ID NO:7 | SEQ ID NO: 12 | SEQ ID NO: 18 | SEQ ID NO: 28 | SEQ ID NO: 45 | SEQ ID NO: 47 |
| 41 | SEQ ID NO:7 | SEQ ID NO: 12 | SEQ ID NO: 18 | SEQ ID NO: 28 | SEQ ID NO: 34 | SEQ ID NO: 48 |
| 42 | SEQ ID NO:7 | SEQ ID NO: 12 | SEQ ID NO: 18 | SEQ ID NO: 28 | SEQ ID NO: 34 | SEQ ID NO: 49 |
| 43 | SEQ ID NO:7 | SEQ ID NO: 12 | SEQ ID NO: 18 | SEQ ID NO: 28 | SEQ ID NO: 34 | SEQ ID NO: 50 |
| 44 | SEQ ID NO:7 | SEQ ID NO: 12 | SEQ ID NO: 18 | SEQ ID NO: 28 | SEQ ID NO: 34 | SEQ ID NO: 51 |
| 45 | SEQ ID NO:7 | SEQ ID NO: 12 | SEQ ID NO: 18 | SEQ ID NO: 32 | SEQ ID NO: 40 | SEQ ID NO: 47 |
| 46 | SEQ ID NO:7 | SEQ ID NO: 12 | SEQ ID NO: 18 | SEQ ID NO: 32 | SEQ ID NO: 44 | SEQ ID NO: 47 |
| 47 | SEQ ID NO:7 | SEQ ID NO: 12 | SEQ ID NO: 18 | SEQ ID NO: 28 | SEQ ID NO: 46 | SEQ ID NO: 47 |
| 48 | SEQ ID NO:8 | SEQ ID NO: 17 | SEQ ID NO: 22 | SEQ ID NO: 32 | SEQ ID NO: 40 | SEQ ID NO: 47 |
| 49 | SEQ ID NO:8 | SEQ ID NO: 17 | SEQ ID NO: 22 | SEQ ID NO: 28 | SEQ ID NO: 46 | SEQ ID NO: 47 |

The antibody or fragment thereof provided by the present invention has one or more activities selected from the group consisting of:

(1) blocking the binding of PD-1 to its ligand PD-L1 or PD-L2;
(2) binding to primate PD-1 specifically, and showing no cross-reactivity with non-primate PD-1;
(3) showing no binding to members of CD28 family other than PD-1;
(4) inducing the production of IL-2 and/or IFN-γ in CD4+ T cells; and
(5) having no ADCC-effector function.

Preferably, the antibody or fragment thereof binds to the carbohydrate chain at N58 of human PD-1; preferably, the heavy chain variable region of the antibody or fragment thereof comprises an amino acid sequence as set forth in SEQ ID NO:47.

Preferably, the light chain variable region comprises an amino acid sequence as set forth in SEQ ID NO:52 or SEQ ID NO:60, or an amino acid sequence having at least 75% sequence identity to the amino acid sequence as set forth in SEQ ID NO:52 or SEQ ID NO:60; and/or, the heavy chain variable region comprises an amino acid sequence as set forth in SEQ ID NO:54 or SEQ ID NO:62, or an amino acid sequence having at least 75% sequence identity to the amino acid sequence as set forth in SEQ ID NO:54 or SEQ ID NO:62.

Preferably, the term "at least 75% sequence identity" refers to any percent identity greater than or equal to 75%, for example at least 80%, preferably at least 85%, more preferably at least 90%, further more preferably at least 91%, 92%, 93%, 94%, 95%, 96%, 97%, 98% or even 99% identity.

According to specific embodiments of the present invention, in the antibody or fragment thereof provided by the present invention, the light chain variable region comprises an amino acid sequence as set forth in SEQ ID NO:52, and the heavy chain variable region comprises an amino acid sequence as set forth in SEQ ID NO:54; or the light chain variable region comprises an amino acid sequence as set forth in SEQ ID NO:60, and the heavy chain variable region comprises an amino acid sequence as set forth in SEQ ID NO:62.

In the antibody or fragment thereof provided by the present invention, preferably, the light chain variable region comprises an amino acid sequence as set forth in SEQ ID NO:60 or a variant thereof; and with reference to the numbering and composition of amino acid residues of the sequence as set forth in SEQ ID NO: 60, the variant has one or more, preferably 1-3 amino acid substitution(s) in comparison with SEQ ID NO:60 selected from the group consisting of: K24R, E30S, V32A, V32Y, W50A, W50G, H55A, H55Q, T56S, Y91A, Y91F, S92D, S92N, R93N, R93S, Y94F, W96G and W96Y; preferably, the variant has one or more, preferably 1-3 amino acid substitution(s) in comparison with SEQ ID NO:60 selected from the group consisting of: K24R, V32A, V32Y, T56S, S92D, S92N, R93S and Y94F; and/or, the heavy chain variable region comprises an amino acid sequence as set forth in SEQ ID NO:62 or a variant thereof; and with reference to the numbering and composition of amino acid residues of the sequence as set forth in SEQ ID NO: 62, the variant has one or more, preferably 1-3 amino acid substitution(s) in comparison with SEQ ID NO:62 selected from the group consisting of:
S31D, Y32A, Y32N, D33S, D33Y, S52K, S52W, G53S, G53Y, G54D, G54S, G55S, S56G, Y57T, Y59A, Y59T, D100E, D100Y, S101A and Y106T; preferably, the variant has one or more, preferably 1-3 amino acid substitution(s) in comparison with SEQ ID NO:62 selected from the group consisting of: S31D, Y32A, D33S, G53S, G54S, G55S, Y59A and D100Y.

According to specific embodiments of the present invention, the present invention provides a variant antibody or fragment thereof, wherein the heavy chain variable region of the variant antibody or fragment thereof comprises an amino acid sequence as set forth in SEQ ID NO:62; while with reference to the numbering and composition of amino acid residues of the sequence as set forth in SEQ ID NO:60, the light chain variable region of the variant antibody or fragment thereof only comprises following amino acid substitution(s): 1) K24R; 2) E30S; 3) V32A; 4) V32Y; 5) W50A; 6) W50G; 7) H55A; 8) H55Q; 9) T56S; 10) Y91A; 11) Y91F; 12) S92D; 13) S92N; 14) R93N; 15) R93S; 16) Y94F; 17) W96G; 18) W96Y; 19) K24R and T56S; 20) K24R and S92N; 21) K24R and Y94F; 22) T56S and S92N; or, 23) K24R, T56S and S92N.

According to specific embodiments of the present invention, the present invention provides a variant antibody or fragment thereof, wherein the light chain variable region of the variant antibody or fragment thereof comprises an amino acid sequence as set forth in SEQ ID NO:60; while with reference to the numbering and composition of amino acid residues of the sequence as set forth in SEQ ID NO:62, the heavy chain variable region of the variant antibody or fragment thereof only comprises following amino acid substitution(s): 1) S31D; 2) Y32A; 3) Y32N; 4) D33S; 5) D33Y; 6) S52K; 7) S52W; 8) G53S; 9) G53Y; 10) G54D; 11) G54S; 12) G55S; 13) S56G; 14) Y57T; 15) Y59A; 16) Y59T; 18) D100E; 19) D100Y; 20) S101A; 21) Y106T; 22) D33S and G54S; 23) D33S and Y59A; or, 24) G54S and Y59A.

According to specific embodiments of the present invention, the present invention provides a variant antibody or fragment thereof, wherein with reference to the numbering and composition of amino acid residues of the sequence as set forth in SEQ ID NO:60, the light chain variable region of the variant antibody or fragment thereof only comprises amino acid substitution(s) K24R, T56S and S92N, and with reference to the numbering and composition of amino acid residues of the sequence as set forth in SEQ ID NO:62, the heavy chain variable region of the variant antibody or fragment thereof only comprises amino acid substitution(s) D33S and G54S, or amino acid substitution(s) G54S and Y59A.

The antibody provided by the present invention can be any of a monoclonal antibody, a single chain antibody, a bifunctional antibody, a single domain antibody, a nanobody, a fully or partially humanized antibody or a chimeric antibody, or the like; preferably, the antibody is an IgA, IgD, IgE, IgG, or IgM antibody, more preferably is an IgG1 or IgG4 antibody.

The fragment provided by the present invention is any fragment of the antibody that is capable of binding to PD-1 or any portion thereof; preferably, the fragment is a fragment scFv, BsFv, dsFv, $(dsFv)_2$, Fab, Fab', $F(ab')_2$ or Fv of the antibody.

Preferably, the heavy chain constant region of the antibody provided by the present invention is of IgG1 or IgG4 subtype, and the light chain constant region is of κ type.

More preferably, the light chain constant region of the antibody comprises an amino acid sequence as set forth in SEQ ID NO:56 or SEQ ID NO:64, or an amino acid sequence having at least 75% sequence identity to the amino acid sequence as set forth in SEQ ID NO:56 or SEQ ID NO:64; and/or the heavy chain constant region of the antibody comprises an amino acid sequence as set forth in SEQ ID NO:58 or SEQ ID NO:66, or an amino acid sequence having at least 75% sequence identity to the amino acid sequence as set forth in SEQ ID NO:58 or SEQ ID NO:66; wherein the term "at least 75% sequence identity" refers to any percent identity greater than or equal to 75%, for example at least 80%, preferably at least 85%, more preferably at least 90%, further more preferably at least 91%, 92%, 93%, 94%, 95%, 96%, 97%, 98% or even 99% identity.

According to specific embodiments of the present invention, in the antibody provided by the present invention, the light chain variable region comprises an amino acid sequence as set forth in SEQ ID NO:52, the light chain constant region comprises an amino acid sequence as set forth in SEQ ID NO:56, the heavy chain variable region comprises an amino acid sequence as set forth in SEQ ID NO:54, and the heavy chain constant region comprises an amino acid sequence as set forth in SEQ ID NO:58; alternatively, the light chain variable region comprises an amino acid sequence as set forth in SEQ ID NO:60, the light chain constant region comprises an amino acid sequence as set forth in SEQ ID NO:62, the heavy chain variable region comprises an amino acid sequence as set forth in SEQ ID NO:64, and the heavy chain constant region comprises an amino acid sequence as set forth in SEQ ID NO:66.

Based on the antibody or fragment thereof provided by the present invention, the present invention also provides a conjugate or fusion protein comprising the antibody or fragment thereof of the present invention. The conjugate or fusion protein may comprise an additional moiety associated with the antibody or fragment thereof of the present invention physically or chemically, wherein said additional moiety can be, for example, a cell surface receptor, a small molecule compound such as an amino acid and a carbohydrate, a small molecule polymer or any other moiety that modifies the antibody of the present invention, or even an active protein or polypeptide. For example, the conjugate or fusion protein can be a bispecific antibody comprising the antibody or fragment thereof of the present invention.

In another aspect, the present invention provides a nucleic acid molecule comprising a nucleotide sequence encoding the heavy chain CDRs, the light CDRs, the heavy chain variable region, the light chain variable region, the heavy chain or the light chain of any antibody or fragment thereof of the present invention.

Preferably, the nucleic acid molecule comprises a nucleotide sequence as set forth in SEQ ID NO:53, SEQ ID NO:55, SEQ ID NO:57, SEQ ID NO:59, SEQ ID NO:61, SEQ ID NO:63, SEQ ID NO:65 or SEQ ID NO:67.

In further another aspect, the present invention provides a vector comprising the nucleic acid molecule of the present invention. The vector can be an eukaryotic expression vector, a prokaryotic expression vector, an artificial chromosome and a phage vector, etc.

The vector or the nucleic acid molecule of the present invention can be used for transforming or transfecting a host cell, or enter into a host cell by any means, for preserving or expressing the antibody and the like.

Therefore, in still another aspect, the present invention provides a host cell comprising the nucleic acid molecule and/or vector of the present invention, or transformed or transfected with the nucleic acid molecule and/or vector of the present invention. The host cell can be any prokaryotic or eukaryotic cell, such as a bacterial or insect, fungal, plant or animal cell.

According to the disclosure of the present invention, the antibody or fragment thereof, and the corresponding conjugate or fusion protein, nucleic acid molecule, vector and/or host cell provided by the present invention can be obtained through conventional technical means known in the art. The antibody or fragment thereof, conjugate or fusion protein, nucleic acid molecule, vector and/or host cell can be comprised in a pharmaceutical composition, or particularly in a pharmaceutical preparation, for various purposes according to practical needs.

Therefore, in yet still another aspect, the present invention also provides a pharmaceutical composition comprising the antibody or fragment thereof, conjugate or fusion protein, nucleic acid molecule, vector and/or host cell provided by the present invention, and optionally a pharmaceutically acceptable excipient.

Based on the antibody or a fragment thereof that specifically binds to human PD-1 or any portion thereof, the present invention also provides related applications of the above-mentioned subject matters.

Specifically, in further still another aspect, the present invention provides use of the antibody or fragment thereof, conjugate or fusion protein, nucleic acid molecule, vector, host cell and/or pharmaceutical composition for manufacturing a medicament for the prevention or treatment of a tumor or cancer. Preferably, the tumor or cancer is selected from the group consisting of non-small cell lung cancer, classical Hodgkin's lymphoma, gastric carcinoma, (primary) liver cancer, melanoma, malignant tumor with d-mmr mutation or MSI-H malignant tumor, cervical cancer, head and neck squamous cell carcinoma, urinary bladder cancer, primary mediastinal B-cell lymphoma, renal cell carcinoma, colorectal cancer and urothelial carcinoma; more preferably, the tumor or cancer is non-small cell lung cancer, melanoma, colorectal cancer, liver cancer, head and neck squamous cell carcinoma, classical Hodgkin's lymphoma or urothelial carcinoma.

In also another aspect, the present invention provides a pharmaceutical combination, comprising:
(1) the antibody or fragment thereof, conjugate or fusion protein, nucleic acid molecule, vector, host cell and/or pharmaceutical composition provided by the present invention; (2) other immunopotentiator or immunopotentiating means, such as a small molecule chemical drug, a targeted agent, a recombinant protein drug such as an antibody etc., a vaccine, a ADC, an oncolytic viruse, a drug for gene and nucleic acid therapy, and a radiotherapy.

In addition, the present invention provides a method of preventing or treating a tumor or cancer, including administrating the antibody or fragment thereof, conjugate or fusion protein, nucleic acid molecule, vector, host cell and/or pharmaceutical composition, and optionally other drug or means to a subject in need thereof. Preferably, the tumor or cancer is selected from the group consisting of non-small cell lung cancer, classical Hodgkin's lymphoma, gastric carcinoma, (primary) liver cancer, melanoma, malignant tumor with d-mmr mutation or MSI-H malignant tumor, cervical cancer, head and neck squamous cell carcinoma, urinary bladder cancer, primary mediastinal B-cell lymphoma, renal cell carcinoma, colorectal cancer and urothelial carcinoma; more preferably, the tumor or cancer is non-small cell lung cancer, melanoma, colorectal cancer, liver cancer, head and neck squamous cell carcinoma, classical Hodgkin's lymphoma or urothelial carcinoma. The term "optional other drug or means" refers to other immunopotentiator or immunopotentiating means that can be administrated combined with the antibody or fragment thereof, conjugate or fusion protein, nucleic acid molecule, vector, host cell and/or pharmaceutical composition of the present invention, such as a small molecule chemical drug, a targeted agent, a recombinant protein drug such as an antibody etc., a vaccine, a ADCs, an oncolytic viruse, a drugs for gene and nucleic acid therapy, and a radiotherapy. The combined administration of the two can be conducted in any order, for example they can be administrated simultaneously, continuously or at a certain time interval. The subject is a mammal, preferably a human.

In still also another aspect, the present invention provides a kit comprising the antibody or fragment thereof, conjugate or fusion protein, nucleic acid molecule, vector, host cell and/or pharmaceutical composition provided by the invention.

In the present invention, mice were immunized with recombinant human PD-1 extracellular domain protein (NCBI accession No.: NP_005009.2, 21aa-167aa), and then spleen cells of the mice were taken and an antibody was obtained through screening with hybridoma technology. The antibody obtained (named "317" in the present invention) has high binding affinity to both the recombinant human PD-1 extracellular domain protein and human PD-1 on cell surface, and can also specifically block the bindings between the receptor and the ligands PD-1/PD-L1, and PD-1/PD-L2. Further, a humanized antibody (named "h317" in the present invention) with unchanged affinity and specificity was obtained through humanized technology and subsequently a comprehensive evaluation on the activities of the whole antibody being IgG4 was performed with assays regarding binding, blocking, promoting T cell activation and anti-tumor efficacy in vivo. Furthermore, a crystal complex of Fab of the obtained antibody and PD-1-ECD was constructed, and the antigen binding epitope of the antibody was confirmed by crystal diffraction and verified with additional mutants.

Experiments have proved that the antibody provided by the present invention can block the binding between PD-1 and PD-L1/PD-L2 effectively, and compete with Nivolumab and Pembrolizumab (Keytruda) for antigen binding. However, according to the analysis results of the crystal structure of antigen-antibody complex, it is found the antigen-binding epitope of the antibody provided by the present invention is different from that of Nivolumab or Keytruda. In this regard, the antibody of the present invention is the first antibody identified to recognize an antigen epitope which is related to the N-glycosylation site of Glu at position 58 of PD-1.

Specifically, the present invention has following advantages in comparison with the prior art: Firstly, the antibody of the present invention is an anti-PDI humanized antibody with high affinity.

h317, the humanized anti-PD-1 antibody of the present invention was shown to specifically bind to human PD-1 protein with an affinity (KD) of 6.16E-09M, while the affinity (KD) of the control antibody Nivolumab was 8.06E-09M. Moreover, under the same conditions, the dissociation constant of h317 (kd(1/s) 9.50E-04) was lower than the dissociation constant of Nivolumab (kd(1/s):1.69E-03), indicating that h317 has a high affinity to human PD-1, which provides a basis for the inhibitory effect of h317 on PD-1/PD-L1 signaling pathway.

Secondly, the antibody of the present invention shows good bioactivities.

Experimental results showed that h317 of the present invention could block the binding between the recombinant human PD-1 and its ligands PD-L1 and PD-L2 effectively, with IC50 value of 1.4 nM for both, which was similar to the blocking activity of the control antibody Nivolumab (IC50: 1.3 nM). In addition, as demonstrated in in vitro experiment, h317 could promote the secretion of killer cytokines IL-2 and IFN-$\gamma$ by human mixed lymphocytes, namely h317 can induce the immune response mediated by Th1 type cells.

Thirdly, the antibody of the present invention recognizes a special antigen epitope.

The antibody of the present invention specifically binds to human PD-1, and the antigen epitope it binds is unique.

The overall structure analysis of the PD-1-h317-Fab complex showed that the PD-1 antigen (extracellular region 1-167aa) existed as a monomer, and formed a complex with the h317-Fab fragment of the anti-PD-1 antibody in a ratio of 1:1. The sites of PD-1 bound by the antibody were mainly located in the loops of PD-1, including BC loop, C' D loop and FG loop. In addition, glycosylation at sites N49, N58, and N116 could be observed in the structure of PD-1 antigen, while the glycosylation at site N74 was not clear. Further, the involvement of the carbohydrate chain at N58 in the binding to h317-Fab could also be observed. In contrast, the carbohydrate chain at this site in the structure of any other reported complex formed between PD-1 antigen and an antibody thereof does not involve in the interaction between the PD-1 antigen and the antibody. Four glycosylation sites on PD-1 were mutated to alanine (A) respectively by site-directed mutation according to the information provided by the crystal complex, plasmids carrying mutated PD-1 (PD-1-mut) obtained were transiently transfected into 293 cells, and the binding between the h317 and the PD-1-mut was detected by FACS. The results showed that the mutation of amino acid at position N58 of PD-1 to A made the position unable to be glycosylated, causing h317 to lose its binding ability to PD-1. In this regard, h317 is the first antibody identified to recognize an antigen epitope which is related to the N58 carbohydrate chain.

Fourthly, the antibody of the present invention has an obvious anti-tumor effect in a PD-1 transgenic mouse xenograft tumor model.

In the PD-1 transgenic mouse xenograft tumor model, h317 was shown to significantly inhibit the growth of subcutaneous xenograft tumor in PD-1 transgenic mice at high dose (10 mg/kg) and medium dose (2 mg/kg), and an inhibitory effect on tumor comparable to that of the control antibody Nivolumab was observed with h317 at the high and medium doses.

## BRIEF DESCRIPTION OF THE DRAWINGS

Embodiments of the present invention are described in detail hereinafter in combination with the accompanying drawings, in which:

Figure 1 includes Figs. 1A-1C which show the binding of the supernatants of the positive hybridomas to the recombinant human PD-1 extracellular domain protein detected by ELISA, in which Figs. 1A, 1B and 1C show results obtained from the first, the second and the third screening respectively.

Figure 2 shows the cross-reactivity of the supernatants of the positive hybridomas with recombinant PD-1 from different species detected by ELISA, in which Nivo represents Nivolumab.

Figure 3 shows the antibody subtype identification of the clone 317, and the results show that the heavy chain is IgG1 subtype, and the light chain is Kappa chain.

Figure 4 shows the results of an affinity assay in which the affinities of the chimeric antibody 317 (Fig. 4A) and Nivolumab (Fig. 4B) to the recombinant human PD-1 extracellular domain protein were detected.

Figure 5 shows the results of an affinity assay in which the affinities of the h317 (Fig. 5A) and Nivolumab (Fig. 5B) to the recombinant human PD-1 extracellular domain protein were detected.

Figure 6 shows the competition of h317 with Nivolumab for inhibiting the binding of PD-1 to PD-L1 (Fig. 6A) and PD-1 to PD-L2 (Fig. 6B) detected by ELISA.

Figure 7 shows the curves for analyzing the binding of h317 to recombinant PD-1 from different species.

Figure 8 shows the curves for analyzing the binding of h317 to members of the CD28 family.

Figure 9 shows the detection results of the production of IL-2 (Fig. 9A) and IFN-$\gamma$ (Fig. 9B) stimulated by h317 in MLR.

Figure 10 shows the results of ADCC effect of human PBMCs against 293T/hPD-1 cells mediated by h317.

Figure 11 shows the statistical results of the volume of subcutaneously transplanted tumors in PD-1 transgenic mice which growth was inhibited by h317.

Figure 12 shows the endocytosis of the h317 mediated by PD-1.

Figure 13 shows the overall structure of the PD-1-h317-Fab complex.

Figure 14 includes panels A-D which show the interaction between the molecules forming the PD-1-h317-Fab complex, in which panel A shows the binding area of PD-1 and h317-Fab, panel B shows the interaction between the BC loop and h317-Fab, panel C shows the interaction between the C' D loop and h317-Fab, and panel D shows the interaction between the FG loop and h317-Fab.

Figure 15 shows the interaction between the carbohydrate chain at N58 of PD-1 and the h317-Fab.

Figure 16 shows the binding of the h317 to the recombinant human PD-1-mut proteins.

Figure 17 includes panels A-B which show that h317-Fab competes with PD-L1 for binding to PD-1, in which panel A shows the structure comparison between the PD-1-h317-Fab complex and PD-1-PD-L1, and PD-1 is shown in grey, PD-L1 is shown in brick red, the heavy and light chains of the h317-Fab are shown in blue and green respectively; and panel B shows the comparison of interaction surfaces, and the surface of PD-1 is shown in grey, the portion shown in green is that interacted with the 317-Fab, the portion shown in brick red is that interacted with PD-L1, and the portion shown in yellow is that interacted with both the 317-Fab and PD-L1.

## DETAILED DESCRIPTION OF PREFERRED EMBODIMENTS

The present invention will be further described in detail in combination with the particular embodiments hereinafter. It will be appreciated by those skilled in the art that the embodiments provided are only used to illustrate the present invention, rather than limiting the scope of the present invention in any way.

Experimental methods in the following Examples are all conventional methods, unless particularly stated. Raw materials and reagents used in the following Examples are all products that commercially available, unless particularly stated.

**Example 1:** Screening and identification of hybridoma cell strains producing anti-human PD-1 antibodies and analysis of antibody sequences

Immunization: two immunization methods employing Freund's adjuvant and water-soluble adjuvant respectively were used for the immunization of mice. In the immunization method employing the Freund's adjuvant, the mice were immunized by intraperitoneal injection of the Freund's adjuvant twice on day 0 and day 14, and in the immunization method employing the water-soluble adjuvant, Balb/c mice aged 8-10 weeks were immunized by intramuscular injection of the water-soluble adjuvant twice on day 0 and day 21. The antigen used for immunization is: recombinant human PD-1/mFc protein (NCBI accession No.: NP_005009.2, 21aa-167aa, Lot: 2016.3.16), which was recombinantly expressed in HEK293 cells and provided by Beijing Kohnoor Science & Technology Co., Ltd. Dose for the first immunization was 50 μg, and dose for the second immunization was 25 μg. Sera of the mice taken before the immunization were served as negative controls for testing. Blood samples were taken from the tail veins of the mice on day 28 after primary immunization with Freund's adjuvant and on day 35 after primary immunization with water-soluble adjuvant, and then serum titers were measured by ELISA using a 96-well plate coated with recombinant human PD-1 protein. Mice whose serum titer met the requirements for fusion were boosted by intraperitoneal injection of 500 μl of 25 μg antigen diluted with D-PBS. Spleen cells from mice with high serum titers were collected on day 38 after the primary immunization for further cell fusion.

Cell fusion and preparation of the hybridoma: on day 38 after the primary immunization, blood was collected by enucleating the eyeball of the mouse whose serum titer met the requirements for fusion, then the serum isolated was used as positive control serum for antibody detection. Further, the spleen of the mouse was taken aseptically, a B lymphocyte suspension was prepared and mixed with FO myeloma cells in a ratio of 5:1 and the two kinds of cells were allowed to fuse under the action of PEG4000. The fused cells were resuspended in HAT medium and aliquoted into a 96-well plate containing macrophages. The plate was placed in an incubator at 37 °C and 5% $CO_2$ for 2 weeks, then the HAT medium therein was replaced with HT medium and the cells were cultured for another two weeks before replacing the HT medium with R1640 complete medium.

Screening for positive hybridomas: after 10-14 days of fusion, a plate was coated with recombinant human PD-1/His protein (NCBI accession No.: NP_005009.2, 21aa-167aa, Lot: 2016.2.22) (10 (μg/ml, pH 9.6, 0.1 M $NaHCO_3$) overnight at 4 °C. The plate was blocked with 4% skim milk powder-PBS for 2 hours at 37 °C, and was washed three times with PBST (0.05% Tween 20-PBS). Afterwards, the supernatants of the cultured hybridioma clones were added into the plate and incubated for 1 hour at 37 °C. Following controls were set: (1) positive control: serum of the mouse separated after immunization (diluted with PBS at a dilution of 1:1000); (2) negative control: serum of the mouse separated before immunization (diluted with PBS at a dilution of 1:1000); (3) blank control: PBS. Again the plate was washed three times with PBST (0.05% Tween 20-PBS), and HRP-Goat anti-mouse IgG (Fcγ) with a dilution of 1:20000 was added and incubated for 1 hour at 37 °C. Once again, the plate was washed five times with PBST (0.05% Tween 20-PBS), and chromogenic reagent OPD was added for developing color for 10-15 min in dark, then the reaction was terminated by adding 2 M $H_2SO_4$. Absorbances at 490 nm (A490) were read with a microplate reader. Clones in wells, which had A490 2.1 times greater than that of the negative control well, were regarded as positive ones. In order to determine the reliability of the positive clones, another screening was performed every other day after changing the medium used in the previous screening, and a total of three rounds of screening were performed. After detection and identification, multiple positive cell strains secreting antibodies were obtained and designated as Clones: 6, 317, 500, 763, 795, and 904 (Figure 1).

A plate was coated with recombinant human PD-1/His protein (NCBI accession No.: NP_005009.2, 21aa-167aa, Lot: 2016.2.22), cynomolgus monkey PD-1 (Cat: 90311-C08H, Sino Biological Inc.), rat PD-1 (Cat: 80448-R08H, Sino Biological Inc.) and mouse PD-1 (Cat: 50124-M08H, Sino Biological Inc.) in a concentration of 1 μg/mL overnight at 4 °C, washed three times with PBS subsequently, and 5% BSA-PBS was added for blocking for 60 min at 37 °C. The plate was washed three times with PBST afterwards, and supernatants of the hybridiomas diluted 100 times were added and incubated for 60 min at 37 °C. Again, the plate was washed four times with PBST, HRP-Goat anti-mouse IgG (Fcγ) (Cat: 115-035-071, Jackson Immuno Research) with a dilution of 1:5000 was added for a 30 min incubation at 37 °C. Once again, the plate was washed four times with PBST, and TMB substrate was added for developing color through incubating for 10 min at 37 °C, then the reaction was terminated by adding 2 M HCl. With 630 nm as reference wavelength, the absorbance at 450 nm (A450nm-630nm) of each well in the plate was read and recorded. Five supernatants of hybridoma cells (corresponding to Clone 317, 500, 763, 795, and 904) were used for detection of cross-reactivity with recombinant PD-1 of different species. Results obtained from ELISA showed that all antibodies in the five supernatants could specifically bind to the recombinant human PD-1 and cynomolgus monkey PD-1, but had no binding to the recombinant rat PD-1 and mouse PD-1, indicating that the antibodies have no cross-reactivity (Figure 2). Clone 317 was selected for the following sequence identification.

Hybridoma cells of Clone 317 secreting anti-human PD-1 antibody were expanded, and the subtype of the antibody was detected using Mouse Monoclonal Antibody IgG Subclass Test Card (Cat: A12403) and Mouse Monoclonal Antibody Light/Heavy Chain Test Card (Cat: A12401) following the protocols for the reagents. The subtype was identified as follows: the antibody was IgG1 for the heavy chain, and was Kappa for the light chain (Figure 3). The results provide a basis for the cloning of the genes of antibody 317.

Total RNA was extracted from the hybridoma cells of Clone 317 using TRIzol (Cat: 15596026, Invitrogen) following the steps described in the instructions. The total RNA of the hybridoma cells was reversely transcribed into cDNA using M-MuLV reverse transcriptase (CAT: M0253S, NEB). The sequences of light chain variable region IgVL (κ) and heavy chain variable region VH of the antibody were amplified using degenerate primers (see Tables 1 and 2) and Phusion kit (Cat: E0553L, NEB). The PCR products were purified with gel extraction kit (Cat: AP-GX-250, Axygen) and the purified PCR products were ligated to T vector following the instructions of the T vector cloning Kit (Cat: ZC205, ZOMANBIO) and then the vector was transformed into competent cells of E. coli for amplification. The plasmid was then extracted for DNA sequencing to obtain the variable region sequences of the monoclonal antibody. The sequencing results show that the light chain variable region of the antibody 317 has a nucleotide sequence as set forth in SEQ ID NO:53, and the amino acid sequence of the light chain variable region of the antibody 317 deduced from the DNA sequence is shown in SEQ ID NO:52; and the heavy chain variable region of the antibody 317 has a nucleotide sequence as set forth in SEQ ID NO:55, and the amino acid sequence of the heavy chain variable region of the antibody 317 deduced from the DNA sequence is shown in SEQ ID NO:54.

Table 1: Primers for the PCR of light chain

| | | |
|---|---|---|
| Upstream primer | MKV1 | ATGAAGTTGCCTGTTAGGCTGTTGGTGCTG ( SEQ ID NO:68 ) |
| | MKV2 | ATGGAGWCAGACACACTCCTGYTATGGGTG ( SEQ ID NO:69 ) |
| | MKV3 | ATGAGTGTGCTCACTCAGGTCCTGGSGTTG ( SEQ ID NO:70 ) |
| | MKV4 | ATGAGGRCCCCTGCTCAGWTTYTTGGMWTCTTG ( SEQ ID NO:71 ) |
| | MKV5 | ATGGATTTWCAGGTGCAGATTWTCAGCTTC ( SEQ ID NO:72 ) |
| | MKV6 | ATGAGGTKCYYTGYTSAGYTYCTGRGG ( SEQ ID NO:73 ) |
| | MKV7 | ATGGGCWTCAAGATGGAGTCACAKWYYCWGG ( SEQ ID NO:74 ) |
| | MKV8 | ATGTGGGGAYCTKTTTYCMMTTTTTCAATTG ( SEQ ID NO:75 ) |
| | MKV9 | ATGGTRTCCWCASCTCAGTTCCTTG ( SEQ ID NO:76 ) |
| | MKV10 | ATGTATATATGTTTGTTGTCTATTTCT ( SEQ ID NO:77 ) |
| | MKV11 | ATGGAAGCCCCAGCTCAGCTTCTCTTCC ( SEQ ID NO:78 ) |
| Downstream primer | MCK | TCATCAACACTCATTCCTgTTgAAgCTCTTgA ( SEQ ID NO:79 ) |

Table 2: Primers for the PCR of heavy chain

| | | |
|---|---|---|
| Upstream primer | MHV1 | ATGAAATGCAGCTGGGGCATSTTCTTC ( SEQ ID NO:80 ) |
| | MHV2 | ATGGGATGGAGCTRTATCATSYTCTT ( SEQ ID NO:81 ) |
| | MHV3 | ATGAAGWTGTGGTTAAACTGGGTTTTT ( SEQ ID NO:82 ) |

(continued)

| | | |
|---|---|---|
| | MHV4 | ATGRACTTTGGGYTCAGCTTGRTTT ( SEQ ID NO:83 ) |
| | MHV5 | ATGGACTCCAGGCTCAATTTAGTTTTCCTT ( SEQ ID NO:84) |
| | MHV6 | ATGGCTTGTCYTRGSGCTRCTCTTCTGC ( SEQ ID NO:85 ) |
| | MHV7 | ATGGRATGGAGCKGGRTCTTTMTCTT ( SEQ ID NO:86 ) |
| | MHV8 | ATGAGAGTGCTGATTCTTTTGTG ( SEQ ID NO:87) |
| | MHV9 | ATGGMTTGGGTGTGGAMCTTGCTATTCCTG ( SEQ ID NO:88 ) |
| | MHV10 | ATGGGCAGACTTACATTCTCATTCCTG ( SEQ ID NO:89 ) |
| | MHV11 | ATGGATTTTGGGCTGATTTTTTTTATTG ( SEQ ID NO:90 ) |
| | MHV12 | ATGATGGTGTTAAGTCTTCTGTACCTG ( SEQ ID NO:91 ) |
| Downstream primer | MIgG1CH1 | CTAACAATCCCTgggCACAATTTTCTTgTCCACC ( SEQ ID NO:92 ) |

**Example 2:** Preparation of anti-human PD-1 chimeric antibody

The light chain variable region gene and heavy chain variable region gene of the monoclonal antibody obtained through cloning, with cleavage sites of restriction enzymes introduced by PCR using the following primers (Table 3), were respectively cloned into the upstream of gene encoding human-kappa light chain constant region and the upstream of gene encoding the human IgG4 heavy chain constant region carried in eukaryotic expression vectors. The obtained human-mouse chimeric light chain expression plasmid (pKN019-317L) and human-mouse chimeric heavy chain expression plasmid (pKN034-317H) were then transformed into E. coli for amplification. A large number of plasmids carrying the human-mouse chimeric light chain and heavy chain were isolated, mixed with 293fectin and co-transfected into HEK293 cells. The cultured supernatant was collected 5-6 days after the transfection and purified with ProA affinity chromatography column, and chimeric antibody 317 was obtained. The affinity of the antibody was determined by the anti-human antibody capture assay with Fortebio (BLITZ pro1.1.0.28). For the determination, anti-Human IgG Fc Capture (AHC) biosensors were soaked in PBS for 10 min. 4 μl diluted antibody samples (chimeric antibody 317, Nivolumab; 20 μg/mL) were loaded onto two AHC biosensors respectively which were then equilibrated in PBS for 30 s. Subsequently, the AHC biosensors were reacted with the diluted recombinant human PD-1-His protein (NCBI accession No.: NP_005009.2, 21aa-167aa, Lot: 2016.2.22) of different concentrations (1 μM, 0.6 μM and 0 nM) for binding for 45 s, and was then transferred into PBS for dissociating for 120 s. When the experiment was finished, data from which response values of blank controls had been deducted was fitted using a 1: 1 Langmuir binding model by software, and then kinetic constants for antigen-antibody binding were calculated.

Figure 4 shows the kinetic curve of chimeric antibody 317 (Fig. 4, 4A) and control antibody Nivolumab (Fig. 4, 4B) binding with recombinant human PD-1. Affinities were calculated by fitting the curves, and results showed that the affinity (KD) of chimeric antibody 317 was 1.02E-09M and the affinity (KD) of Nivolumab was 1.01E-09M. See Table 4 for kinetic parameters in detail. The results showed that chimeric antibody 317 had a high affinity to human PD-1, and under the same conditions, the dissociation constant of the chimeric antibody 317 was similar to that of Nivolumab. The results provide a theoretical basis for the humanization of antibody 317 and the inhibitory effect of 317 on PD-1/PD-L1 signaling pathway.

Table 3: primers for cloning the light chain and the heavy chain variable regions of the antibody 317

| | | | |
|---|---|---|---|
| Primers for cloning the light chain variable region | Upstream primer | hM-h15 | CTCAAGCTTAATTGCCGCCACCATG ( SEQ ID NO:93 ) |
| | | SP-317L-F | CCTGGAGCCATCGGAGACATTGTGATGACC (SEQ ID NO: 94) |
| | Downstream primer | SP-317L-R | GGTCATCACAATGTCTCCGATGGCTCCAGG ( SEQ ID NO: 95 ) |
| | | 317L-R | GCTGGCGCCGCATCAGCCCGTTTGATTTC ( SEQ ID NO: 96 ) |

(continued)

| | | | |
|---|---|---|---|
| Primers for cloning the heavy chain variable region | Upstream primer | hM-h15 | CTCAAGCTTAATTGCCGCCACCATG (SEQ ID NO:97) |
| | | SP-317H-F | GAAGGGCGTGCAGTGCGAAGTGAAGCTGGTG ( SEQ ID NO:98 ) |
| | Downstream primer | SP-317H-R | CACCAGCTTCACTTCGCACTGCACGCCCTTC ( SEQ ID NO: 99 ) |
| | | 317H-R | GCGCTAGCTGCAGAGACAGTGACCAGAG ( SEQ ID NO: 100 ) |

Table 4: Detection results of the affinity of the chimeric antibody to the recombinant human PD-1 extracellular domain protein

| | KD (M) | kon (1/Ms) | kd (1/s) |
|---|---|---|---|
| 317 | 2.64E-09 | 2.15E+05 | 5.66E-04 |
| Nivolumab | 2.018E-09 | 3E+05 | 6.05E-04 |

**Example 3:** Humanization of the anti-human PD-1 monoclonal antibody and construction of a stable cell strain

Firstly, a comprehensive analysis was conducted on the heavy chain sequence of the mouse antibody for determining the antigen complementary determinant regions (CDRs) in the antibody that bind with the antigen and the framework regions supporting the conserved three-dimensional conformation of the antibody. The closest human antibody matches were sought in human antibody germline library (http://www2.mrc-1mb.cam.ac.uk/vbase/alignments2.php#VHEX) according to results of homologous comparison. VH3 (3-21) was finally selected as the basic template, and CDR grafting was performed with considering the frequency of amino acid at specific position of FR region (A49) in the rearranged antibody in combination with blast results of complete sequence. Furthermore, S98 in the FR3 of the mouse antibody was not replaced since it was close to the CDR3 region. Meanwhile, JH4 (wgqgtlvtvss) was selected as the sequence for J region according to the sequence of CDR3 (Pdssgvay). As a result, a high humanization of the frame region in HI was achieved. In addition, the closest human antibody matches were sought in human antibody germline library (http://www2.mrc-lmb.cam.ac.uk/vbase/alignments2.php#VHEX) according to results of homologous comparison. VK II (A19) was finally selected as the basic template , and CDR grafting was performed with considering the frequency of amino acid at specific position of FR region (R45) in the rearranged antibody in combination with blast results of complete sequence. Meanwhile, JK1 (fgqgtkveik) was selected as the sequence for JK region according to the sequence of CDR3 (Qqysrypw). Thus, the fully humanization of light chain frame region was achieved.

Fully humanized design was made on the sequence of murine origin antibody 317 and the obtained humanized sequence was fully synthesized, and the antibody obtained by the humanization of the antibody 317 was named h317. Further, expression vectors expressing the light chain and the heavy chain of the h317 were constructed by cloning the humanized h317-VH1 into upstream of gene encoding heavy chain constant region of human IgG4 carried in the eukaryotic expression vector pKN034 through enzyme digestion, and cloning the humanized h317-VL2 into upstream of gene encoding CK of human light chain carried in the eukaryotic expression vector pKN019 through enzyme digestion. Subsequently, through enzyme digestion, the obtained heavy chain of h317 (h317-H) and light chain of h317 (h317-L) were cloned into the eukaryotic expression vector pKN002. The recombinant vectors were then linearized with Pvu I, and genes of h317 were integrated into the DNA of CHO cells using Nucleofector 2B (300452, Lonza). The CHO cells were screened by MSX (Cat: M5379-500MG, Sigma) and subcloned before finally obtaining a stable cell strain highly expressing h317. The nucleotide sequence of the light chain variable region of h317 is as set forth in SEQ ID NO:61, and the amino acid sequence thereof is as set forth in SEQ ID NO:60; the nucleotide sequence of the heavy chain variable region of h317 is as set forth in SEQ ID NO:63, and the amino acid sequence thereof is as set forth in SEQ ID NO:62.

**Example 4:** Kinetic research on binding of h317 to human PD-1 protein

Biotinylation of h317 and Nivolumab: 200 μL of 5 mg/ml EZ-LinkNHS-LC-LC-Biotin (Cat: 21343, Thermo) was placed at RT for 5 min and then mixed with 1 ml of 1 mg/mL h317 (Lot: DP201805002, Jiangsu T-mab BioPharma) or Nivolumab (Lot: AAW4553, BMS). The obtained mixture was placed at RT for 30 min, and then was concentrated by ultrafiltration to 0.5 mL. After determined at 280 nm for the protein content therein, the protein concentrate was aliquoted, and stored at -80°C, with frozen and thawed not more than once.

Determination of the binding kinetics: the affinity of the antibody was determined with Octet QKe system from Fortebio using an assay of capturing biotin-labeled antibody with streptavidin. Specifically, the biotin-labeled antibodies (h317-biotin, and Nivolumab-biotin) were diluted to 5 μg/mL with PBS, and passed through the surface of streptavidin pre-coated biosensors (Cat: 1612101, PALL) for 120 s. The recombinant human PD-1 protein (NCBI accession No.: NP_005009.2, 21aa-167aa) as the mobile phase was diluted in PBS serially to get two-fold serial dilutions, covering a concentration range of 100 nM -12.5 nM. Both the binding and dissociating were conducted for 300 s. When the experiment was finished, data from which response values of blank controls had been deducted was fitted using a 1: 1 Langmuir binding model by software, and then kinetic constants for antigen-antibody binding were calculated.

Figure 5 shows the kinetic curve of h317 (Fig. 5, 5A) and control antibody Nivolumab (Fig. 5, 5B) binding with recombinant human PD-1 protein. Affinity were calculated by fitting the curves, and results showed that the affinity (KD) of h317 was 6.16E-09M and the affinity (KD) of Nivolumab was 8.06E-09M. See Table 5 for kinetic parameters in detail. The results showed that h317 had a high affinity to human PD-1, and under the same conditions, the dissociation constant of h317 was lower than the dissociation constant of Nivolumab. The results provide a basis for the inhibitory effect of h317 on PD-1/PD-L1 signaling pathway.

Table 5: Detection results of the affinity of the h317 to the recombinant human PD-1 extracellular domain protein

| | KD (M) | kon (1/Ms) | kd (1/s) |
|---|---|---|---|
| h317 | 6.16E-09 | 1.54E+05 | 9.50E-04 |
| Nivolumab | 8.06E-09 | 2.09E+05 | 1.69E-03 |

**Example 5:** Detection of inhibitory effect of h317 on binding of PD-1 to its ligands PD-L1 and PD-L2 in ELISA

Detection of inhibitory effect of h317 on binding of PD-1 to PD-L1 in ELISA: a plate was coated with human PD-1-hFc (NCBI accession No.: NP_005009.2, 21aa-167aa, Lot: 20180329) having a dilution concentration of 0.5 μg/mL overnight at 4 °C, and was then blocked with 5% BSA in a constant temperature incubator at 37 °C for 60 min. h317 (Lot: DP201805002, Jiangsu T-mab BioPharma) or Nivolumab (Lot: AAW4553, BMS) (1.5-fold serial dilutions with an initial concentration of 3 μg/mL) was subsequently added for reacting in the constant temperature incubator at 37 °C for 120 min. 1 μg/mL PD-L1-mFc (NCBI accession No.: NP_054862.1, 19aa-238aa, Lot: 20180412) was added to the plate for incubating with the antibodies in the constant temperature incubator at 37 °C for 60 min. Afterwards, HRP-anti-mouse Fc (Cat: 115-035-071, Jackson Immuno Research) which was diluted at a dilution of 1: 5000 was added to the plate for reacting for 45 min. Later, TMB substrate (Cat: ME142, Beijing Taitianhe Biotech Co., Ltd.) was added to develop color for 15 min, which was followed by the addition of 2 M HCl to terminate the reaction and then the plate was detected by a microplate reader. With 630 nm as reference wavelength, the absorbance at 450 nm (A450nm-630nm) of each well in the plate was read and recorded.

Detection of inhibitory effect of h317 on binding of PD-1 to PD-L2 in ELISA: a plate was coated with PD-1-hFc (NCBI accession No.: NP_005009.2, 21aa-167aa, Lot: 20180329) having a dilution concentration of 0.5 μg/mL overnight at 4 °C, and was then blocked with 5% BSA in a constant temperature incubator at 37 °C for 60 min. h317 (Lot: DP201805002, Jiangsu T-mab BioPharma) or Nivolumab (Lot: AAW4553, BMS) (1.5-fold serial dilutions with an initial concentration of 3 μg/mL) was subsequently added for reacting in the constant temperature incubator at 37 °C for 120min. 2 μg/mL PD-L2-hFc-Biotin (NCBI accession No.: NP_079515.2, 20aa-220aa, Lot: 2016.12.05) was added to the plate for incubating with the antibodies in the constant temperature incubator at 37 °C for 60 min. Afterwards, HRP-streptavidin (Cat: S2438, Sigma) which was diluted at a dilution of 1: 2000 was added to the plate for reacting for 30 min. Later, TMB substrate was added to develop color for 15 min, which was followed by the addition of 2 M HCl to terminate the reaction and then the plate was detected by a microplate reader. With 630 nm as reference wavelength, the absorbance at 450 nm (A450nm-630nm) of each well in the plate was read and recorded.

The results showed that h317 could block the binding between the recombinant human PD-1 and its ligands PD-L1 and PD-L2 effectively. The competitive inhibitory effects of h317 and Nivolumab on the binding of PD-1 to its ligand PD-L1 were determined by ELISA, and the obtained half maximal inhibitory concentrations (IC50) were 1.4 nM and 1.3 nM respectively (Figure 6, 6A). Meanwhile, the competitive inhibitory effects of h317 and Nivolumab on the binding of PD-1 to its ligand PD-L2 were determined by ELISA, and the obtained half maximal inhibitory concentrations (IC50) were 1.2 nM and 1.0 nM respectively (Figure 6, 6A). It can be seen from the experiments that h317 has a similar blocking activity to Nivolumab.

**Example 6:** Research on species-specificity of binding of h317 to PD-1 by ELISA

ELISA plates were coated with recombinant human PD-1 (NCBI accession No.: NP_005009.2, 21aa-167aa, Lot: 20180423), cynomolgus monkey PD-1 (Cat: 90311-C08H, Sino Biological Inc.), rat PD-1 (Cat: 80448-R08H, Sino Biological Inc.) and mouse PD-1 (Cat: 50124-M08H, Sino Biological Inc.) proteins in a concentration of 1 μg/mL respectively overnight at 4 °C. The plates were washed three times with PBS subsequently, and 5% BSA-PBS was added for blocking for 60 min at 37 °C. Then the plates were washed three times with PBST, and biotinylated h317 (the same as that used in Example 4) with different dilutions (11 concentrations were obtained by diluting serially in a 3-fold gradient dilution with an initial concentration of 1 μg/mL) was added and incubated for 60 min at 37 °C. Again, the plates were washed four times with PBST, then HRP-streptavidin (Cat: 019-035-098, Jackson Immuno Research) with a dilution of 1:100 was added for a 30 min incubation at 37 °C. Once again, the plates were washed four times with PBST, and TMB substrate was added for developing color through incubating for 10 min at 37 °C, then the reaction was terminated by adding 2 M HCl. With 630 nm as reference wavelength, the absorbance at a wavelength of 450nm (A450nm-630nm) of each well in the plates was read and recorded.

The results showed that h317 could specifically bind to recombinant human PD-1 and cynomolgus monkey PD-1 in a half maximal effective concentration (EC50) of 0.004703 nM and 0.01823 nM respectively, while had no binding affinity to rat PD-1 and mouse PD-1, indicating that h317 has no cross-reactivity (Figure 7). The results provide a basis for the in vivo and *in vitro* pharmacodynamic experiments of h317.

**Example 7:** Research on specificity of binding of h317 to members of the CD28 family

ELISA plates were coated with recombinant human PD-1 (NCBI accession No.: NP_005009.2, 21aa-167aa, Lot: 20180423), CD28 (Cat: 11524-HCCH, Sino Biological Inc.), CTLA4 (Cat: 11159-H08H, Sino Biological Inc.) and ICOS (NCBI accession No.: NP_036224.1, 1aa-199aa) proteins in a concentration of 1 μg/mL respectively overnight at 4 °C. The plates were washed three times with PBS subsequently, and 5% BSA-PBS was added for blocking for 60 min at 37 °C. Then the plates were washed three times with PBST, and biotinylated h317 (the same as that used in Example 4) with different dilutions (11 concentrations were obtained by diluting in a 3-fold gradient dilution with an initial concentration of 1 μg/mL) was added and incubated for 60 min at 37 °C. Again, the plates were washed four times with PBST, then HRP-streptavidin (Cat: 019-035-098, Jackson Immuno Research) with a dilution of 1:100 was added for a 30 min incubation at 37 °C. Once again, the plates were washed four times with PBST, and TMB substrate was added for developing color through incubating for 10 min at 37 °C, then the reaction was terminated by adding 2 M HCl. With 630 nm as reference wavelength, the absorbance at a wavelength of 450nm (A450nm-630nm) of each well in the plates was read and recorded.

The results showed that h317 could specifically bind to recombinant human PD-1, while had no binding affinity to other members of the CD28 family, indicating that h317 has no cross-reactivity (Figure 8). The results provide a basis for the *in vivo* and *in vitro* pharmacodynamic experiments of h317.

**Example 8:** Research on activity of h317 in stimulating human T cells (PBMCs) in vitro

A. Isolation of CD14+ monocytes and differentiation of Dendritic Cells

PBMCs were extracted using Ficoll-Paque PLUS (Cat: 17-1440-03, Lot: 10246684, GE Healthcare) following the manufacturer's instructions and then CD14+ monocytes were positively selected with CD14 MicroBeads (Cat: 130-050-201, Lot: 5170814438, Miltenyi) following the manufacturer's instructions. The obtained cells were resuspended to $5\times10^5$ cells/ml with ImmunoCult™ DC Differentiation Medium (Cat: 10988, Lot: 17G83035, STEMCELL), and then 5 ml suspension was taken and inoculated into a T-25 flask, and cultured in an incubator at 37 °C, 5% $CO_2$ for 3 days. The culture was centrifuged and new medium was added for a 2 day culture in the incubator at 37 °C, 5% $CO_2$. Then 50 μl ImmunoCult™ Dendritic Cell Maturation Supplement (Cat: 10989, Lot: 17B77271, STEMCELL) was added, mixed and the cells were cultured in the incubator at 37 °C, 5% $CO_2$ for 2 days. Cells obtained were used for the following MLR assay.

B. Extraction and isolation of CD4+ T cells

PBMCs were extracted using Ficoll-Paque PLUS (Cat: 17-1440-03, Lot: 10246684, GE Healthcare) following the manufacturer's instructions, and then CD4+ T cells were isolated from the PBMCs using CD4+ T cells isolation kit (Cat: 130-096-533, Lot: 5171016623, Miltenyi) according to the instructions in the kit. Cells obtained were used for the following MLR assay.

C. MLR assay

Solutions of the antibodies (h317 (Lot: DP201805002, Jiangsu T-mab BioPharma)), Nivolumab (Lot: AAW4553, BMS), and Human NC-IgG4 control (Lot: AB170090) to be tested at 8 μg/mL were prepared respectively with a Complete Medium, and then were diluted serially in a 10-fold gradient dilution and a total of 6 concentrations were prepared. Later, 50 μL of each of the solutions containing the antibodies was added to corresponding wells of a 96-well plate according to the setup for the plate used in the experiment. Subsequently, 100 μL suspension of CD4+ T cells with an adjusted concentration of $1\times10^6$ cells/mL was added to the corresponding wells ($1\times10^5$ cells/well). DCs were digested with 2 mM EDTA, centrifuged at 1500 rpm for 5 min, and 50 μL suspension of the DCs with an adjusted concentration of $2\times10^5$ cells/mL was added to the corresponding wells ($1\times10^4$ cells/well). Then, the total volume of the mixture in one well was 200 μL, and the ratio of CD4+ T cells to DCs was 10:1. Then the 96-well plate was placed in an incubator for culturing for 5 days at 37 °C. Cell supernatants obtained thereby were collected and concentrations of IFN-γ and IL-2 therein were detected using IFN-γ ELISA kit (Cat: 430106, Lot: B247782, Biolegend) and IL-2 ELISA kit (Cat: DY202, Lot: P155804, R&D).

The detection results showed that h317 could stimulate human mixed lymphocytes and promote the cells to secret killer cytokines IL-2 (Figure 9, 9A) and IFN-γ (Figure 9, 9B) *in vitro,* which suggests that h317 can induce the immune response mediated by Th1 type cells.

**Example 9:** Research on the ADCC activity of h317 on 293T/hPD-1 cells

PBMCs were extracted using Ficoll-Paque PLUS (Cat: 17-1440-03, Lot: 10246684, GE Healthcare) following the manufacturer's instructions, and then resuspended in a medium for ADCC assay (phenol-red-free RPMI 1640 medium (Cat: 11835-030, Lot: 1860152, Gibco) containing 2% FBS (Cat: SH30084.03, Lot: GAE0138, Hyclone)). PBMCs obtained were counted, and NK cells, which were subsequently used in the analysis of ADCC, were isolated according to the instructions of the NK Cell Isolation Kit (Cat: 130-092-657, Lot: 5170911524, Miltenyi). 293T cells expressing human PD-1 at the exponential phase were taken, and according to CytoTox 96® Non-Radioactive Cytotoxicity Assay (Cat: G1781, Lot: 0000265175, Promega), the 293T cells were then diluted with the medium for ADCC to obtain a cell suspension containing the target cells in a concentration of $50\times10^4$ cells/mL. For each well in a 96-well round bottom plate, 40 μL of the cell suspension and 10 μL of solutions of antibodies to be tested (each concentration of the antibodies to be tested was assayed in triplicate) were added and then incubated at room temperature for 30 min. Final concentrations of the antibodies to be tested (h317 (Lot: DP201805002, Jiangsu T-mab BioPharma), h317-hIgG1 (Lot: 20180528), and Human NC-IgG4 (Lot: 20180521)) were 10000 ng/mL, 1000 ng/mL, 100 ng/mL, 10 ng/mL, 1 ng/mL, 0.1 ng/mL and 0.01 ng/mL. Further, two E:T ratios of 10:1 and 5:1 were set by adding 50 μL of NK cell suspensions in different concentrations to each well. Later, the 96-well plate was centrifuged at 250 g for 4 min to allow a sufficient contact between the effector cells and the target cells before incubating the cells for 4 h at 37 °C, 5% $CO_2$/95% air. 10 μL lysis buffer (10 μl per 100 μl) was added to the max lysis control well 45 min, before the plate was centrifuged at 250 g for 5 min and supernatants were transferred. Supernatants of 50 μL from the wells were transferred to a clean 96-well plate, and to each well 50 μl reaction substrate was added, mixed for 30 s and the plate was incubated at room temperature for 30 min. Following, 50 μL termination solution was added to the plate and mixed for 30 s. Absorbances (OD490nm) were read.

The average value of the control wells containing the culture medium was subtracted from that of the test wells, that of the control wells containing target cells, and that of the wells containing NK cells and target cells. % cytotoxiticy was calculated using values obtained according to the following formula.

$$\%\ \text{Cytotoxicity} = \frac{\text{Experimental release} - \text{Ave (Target + Effector control)}}{\text{Ave (Target maximum)} - \text{Ave (Target control)}} \times 100\%$$

Experimental results showed that the killing effect of NK cells in human PBMCs (peripheral blood mononuclear cells) on target cells was not initiated by the binding of h317 to hPD-1 on 293T cells (Figure 10). Compared with the control group without anti-hPD-1 antibody, h317 showed no ADCC activity against 293T cells with high hPD-1 expression, indicating that h317 has no ADCC-effector function.

**Example 10:** Evaluation of anti-tumor efficacy of h317 in PD-1 transgenic mouse xenograft tumor model

A total of 65 HuGEMMPD-1 mice (aged 6-8 weeks, Shanghai Model organisms) were selected and inoculated subcutaneously with MC38-hPD-L1 tumor cells ($1\times10^6$ cells/mouse, $1\times10^6$ cells resuspended in 100 μL PBS) on the right side. When the average tumor volume of the tumor-bearing mice reached about 60-100 $mm^3$, each mouse was weighted, and the tumor volume was measured with a vernier caliper. The mice were randomly divided into 6 groups using StudyDirector™ (version 3.1.399.19, supplied by Studylog System, Inc., S. San Francisco, CA, USA) before administration. The administration was started at day 0 (D0), and Table 6 showed the information about number of animals in each group as well as the routes, doses and protocols of the administration in detail. The administration, measurement of the tumor volume, weighting and the like were all performed in a biosafety cabinet or clean bench. During the experiment, experimental data was collected with the software StudyDirector™ (version 3.1.399.19, supplied by Studylog System, Inc.), including measuring the long and short diameters of tumors as well as weighting the mice, and initial data was directly imported into the software after obtained by measuring using a balance and a vernier caliper. The experiment was terminated when the mean volume of the tumors in the control group exceeded 2000 $mm^3$ or one week after the last administration. The tumors were collected, weighted and taken pictures. FACS assay was performed using tumors sampled on the day the experiment ended, from 4 mice of each group (the mice had numbers corresponding to those of the mice whose blood was sampled below), and markers were: CD3, CD4, CD8, and CD45, Live/Dead. Another FACS assay was performed using whole blood sampled on the day the experiment ended, from 4 mice of each group (the mice had numbers corresponding to those of the mice whose tumors were sampled), and markers were: CD3, CD4, CD8, and CD45.

Table 6: Design for the pharmacodynamics experiment

| Group | Number of mice | Drug administrated | Dose (mg/kg) | Administration route | Administration cycle |
|---|---|---|---|---|---|
| 1 | 8 | hIgG control | 10 | i.p. | BIW×3 weeks |
| 2 | 8 | h317-H | 10 | i.p. | BIW×3 weeks |
| 3 | 8 | h317-M | 2 | i.p. | BIW×3 weeks |
| 4 | 8 | h317-L | 0.5 | i.p. | BIW×3 weeks |
| 5 | 8 | Nivolumab-L | 2 | i.p. | BIW×3 weeks |
| 6 | 6-8 (TBD) | Nivolumab-H | 10 | i.p. | BIW×3 weeks |
| Note: volume for administration was 10 μL/g; hIgG control (Lot: 20180521), h317 (Lot: DP201805002, Jiangsu T-mab BioPharma), Nivolumab (Lot: AAW4553, BMS). | | | | | |

HuGEMMPD-1 mouse model, which is a kind of genetically engineered mouse, is established by inserting the coding region of human PD-1 protein into the ATG position in the mouse PD-1 under the genetic background of C57BL/6J, such that the mouse expresses human PD-1 instead of mouse PD-1. MC38 is a murine intestinal cancer cell line induced in C57BL/6 mouse. MC38 cell line expressing human PD-L1, named as MC38-hPD-L1 cell line, is obtained by knocking out the murine PD-L1 of the MC38 and knocking in the human PD-L1 using genetic engineering. With the PD-1 transgenic mouse xenograft tumor model, the inhibitory effects of h317 and control antibody Nivolumab on tumor growth were evaluated and compared. The results (see Figure 11 and Table 7) showed that, h317 could significantly inhibit the growth of subcutaneous xenograft tumor in PD-1 transgenic mice at high dose and medium dose, and an inhibitory effect on tumor comparable to that of the control antibody Nivolumab was observed with h317 at high and medium doses.

Table 7: Tumor volume and statistical analysis thereof of subcutaneous xenograft tumors in PD-1 transgenic mice of each group

| Number | Treatment | D0 tumor volume ($mm^3$)[a] | D11 tumor volume ($mm^3$)[a] | T/C (RTV) % | TGI | P |
|---|---|---|---|---|---|---|
| | | | | | (%) | Value[b] |
| 1 | hIgG control, 10 mg/kg BIW*4 times, i.p. | 68.21±9.16 | 2600.18±178.98 | - | - | - |
| 2 | h317-H, 10 mg/kg, BIW*4 times, i.p. | 67.90±8.74 | 76.84±33.94 | 2.96 | 97.04 | <0.001 |

(continued)

| Number | Treatment | D0 tumor volume ($mm^3$)[a] | D11 tumor volume ($mm^3$)[a] | T/C (RTV) % | TGI | P |
|---|---|---|---|---|---|---|
| | | | | | (%) | Value[b] |
| 3 | h317-M, 2 mg/kg, BIW*4 times, i.p. | 68.09±8.40 | 354.89±157.70 | 11.25 | 88.75 | <0.001 |
| 4 | h317-L, 0.5 mg/kg, | 67.51±7.62 | 2581.85±230.58 | 101.60 | -1.60 | 1.000 |
| | BIW*4 times, i.p. | | | | | |
| 5 | Nivolumab-L, 2 mg/kg, BIW*4 times, i.p. | 67.17±8.05 | 594.63±212.98 | 23.72 | 76.28 | <0.001 |
| 6 | Nivolumab-H, 10 mg/kg, BIW*4 times, i.p. | 67.75±7.83 | 84.82±66.02 | 2.31 | 97.69 | <0.001 |
| Note: a. the data was shown as "mean ± standard error"; n=8 | | | | | | |

b. P value was obtained by using one way ANOVA of tumor volume, and F value analyzed by Dunnett's T3 was significantly different (P <0.05).

**Example 11:** Binding to PD-1 receptor and internalization of h317

Construction of 293 cell line transiently expressing human PD-1: 10 ml of HEK293 cell suspension was inoculated into a shaking flask at a density of $6*10^5$ cells/mL, and at 37 °C, 5% $CO_2$, shaken and cultured in a shaking incubator at 130 rpm for 24 h. An expression plasmid encoding full-length human PD-1 (NP_005009.2, 21aa-288aa) and 293fectin (Cat:12347019, Gibco) in a ratio of 1: 1.2 were respectively let stand in 0.5 mL Opti-MEM (Cat: 11058021, Gibco) for 5 min, then mixed and let stand for 15 min at room temperature before added to the HEK293 cells. After a 48 h shaking culture at 130 rpm in a shaking incubator at 37 °C, 5% $CO_2$, the obtained 293/hPD-1 cells were used for detection via FACS.

Endocytosis of the h317 mediated by PD-1: h317 (Lot: DP201805002, Jiangsu T-mab BioPharma) and Nivolumab (Lot: AAW4553, BMS) were labeled with Mix-n-Stain™ CF™ 488A (Cat: MX488AS100, Sigma); and the labeled antibodies (h317-CF488A and Nivolumab-CF488A) were diluted to 10 μg/mL with 5% BSA. The 293/hPD-1 cells were washed one time with PBS, and then the diluted h317-CF488A and Nivolumab-CF488A were added thereto respectively. Each mixture was divided into two groups, one was placed in an electro-heating standing-temperature cultivator at 37 °C and the other was placed in a refrigerator at 4°C as a negative control. The cells were washed three times with PBS after 1 h incubation, and placed back for incubation at 37 °C or 4°C. The cells were observed and taken pictures with a fluorescence microscope after incubation for 3 hand incubation for 6 h.

Endocytosis of the h317 mediated by PD-1 was observed with a fluorescence microscope and the results (Figure 12) showed that at 37 °C, both h317 and Nivolumab could be endocytosed through mediating by PD-1.

**Example 12:** Study on epitopes recognized by 317

Preparation of PD-1-h317-Fab complex: Molecular weight of the recombinant human PD-1 extracellular domain protein (NCBI accession No.: NP_005009.2, 21aa-167aa, Lot: 20170626) is 16.4 Kd, and molecular weight of h317-Fab (Lot: 20170626) is about 46.5 Kd. A mixture was obtained by mixing the PD1 and h317-Fab in 20 mM Tris 150 mM NaCl at a molar ratio of 1: 1.5, and was then placed on ice for 30 min to form a complex. The complex was concentrated by ultrafiltration to a concentration of 12 mg/ml for crystallization analysis.

Crystallization of PD-1-h317-Fab complex and diffraction data collection and structure analysis of the obtained crystals: this experiment was accomplished by the sitting drop method using Automated Crystallization Screening System. 0.2 μl protein sample was added to each well of the crystallization plate equipped in the system, and when the addition was completed, the crystallization plate was sealed and placed in a constant temperature chamber for culture. The temperature for crystal growth was 16 °C or 4 °C, and the protein concentration added was 8 mg/ml or 12 mg/ml. Kits used for the screening included: Crystalscreen (1-98), Index (1-96), PEGRX (1-96), SaltRx (1-96), Nartrix (1-96), and PEG/ion (1-96) supplied by HAMPTON; Protein Complex Suite (1-96) supplied by QIAGEN; WIZARD I/II (1-96), and WIZARD III/IV (1-96) supplied by Emerald Biosystems. Crystal growth was observed for 15 days and the growth conditions under which better crystals formed in a preliminary screening were optimized. Before the diffraction experiment, crystals obtained under the optimized conditions were immersed in a crystallization solution supplemented with 10% ethylene glycol for a few seconds, then immersed in liquid nitrogen for a quick freezing. The diffraction experiment was performed at BL17U in Shanghai Synchrotron Radiation Facility (SSRF) using a X-ray wavelength of 0.9792 Å and a crystal diffraction temperature of 100 K. Diffraction data was collected using Q315r detector supplied by ADSC, and was then processed using HKL2000 software. The crystals were determined belonging to P2 space group, and diffracted to a resolution of 2.90 Å. Crystal structure was analyzed through molecular replacement method using PHASER software, and the models used for the molecular replacement were PD-1 single protein structure (PDB ID 3RRQ) and PD-1 Fab (PDB ID 5WT9) structure. Subsequently, COOT software was used to rebuild the model, and Refmac5 and Phenix softwares were used for structure refinement.

Binding between deglycosylated recombinant human PD-1 protein and h317: Four glycosylation sites N49, N58, N116, and N74, which can be observed in the structure of human PD-1 antigen (NP_005009.2), were mutated to alanine (A) by site-directed mutation. The mutants obtained were inserted into an expression vector carrying EGFP to construct five plasmids expressing full-length human PD-1 (WT, N49A, N58A, N116A, N74A). Later, the plasmids were mixed with 293fectin and co-transfected into HEK293 cells. 2 days after the transfection, the cells were collected to a 96-well plate, centrifuged at 1500 rpm for 2 minutes, and the supernatants in the wells were discarded. The cells were resuspended by adding 200 μl PBS and the suspensions obtained were then centrifuged at 1500 rpm for 2 minutes, then supernatants were discarded. 200 μl of each primary antibody (h317 (Lot: 2017.05.24) or Nivolumab (Lot: 2017.04.26)) diluted with 5% BSA was added to the cells to incubate for 1 h at room temperature. The working concentration of the primary antibodies was 2 μg/ml, and cells without primary antibodies added were used as negative controls. The mixtures obtained were centrifuged at 1500 rpm for 2 minutes, and supernatants were discarded. The cells in each well were resuspended by adding 200 μl PBS and the suspensions obtained were then centrifuged at 1500 rpm for 2 minutes, then supernatants were discarded. Later, the obtained cells were incubated with 200 μl goat anti-human IgG(Fc)-Cy5 (Cat: 12136, Lot: 017M4800V, Sigma) diluted with 5% BSA added into each well for 1 h at room temperature, then centrifuged at 1500 rpm for 2 minutes, and supernatants were discarded. The cells were resuspended by adding 200 μl PBS and the suspensions obtained were then centrifuged at 1500 rpm for 2 minutes, then supernatants were discarded. The cells again were resuspended by adding 200 μl PBS thereto and detected by flow cytometry (B49007AD, SNAW31211).

The overall structure analysis of the PD-1-h317-Fab complex showed that the PD-1 antigen (extracellular region 1-167aa) existed as a monomer, and formed a complex with the h317-Fab fragment of the anti-PD-1 antibody in a ratio of 1:1 (Figure 13). The sites of PD-1 bound by the antibody were mainly located in the loops of PD-1, including BC loop, C' D loop and FG loop (Figure 14). Interactions between PD-1 and Fab317 included hydrogen bond, salt bridge, van der waals interaction, and hydrophobic interaction. Table 8 shows amino acids involved in the interactions and the corresponding interactions thereof.

Table 8: amino acids involved in the interactions between PD-1 and Fab317

| PD-1 | | Heavy chain | | Light chain | | Interaction |
|---|---|---|---|---|---|---|
| Region | Residue | Region | Residue | Region | Residue | |
| BC loop | T59 | CDR2 | Y57 | | | Hydrogen bond |
| | S60 | CDR2 | Y57 | | | Van der Waals interaction |
| | E61 | CDR1 | D33 | | | Hydrogen bond (via water) |
| | | CDR2 | S52 | | | Hydrogen bond |
| | | CDR2 | G53 | | | Hydrogen bond (via water) |
| | | CDR2 | G54 | | | Hydrogen bond |
| | | CDR2 | G55 | | | Hydrogen bond |
| | | CDR2 | S56 | | | Hydrogen bond |
| | S62 | CDR1 | D33 | | | Hydrogen bond |
| | V64 | CDR3 | S101 | | | Van der Waals interaction |
| C'D loop | F82 | CDR1 | S31 | | | Van der Waals interaction |
| | P83 | CDR1 | S31 | | | Van der Waals interaction |
| | | CDR1 | Y32 | | | Van der Waals interaction |
| | E84 | CDR1 | Y32 | | | Hydrogen bond |

(continued)

| PD-1 | | Heavy chain | | Light chain | | Interaction |
|---|---|---|---|---|---|---|
| Region | Residue | Region | Residue | Region | Residue | |
| | R86 | N-ter | V2 | | | Hydrophobic interaction |
| | | CDR1 | F27 | | | Van der Waals interaction |
| | | CDR1 | Y32 | | | Van der Waals interaction |
| | | CDR3 | S98 | | | Hydrogen bond |
| | | CDR3 | D100 | | | Salt bridge |
| | | CDR3 | Y106 | | | Hydrophobic interaction |
| | S87 | CDR3 | Y106 | | | Van der Waals interaction |
| | | | | CDR2 | H55 | Van der Waals interaction |
| | | | | CDR2 | T56 | Van der Waals interaction |
| | Q88 | | | CDR2 | T56 | Van der Waals interaction |
| FG loop | I126 | CDR3 | S101 | | | Van der Waals interaction |
| | | | | CDR2 | W50 | Hydrophobic interaction |
| | L128 | CDR1 | D33 | | | Van der Waals interaction |
| | | CDR3 | D100 | | | Van der Waals interaction |
| | | CDR3 | S101 | | | Van der Waals interaction |
| | | | | CDR3 | Y91 | Hydrophobic interaction |
| | | | | CDR3 | Y94 | Van der Waals interaction |
| | | | | CDR3 | W96 | Van der Waals interaction |
| | A129 | CDR2 | Y59 | | | Hydrophobic interaction |
| | | | | CDR3 | Y94 | Hydrophobic interaction |
| | P130 | CDR2 | Y59 | | | Hydrophobic interaction |
| | | | | CDR3 | R93 | Van der Waals interaction |
| | | | | CDR3 | Y94 | Hydrophobic interaction |
| | K131 | | | CDR1 | E30 | Salt bridge |
| | | | | CDR3 | S92 | Van der Waals interaction |
| | | | | CDR3 | R93 | Hydrogen bond |
| | A132 | | | CDR1 | E30 | Van der Waals interaction |
| | | | | CDR1 | V32 | Hydrophobic interaction |
| | | | | CDR3 | Y91 | Van der Waals interaction |
| | | | | CDR3 | S92 | Hydrogen bond |
| | I134 | | | CDR2 | W50 | Hydrophobic interaction |

In addition, glycosylation at sites N49, N58, and N116 could be observed in the structure of PD-1 antigen, while the glycosylation at site N74 was not clear. Further, the involvement of the carbohydrate chain at N58 in the binding to h317-Fab could also be observed (Figure 15). In contrast, the carbohydrate chain at this site in the structure of any other reported complex formed between PD-1 antigen and an antibody thereof does not involve in the interaction between the PD-1 antigen and the antibody. In this regard, the interaction between h317-Fab and the carbohydrate chain at N58 may improve the specificity of recognition and binding therebetween. Glycosylation sites on PD-1 were mutated and plasmids carrying mutated PD-1 (PD-1-mut) obtained were transiently transfected into 293 cells, then the binding between the h317 and the PD-1-mut was detected by FACS. The FACS results showed that carbohydrate chain at N58 of PD-1 involved in the binding of h317 and PD-1. Specifically, the mutation of amino acid N58 of PD-1 to Alanine (A) made h317 lose its binding ability to the cells expressing PD-1 (Figure 16), indicating that carbohydrate chain at N58 of PD-1 involves in the binding of h317 and PD-1. In addition, it was also found from the structure comparison between PD-1-h317-Fab complex and PD-1/PD-L1 complex that there was a large overlap between the interaction interface of h317-Fab and PD-1 and the interaction interface of PD-L1 and PD-1 (Figure 17A), and most of the overlap existed in the FG loop of PD-1 (Figure 17B), indicating that h317-Fab competes with PD-L1 for binding to PD-1 and can block the interaction between PD-1 and PD-L1.

**Example 13:** site-directed mutation in light chain CDRs and heavy chain CDRs of h317

1. Mutants of light chain CDRs and heavy chain CDRs were constructed according to the structure analysis of the PD-1-h317Fab complex. Expression vectors were constructed using site-directed mutation method. See Ronghao Wang, Runchuan Chen, and Runzhong Liu, Journal of Xiamen University (Natural Science) 2008, Vol 47, sup 2, 282-285, for details.

Methods for expression and purification of the variant antibodies were the same as above.

2. Comparison of relative affinity of the variant antibodies

The comparison of affinity between the variant antibodies and the parent antibody was performed using Octet QKe system supplied by Fortebio. The method used was similar to that used in Example 4, except that the affinity was measured via only single antigen concentration binding for all variant antibodies to assess their relative affinities. Firstly, the antibodies to be tested at the same target value were coated, and the relative affinities were determined using 100 nM recombinant human PD-1 protein (NCBI accession No.: NP_005009.2, 21aa-167aa) as the mobile phase.

The detection results of the affinities of the variant antibodies showed that the affinities of the variant antibodies having various mutations changed significantly. Table 9 shows the mutations and Table 10 shows certain affinity changes.

Table 9: Amino acid sequences of CDRs of h317 variant antibodies

| LCDR1 | LCDR2 | LCDR3 |
|---|---|---|
| X1ASQDVX2TX3VA | X1ASTRX2X3 | QQX1X2X3X4PX5 |
| X1= K or R<br>X2= E or S<br>X3= A or V or Y | X1= A or G or W<br>X2= A or H or Q<br>X3= S or T | X1= A or F or Y<br>X2= D or N or S<br>X3= R or N or S<br>X4= F or Y<br>X5= G or W or Y |
| **HCDR1** | **HCDR2** | **HCDR3** |
| X1X2X3MS | TIX1X2X3X4X5X6TX7YPDSVKG | PX1X2SGVAX3 |
| X1= D or S<br>X2= A or N or Y<br>X3= D or S or Y | X1= K or S or W<br>X2= G or S or Y<br>X3= D or G or S<br>X4= G or S<br>X5= G or S | X1= D or E or Y<br>X2= A or S<br>X3= T or Y |
| | X6= T or Y<br>X7= A or T or Y | |

Table 10: Changes in affinities of h317 variant antibodies with CDR mutated (other amino acids in the variable regions are the same as h317 except for the mutations shown)

| LCDR1: KASQDVETVVA | | HCDR1: SYDMS | |
|---|---|---|---|
| MUT | KD(M) | MUT | KD(M) |
| K24R | 1.02E-09 | S31D | 5.12E-09 |

(continued)

| **LCDR1: KASQDVETVVA** | | **HCDR1: SYDMS** | |
|---|---|---|---|
| **MUT** | **KD(M)** | **MUT** | **KD(M)** |
| E30S | 1.42E-08 | Y32A | 6.83E-09 |
| V32A | 6.21E-09 | Y32N | 1.81E-08 |
| V32Y | 9.81E-09 | D33S | 2.89E-09 |
| LCDR2: WASTRHT | | D33Y | 2.36E-07 |
| **MUT** | **KD(M)** | **HCDR2: TISGGGSYTYYPDSVKG** | |
| W50A | 1.23E-08 | **MUT** | **KD(M)** |
| W50G | 6.14E-08 | S52K | 2.72E-08 |
| H55A | 1.72E-07 | S52W | 3.94E-08 |
| H55Q | 1.42E-08 | G53S | 5.53E-09 |
| T56S | 3.02E-09 | G53Y | 1.03E-08 |
| **LCDR3: QQYSRYPW** | | G54D | 2.93E-08 |
| **MUT** | **KD(M)** | G54S | 1.32E-09 |
| Y91A | 5.12E-08 | G55S | 9.09E-09 |
| Y91F | 4.76E-08 | S56G | 1.48E-07 |
| S92D | 7.52E-09 | Y57T | 6.12E-08 |
| S92N | 3.49E-09 | Y59A | 3.29E-09 |
| R93N | 3.64E-08 | Y59T | 1.89E-08 |
| R93S | 7.94E-09 | **HCDR3: PDSSGVAY** | |
| Y94F | 6.81E-09 | MUT | KD(M) |
| W96G | 1.53E-08 | D100E | 7.44E-08 |
| W96Y | 4.12E-07 | D100Y | 8.23E-09 |
| **Combined mutation of LCDR** | | S101A | 1.63E-08 |
| MUT | KD(M) | Y106T | 2.94E-07 |
| 24R(CDR1)<br>56S(CDR2) | 3.23E-09 | **Combined mutation of HCDR** | |
| 24R(CDR1)<br>92N(CDR3) | 4.17E-09 | MUT | KD(M) |
| 24R(CDR1)<br>94F(CDR3) | 5.32E-09 | 33S(CDR1)<br>54S(CDR2) | 3.12E-09 |
| 56S(CDR2)<br>92N(CDR3) | 3.98E-09 | 33S(CDR1)<br>59A(CDR2) | 3.96E-09 |
| 24R(CDR1)<br>56S(CDR2)<br>92N(CDR3) | 2.91E-09 | 54S(CDR2)<br>59A(CDR2) | 2.68E-09 |
| **Combined mutation of LCDR+HCDR** | | | |
| **MUT** | **KD(M)** | **MUT** | **KD(M)** |
| L-:<br>24R(CDR1)<br>56S(CDR2) | | L-:<br>24R(CDR1)<br>56S(CDR2) | |

(continued)

| Combined mutation of LCDR+HCDR | | | |
|---|---|---|---|
| **MUT** | **KD(M)** | **MUT** | **KD(M)** |
| 92N(CDR3);<br>H-:<br>33S(CDR1)<br>54S(CDR2) | 3.38E-09 | 92N(CDR3);<br>H-:<br>54S(CDR2)<br>59A(CDR2) | 2.88E-09 |

Data in the Table showed that the affinity was affected greatly, for example lowered by more than 10 times, if the amino acids at certain positions were mutated. While, the affinity of the antibody was improved if the amino acids at certain positions were mutated, for example, the affinity was improved to a certain extent by mutating K at position 24 of the light chain to R, T at position 56 of the light chain to S, S at position 92 of the light chain to N, D at position 33 of the heavy chain to S, G at position 54 of the heavy chain to S, and/or Y at position 59 of the heavy chain to A. Furthermore, the affinity remained unchanged or was further improved by combining some of the mutations at the above positions. Meanwhile, it was also found that HCDR3 was essential for the binding of the antibody and most variant antibodies having mutations in HCDR3 exhibited reduced affinities.

The above description for the embodiments of the present invention is not intended to limit the present invention, and those skilled in the art can make various changes and variations according to the present invention, which are within the protection scope of the claims of the present invention without departing from the spirit of the same.

## Claims

1. An antibody capable of binding to programmed cell death protein 1 (PD-1) or a fragment thereof, the antibody or fragment thereof comprising a light chain variable region and/or a heavy chain variable region, wherein the light chain variable region comprises light chain CDR1 (LCDR1), CDR2 (LCDR2), CDR3 (LCDR3) and/or the heavy chain variable region comprises heavy chain CDR1 (HCDR1), CDR2 (HCDR2) and CDR3 (HCDR3) as set forth in following sequences respectively:

| LCDR1 | LCDR2 | LCDR3 |
|---|---|---|
| X1ASQDVX2TX3VA (SEQ ID NO: 1) | X1ASTRX2X3 (SEQ ID NO:2) | QQX1X2X3X4PX5 (SEQ ID NO: 3) |
| X1= K or R;<br>X2= E or S;<br>X3= A, V or Y | X1= A, G or W;<br>X2= A, H or Q;<br>X3= S or T | X1= A, F or Y;<br>X2= D, N or S;<br>X3= R, N or S;<br>X4= F or Y;<br>X5= G, W or Y |
| **HCDR1** | **HCDR2** | **HCDR3** |
| X1X2X3MS (SEQ ID NO:4) | TIX1X2X3X4X5X6TX7YPDSVKG (SEQ ID NO:5) | PX1X2SGVAX3 (SEQ ID NO:6) |
| X1= D or S;<br>X2= A, N or Y;<br>X3= D, S or Y | X1= K, S or W;<br>X2= G, S or Y;<br>X3= D, G or S;<br>X4= G or S;<br>X5= G or S;<br>X6= T or Y;<br>X7= A, T or Y | X1= D, E or Y;<br>X2= A or S;<br>X3= T or Y |

2. The antibody or fragment thereof according to Claim 1, wherein the light chain variable region and/or the heavy chain variable region comprises LCDR1, LCDR2, LCDR3 and/or HCDR1, HCDR2, HCDR3 as set forth in following sequences respectively:

| LCDR1 | LCDR2 | LCDR3 |
|---|---|---|
| X1ASQDVX2TX3VA (SEQ ID NO:1) | X1ASTRX2X3 (SEQ ID NO:2) | QQX1X2X3X4PX5 (SEQ ID NO:3) |
| X1= K or R;<br>X2= E;<br>X3= A, V or Y | X1= W;<br>X2= H;<br>X3= S or T | X1= Y;<br>X2= D, N or S;<br>X3= R or S;<br>X4= F or Y;<br>X5= W |
| **HCDR1** | **HCDR2** | **HCDR3** |
| X1X2X3MS (SEQ ID NO:4 ) | TIX1X2X3X4X5X6TX7YPDSVKG (SEQ ID NO:5 ) | PX1X2SGVAX3 (SEQ ID NO: 6 ) |
| X1= D or S;<br>X2= A or Y;<br>X3= D or S | X1= S;<br>X2= G or S;<br>X3= D, G or S;<br>X4= G or S;<br>X5= S;<br>X6= Y;<br>X7= A or Y | X1= D or Y;<br>X2= S;<br>X3= Y |

3. The antibody or fragment thereof according to Claim 1 or 2, wherein the light chain variable region and/or the heavy chain variable region comprises LCDR1, LCDR2, LCDR3 and/or HCDR1, HCDR2, HCDR3 as set forth in following sequences respectively:

LCDR1 selected from the group consisting of SEQ ID NO:7, SEQ ID NO:8, SEQ ID NO:9, SEQ ID NO:10 and SEQ ID NO:11;
LCDR2 selected from the group consisting of SEQ ID NO:12, SEQ ID NO:13, SEQ ID NO:14, SEQ ID NO:15, SEQ ID NO:16 and SEQ ID NO:17;
LCDR3 selected from the group consisting of SEQ ID NO:18, SEQ ID NO:19, SEQ ID NO:20, SEQ ID NO:21, SEQ ID NO:22, SEQ ID NO:23, SEQ ID NO:24, SEQ ID NO:25, SEQ ID NO:26 and SEQ ID NO:27; and/or
HCDR1 selected from the group consisting of SEQ ID NO:28, SEQ ID NO:29, SEQ ID NO:30, SEQ ID NO:31, SEQ ID NO:32 and SEQ ID NO:33;
HCDR2 selected from the group consisting of SEQ ID NO:34, SEQ ID NO:35, SEQ ID NO:36, SEQ ID NO:37, SEQ ID NO:38, SEQ ID NO:39, SEQ ID NO:40, SEQ ID NO:41, SEQ ID NO:42, SEQ ID NO:43, SEQ ID NO:44, SEQ ID NO:45 and SEQ ID NO:46;
HCDR3 selected from the group consisting of SEQ ID NO:47, SEQ ID NO:48, SEQ ID NO:49, SEQ ID NO:50, and SEQ ID NO:51.

**4.** The antibody or fragment thereof according to any one of Claims 1-3, wherein the light chain variable region comprises LCDR1, LCDR2, LCDR3 selected from the group consisting of sequence combinations as follows:

| Combination | LCDR1 | LCDR2 | LCDR3 |
|---|---|---|---|
| 1 | SEQ ID NO:7 | SEQ ID NO:12 | SEQ ID NO:18 |
| 2 | SEQ ID NO:8 | SEQ ID NO:12 | SEQ ID NO:18 |
| 3 | SEQ ID NO:9 | SEQ ID NO:12 | SEQ ID NO:18 |
| 4 | SEQ ID NO:10 | SEQ ID NO:12 | SEQ ID NO:18 |
| 5 | SEQ ID NO:11 | SEQ ID NO:12 | SEQ ID NO:18 |
| 6 | SEQ ID NO:7 | SEQ ID NO:13 | SEQ ID NO:18 |
| 7 | SEQ ID NO:7 | SEQ ID NO:14 | SEQ ID NO:18 |
| 8 | SEQ ID NO:7 | SEQ ID NO:15 | SEQ ID NO:18 |
| 9 | SEQ ID NO:7 | SEQ ID NO:16 | SEQ ID NO:18 |
| 10 | SEQ ID NO:7 | SEQ ID NO:17 | SEQ ID NO:18 |
| 11 | SEQ ID NO:7 | SEQ ID NO:12 | SEQ ID NO:19 |
| 12 | SEQ ID NO:7 | SEQ ID NO:12 | SEQ ID NO:20 |
| 13 | SEQ ID NO:7 | SEQ ID NO:12 | SEQ ID NO:21 |
| 14 | SEQ ID NO:7 | SEQ ID NO:12 | SEQ ID NO:22 |
| 15 | SEQ ID NO:7 | SEQ ID NO:12 | SEQ ID NO:23 |
| 16 | SEQ ID NO:7 | SEQ ID NO:12 | SEQ ID NO:24 |
| 17 | SEQ ID NO:7 | SEQ ID NO:12 | SEQ ID NO:25 |
| 18 | SEQ ID NO:7 | SEQ ID NO:12 | SEQ ID NO:26 |
| 19 | SEQ ID NO:7 | SEQ ID NO:12 | SEQ ID NO:27 |
| 20 | SEQ ID NO:8 | SEQ ID NO:17 | SEQ ID NO:18 |
| 21 | SEQ ID NO:8 | SEQ ID NO:12 | SEQ ID NO:22 |
| 22 | SEQ ID NO:8 | SEQ ID NO:12 | SEQ ID NO:25 |
| 23 | SEQ ID NO:7 | SEQ ID NO:17 | SEQ ID NO:22 |
| 24 | SEQ ID NO:8 | SEQ ID NO:17 | SEQ ID NO:22 |

,
and/or the heavy chain variable region comprises HCDR1, HCDR2, HCDR3 selected from the group consisting of sequence combinations as follows:

| Combination | HCDR1 | HCDR2 | HCDR3 |
|---|---|---|---|
| 1 | SEQ ID NO:28 | SEQ ID NO:34 | SEQ ID NO:47 |
| 2 | SEQ ID NO:29 | SEQ ID NO:34 | SEQ ID NO:47 |
| 3 | SEQ ID NO:30 | SEQ ID NO:34 | SEQ ID NO:47 |
| 4 | SEQ ID NO:31 | SEQ ID NO:34 | SEQ ID NO:47 |
| 5 | SEQ ID NO:32 | SEQ ID NO:34 | SEQ ID NO:47 |
| 6 | SEQ ID NO:33 | SEQ ID NO:34 | SEQ ID NO:47 |
| 7 | SEQ ID NO:28 | SEQ ID NO:35 | SEQ ID NO:47 |
| 8 | SEQ ID NO:28 | SEQ ID NO:36 | SEQ ID NO:47 |
| 9 | SEQ ID NO:28 | SEQ ID NO:37 | SEQ ID NO:47 |
| 10 | SEQ ID NO:28 | SEQ ID NO:38 | SEQ ID NO:47 |
| 11 | SEQ ID NO:28 | SEQ ID NO:39 | SEQ ID NO:47 |
| 12 | SEQ ID NO:28 | SEQ ID NO:40 | SEQ ID NO:47 |
| 13 | SEQ ID NO:28 | SEQ ID NO:41 | SEQ ID NO:47 |
| 14 | SEQ ID NO:28 | SEQ ID NO:42 | SEQ ID NO:47 |
| 15 | SEQ ID NO:28 | SEQ ID NO:43 | SEQ ID NO:47 |
| 16 | SEQ ID NO:28 | SEQ ID NO:44 | SEQ ID NO:47 |
| 17 | SEQ ID NO:28 | SEQ ID NO:45 | SEQ ID NO:47 |
| 18 | SEQ ID NO:28 | SEQ ID NO:34 | SEQ ID NO:48 |
| 19 | SEQ ID NO:28 | SEQ ID NO:34 | SEQ ID NO:49 |
| 20 | SEQ ID NO:28 | SEQ ID NO:34 | SEQ ID NO:50 |
| 21 | SEQ ID NO:28 | SEQ ID NO:34 | SEQ ID NO:51 |
| 22 | SEQ ID NO:32 | SEQ ID NO:40 | SEQ ID NO:47 |
| 23 | SEQ ID NO:32 | SEQ ID NO:44 | SEQ ID NO:47 |
| 24 | SEQ ID NO:28 | SEQ ID NO:46 | SEQ ID NO:47 |
| 25 | SEQ ID NO:32 | SEQ ID NO:40 | SEQ ID NO:47 |
| 26 | SEQ ID NO:28 | SEQ ID NO:46 | SEQ ID NO:47 |

;

preferably, the light chain variable region and the heavy chain variable region comprise LCDR1, LCDR2, LCDR3 and HCDR1, HCDR2, HCDR3 selected from the group consisting of sequence combinations as follows:

| Combination | LCDR1 | LCDR2 | LCDR3 | **HCDR1** | HCDR2 | HCDR3 |
|---|---|---|---|---|---|---|
| 1 | SEQ ID NO:7 | SEQ ID NO: 12 | SEQ ID NO: 18 | SEQ ID NO: 28 | SEQ ID NO: 34 | SEQ ID NO: 47 |
| 2 | SEQ ID NO:8 | SEQ ID NO: 12 | SEQ ID NO: 18 | SEQ ID NO: 28 | SEQ ID NO: 34 | SEQ ID NO: 47 |
| 3 | SEQ ID NO:9 | SEQ ID NO: 12 | SEQ ID NO: 18 | SEQ ID NO: 28 | SEQ ID NO: 34 | SEQ ID NO: 47 |
| 4 | SEQ ID NO: 10 | SEQ ID NO: 12 | SEQ ID NO: 18 | SEQ ID NO: 28 | SEQ ID NO: 34 | SEQ ID NO: 47 |

(continued)

| Combination | LCDR1 | LCDR2 | LCDR3 | **HCDR1** | HCDR2 | HCDR3 |
|---|---|---|---|---|---|---|
| 5 | SEQ ID NO: 11 | SEQ ID NO: 12 | SEQ ID NO: 18 | SEQ ID NO: 28 | SEQ ID NO: 34 | SEQ ID NO: 47 |
| 6 | SEQ ID NO:7 | SEQ ID NO: 13 | SEQ ID NO: 18 | SEQ ID NO: 28 | SEQ ID NO: 34 | SEQ ID NO: 47 |
| 7 | SEQ ID NO:7 | SEQ ID NO: 14 | SEQ ID NO: 18 | SEQ ID NO: 28 | SEQ ID NO: 34 | SEQ ID NO: 47 |
| 8 | SEQ ID NO:7 | SEQ ID NO: 15 | SEQ ID NO: 18 | SEQ ID NO: 28 | SEQ ID NO: 34 | SEQ ID NO: 47 |
| 9 | SEQ ID NO:7 | SEQ ID NO: 16 | SEQ ID NO: 18 | SEQ ID NO: 28 | SEQ ID NO: 34 | SEQ ID NO: 47 |
| 10 | SEQ ID NO:7 | SEQ ID NO: 17 | SEQ ID NO: 18 | SEQ ID NO: 28 | SEQ ID NO: 34 | SEQ ID NO: 47 |
| 11 | SEQ ID NO:7 | SEQ ID NO: 12 | SEQ ID NO: 19 | SEQ ID NO: 28 | SEQ ID NO: 34 | SEQ ID NO: 47 |
| 12 | SEQ ID NO:7 | SEQ ID NO: 12 | SEQ ID NO: 20 | SEQ ID NO: 28 | SEQ ID NO: 34 | SEQ ID NO: 47 |
| 13 | SEQ ID NO:7 | SEQ ID NO: 12 | SEQ ID NO: 21 | SEQ ID NO: 28 | SEQ ID NO: 34 | SEQ ID NO: 47 |
| 14 | SEQ ID NO:7 | SEQ ID NO: 12 | SEQ ID NO: 22 | SEQ ID NO: 28 | SEQ ID NO: 34 | SEQ ID NO: 47 |
| 15 | SEQ ID NO:7 | SEQ ID NO: 12 | SEQ ID NO: 23 | SEQ ID NO: 28 | SEQ ID NO: 34 | SEQ ID NO: 47 |
| 16 | SEQ ID NO:7 | SEQ ID NO: 12 | SEQ ID NO: 24 | SEQ ID NO: 28 | SEQ ID NO: 34 | SEQ ID NO: 47 |
| 17 | SEQ ID NO:7 | SEQ ID NO: 12 | SEQ ID NO: 25 | SEQ ID NO: 28 | SEQ ID NO: 34 | SEQ ID NO: 47 |
| 18 | SEQ ID NO:7 | SEQ ID NO: 12 | SEQ ID NO: 26 | SEQ ID NO: 28 | SEQ ID NO: 34 | SEQ ID NO: 47 |
| 19 | SEQ ID NO:7 | SEQ ID NO: 12 | SEQ ID NO: 27 | SEQ ID NO: 28 | SEQ ID NO: 34 | SEQ ID NO: 47 |
| 20 | SEQ ID NO:8 | SEQ ID NO: 17 | SEQ ID NO: 18 | SEQ ID NO: 28 | SEQ ID NO: 34 | SEQ ID NO: 47 |
| 21 | SEQ ID NO:8 | SEQ ID NO: 12 | SEQ ID NO: 22 | SEQ ID NO: 28 | SEQ ID NO: 34 | SEQ ID NO: 47 |
| 22 | SEQ ID NO:8 | SEQ ID NO: 12 | SEQ ID NO: 25 | SEQ ID NO: 28 | SEQ ID NO: 34 | SEQ ID NO: 47 |
| 23 | SEQ ID NO:7 | SEQ ID NO: 17 | SEQ ID NO: 22 | SEQ ID NO: 28 | SEQ ID NO: 34 | SEQ ID NO: 47 |
| 24 | SEQ ID NO:8 | SEQ ID NO: 17 | SEQ ID NO: 22 | SEQ ID NO: 28 | SEQ ID NO: 34 | SEQ ID NO: 47 |
| 25 | SEQ ID NO:7 | SEQ ID NO: 12 | SEQ ID NO: 18 | SEQ ID NO: 29 | SEQ ID NO: 34 | SEQ ID NO: 47 |
| 26 | SEQ ID NO:7 | SEQ ID NO: 12 | SEQ ID NO: 18 | SEQ ID NO: 30 | SEQ ID NO: 34 | SEQ ID NO: 47 |

(continued)

| Combination | LCDR1 | LCDR2 | LCDR3 | HCDR1 | HCDR2 | HCDR3 |
|---|---|---|---|---|---|---|
| 27 | SEQ ID NO:7 | SEQ ID NO: 12 | SEQ ID NO: 18 | SEQ ID NO: 31 | SEQ ID NO: 34 | SEQ ID NO: 47 |
| 28 | SEQ ID NO:7 | SEQ ID NO: 12 | SEQ ID NO: 18 | SEQ ID NO: 32 | SEQ ID NO: 34 | SEQ ID NO: 47 |
| 29 | SEQ ID NO:7 | SEQ ID NO: 12 | SEQ ID NO: 18 | SEQ ID NO: 33 | SEQ ID NO: 34 | SEQ ID NO: 47 |
| 30 | SEQ ID NO:7 | SEQ ID NO: 12 | SEQ ID NO: 18 | SEQ ID NO: 28 | SEQ ID NO: 35 | SEQ ID NO: 47 |
| 31 | SEQ ID NO:7 | SEQ ID NO: 12 | SEQ ID NO: 18 | SEQ ID NO: 28 | SEQ ID NO: 36 | SEQ ID NO: 47 |
| 32 | SEQ ID NO:7 | SEQ ID NO: 12 | SEQ ID NO: 18 | SEQ ID NO: 28 | SEQ ID NO: 37 | SEQ ID NO: 47 |
| 33 | SEQ ID NO:7 | SEQ ID NO: 12 | SEQ ID NO: 18 | SEQ ID NO: 28 | SEQ ID NO: 38 | SEQ ID NO: 47 |
| 34 | SEQ ID NO:7 | SEQ ID NO: 12 | SEQ ID NO: 18 | SEQ ID NO: 28 | SEQ ID NO: 39 | SEQ ID NO: 47 |
| 35 | SEQ ID NO:7 | SEQ ID NO: 12 | SEQ ID NO: 18 | SEQ ID NO: 28 | SEQ ID NO: 40 | SEQ ID NO: 47 |
| 36 | SEQ ID NO:7 | SEQ ID NO: 12 | SEQ ID NO: 18 | SEQ ID NO: 28 | SEQ ID NO: 41 | SEQ ID NO: 47 |
| 37 | SEQ ID NO:7 | SEQ ID NO: 12 | SEQ ID NO: 18 | SEQ ID NO: 28 | SEQ ID NO: 42 | SEQ ID NO: 47 |
| 38 | SEQ ID NO:7 | SEQ ID NO: 12 | SEQ ID NO: 18 | SEQ ID NO: 28 | SEQ ID NO: 43 | SEQ ID NO: 47 |
| 39 | SEQ ID NO:7 | SEQ ID NO: 12 | SEQ ID NO: 18 | SEQ ID NO: 28 | SEQ ID NO: 44 | SEQ ID NO: 47 |
| 40 | SEQ ID NO:7 | SEQ ID NO: 12 | SEQ ID NO: 18 | SEQ ID NO: 28 | SEQ ID NO: 45 | SEQ ID NO: 47 |
| 41 | SEQ ID NO:7 | SEQ ID NO: 12 | SEQ ID NO: 18 | SEQ ID NO: 28 | SEQ ID NO: 34 | SEQ ID NO: 48 |
| 42 | SEQ ID NO:7 | SEQ ID NO: 12 | SEQ ID NO: 18 | SEQ ID NO: 28 | SEQ ID NO: 34 | SEQ ID NO: 49 |
| 43 | SEQ ID NO:7 | SEQ ID NO: 12 | SEQ ID NO: 18 | SEQ ID NO: 28 | SEQ ID NO: 34 | SEQ ID NO: 50 |
| 44 | SEQ ID NO:7 | SEQ ID NO: 12 | SEQ ID NO: 18 | SEQ ID NO: 28 | SEQ ID NO: 34 | SEQ ID NO: 51 |
| 45 | SEQ ID NO:7 | SEQ ID NO: 12 | SEQ ID NO: 18 | SEQ ID NO: 32 | SEQ ID NO: 40 | SEQ ID NO: 47 |
| 46 | SEQ ID NO:7 | SEQ ID NO: 12 | SEQ ID NO: 18 | SEQ ID NO: 32 | SEQ ID NO: 44 | SEQ ID NO: 47 |
| 47 | SEQ ID NO:7 | SEQ ID NO: 12 | SEQ ID NO: 18 | SEQ ID NO: 28 | SEQ ID NO: 46 | SEQ ID NO: 47 |
| 48 | SEQ ID NO:8 | SEQ ID NO: 17 | SEQ ID NO: 22 | SEQ ID NO: 32 | SEQ ID NO: 40 | SEQ ID NO: 47 |

(continued)

| Combination | LCDR1 | LCDR2 | LCDR3 | **HCDR1** | HCDR2 | HCDR3 |
|---|---|---|---|---|---|---|
| 49 | SEQ ID NO:8 | SEQ ID NO: 17 | SEQ ID NO: 22 | SEQ ID NO: 28 | SEQ ID NO: 46 | SEQ ID NO: 47 |

**5.** The antibody or fragment thereof according to any one of Claims 1-4, wherein the antibody or fragment thereof has one or more activities selected from the group consisting of:

(1) blocking the binding of PD-1 to its ligand PD-L1 or PD-L2;
(2) binding to primate PD-1 specifically, and showing no cross-reactivity with non-primate PD-1;
(3) showing no binding to members of CD28 family other than PD-1;
(4) inducing the production of IL-2 and/or IFN-$\gamma$ in CD4+ T cells; and
(5) having no ADCC-effector function;

preferably, the antibody or fragment thereof binds to the carbohydrate chain at N58 of human PD-1; preferably, the heavy chain variable region of the antibody or fragment thereof comprises an amino acid sequence as set forth in SEQ ID NO:47;
preferably, the light chain variable region comprises an amino acid sequence as set forth in SEQ ID NO:52 or SEQ ID NO:60, or an amino acid sequence having at least 75% sequence identity to the amino acid sequence as set forth in SEQ ID NO:52 or SEQ ID NO:60; and/or the heavy chain variable region comprises an amino acid sequence as set forth in SEQ ID NO:54 or SEQ ID NO:62, or an amino acid sequence having at least 75% sequence identity to the amino acid sequence as set forth in SEQ ID NO:54 or SEQ ID NO:62;
preferably, the "at least 75% sequence identity" refers to any percent identity greater than or equal to 75%, for example at least 80%, preferably at least 85%, more preferably at least 90%, further more preferably at least 91%, 92%, 93%, 94%, 95%, 96%, 97%, 98% or even 99% identity.

**6.** The antibody or fragment thereof according to any one of Claims 1-5, wherein the light chain variable region comprises an amino acid sequence as set forth in SEQ ID NO:60 or a variant thereof; and with reference to the numbering and composition of amino acid residues of the sequence as set forth in SEQ ID NO: 60, the variant has one or more, preferably 1-3 amino acid substitution(s) in comparison with SEQ ID NO:60 selected from the group consisting of: K24R, E30S, V32A, V32Y, W50A, W50G, H55A, H55Q, T56S, Y91A, Y91F, S92D, S92N, R93N, R93S, Y94F, W96G and W96Y;
preferably, the variant has one or more, preferably 1-3 amino acid substitution(s) in comparison with SEQ ID NO:60 selected from the group consisting of: K24R, V32A, V32Y, T56S, S92D, S92N, R93S and Y94F.

**7.** The antibody or fragment thereof according to any one of Claims 1-6, wherein the heavy chain variable region comprises an amino acid sequence as set forth in SEQ ID NO:62 or a variant thereof; and with reference to the numbering and composition of amino acid residues of the sequence as set forth in SEQ ID NO: 62, the variant has one or more, preferably 1-3 amino acid substitution(s) in comparison with SEQ ID NO:62 selected from the group consisting of: S31D, Y32A, Y32N, D33S, D33Y, S52K, S52W, G53S, G53Y, G54D, G54S, G55S, S56G, Y57T, Y59A, Y59T, D100E, D100Y, S101A and Y106T;
preferably, the variant has one or more, preferably 1-3 amino acid substitution(s) in comparison with SEQ ID NO:62 selected from the group consisting of: S31D, Y32A, D33S, G53S, G54S, G55S, Y59A and D100Y.

**8.** The antibody or fragment thereof according to any one of Claims 1-7, wherein the antibody can be any of a monoclonal antibody, a single chain antibody, a bifunctional antibody, a single domain antibody, a nanobody, a fully or partially humanized antibody or chimeric antibody, or the like; preferably, the antibody is an IgA, IgD, IgE, IgG, or IgM antibody, more preferably is an IgG1 or IgG4 antibody;

preferably, the fragment is any fragment of the antibody that is capable of specifically binding to PD-1 or any portion thereof; more preferably, the fragment is a fragment scFv, BsFv, dsFv, $(dsFv)_2$, Fab, Fab', $F(ab')_2$ or Fv of the antibody;
more preferably, the heavy chain constant region of the antibody is of IgG1 or IgG4 subtype, and the light chain constant region is of $\kappa$ type;
further preferably, the light chain constant region of the antibody comprises an amino acid sequence as set forth in SEQ ID NO:56 or SEQ ID NO:64, or an amino acid sequence having at least 75% sequence identity to the amino acid sequence as set forth in SEQ ID NO:56 or SEQ ID NO:64; and/or the heavy chain constant region comprises an amino acid sequence as set forth in SEQ ID NO:58 or SEQ ID NO:66, or an amino acid sequence having at least 75% sequence identity to the amino acid sequence as set forth in SEQ ID NO:58 or SEQ ID NO:66;

preferably, the "at least 75% sequence identity" refers to any percent identity greater than or equal to 75%, for example at least 80%, preferably at least 85%, more preferably at least 90%, further more preferably at least 91%, 92%, 93%, 94%, 95%, 96%, 97%, 98% or even 99% identity.

**9.** A conjugate or fusion protein comprising the antibody or fragment thereof according to any one of Claims 1-8.

**10.** A nucleic acid molecule comprising a nucleotide sequence encoding the heavy chain CDRs, the light CDRs, the heavy chain variable region, the light chain variable region, the heavy chain or the light chain in the antibody or fragment thereof according to any one of Claims 1-8; preferably, the nucleic acid molecule comprises a nucleotide sequence as set forth in SEQ ID NO:53, SEQ ID NO:55, SEQ ID NO:57, SEQ ID NO:59, SEQ ID NO:61, SEQ ID NO:63, SEQ ID NO:65 or SEQ ID NO:67..

**11.** A vector comprising the nucleic acid molecule according to Claim 10.

**12.** A host cell comprising the nucleic acid molecule according to Claim 10 and/or the vector according to Claim 11, or transformed or transfected with the nucleic acid molecule according to Claim 10 and/or the vector according to Claim 11.

**13.** A pharmaceutical composition comprising the antibody or fragment thereof according to any one of Claims 1-8, the conjugate or fusion protein according Claim 9, the nucleic acid molecule according to Claim 10, the vector according to Claim 11 and/or the host cell according to Claim 12, and optionally a pharmaceutically acceptable excipient.

**14.** Use of the antibody or fragment thereof according to any one of Claims 1-8, the conjugate or fusion protein according to Claim 9, the nucleic acid molecule according to Claim 10, the vector according to Claim 11, the host cell according to Claim 12 and/or the pharmaceutical composition according to Claim 13 for manufacturing a medicament for the prevention or treatment of a tumor or cancer.

**15.** A pharmaceutical combination comprising:

(1) the antibody or fragment thereof according to any one of Claims 1-8, the conjugate or fusion protein according to Claim 9, the nucleic acid molecule according to Claim 10, the vector according to Claim 11, the host cell according to Claim 12 and/or the pharmaceutical composition according to Claim 13;

(2) other immunopotentiator or immunopotentiating means, such as a small molecule chemical drug, a targeted agent, a recombinant protein drug such as an antibody, a vaccine, a ADC, an oncolytic viruse, a drug for gene and nucleic acid therapy, and a radiotherapy.

**16.** A kit comprising the antibody or fragment thereof according to any one of Claims 1-8, the conjugate or fusion protein according to Claim 9, the nucleic acid molecule according to Claim 10, the vector according to Claim 11, the host cell according to Claim 12 and/or the pharmaceutical composition according to Claim 13.

**17.** A method of preventing or treating a tumors or cancer, including administrating the antibody or fragment according to any one of Claims 1-8, the conjugate or fusion protein according to Claim 9, the nucleic acid molecule according to Claim 10, the vector according to Claim 11, the host cell according to Claim 12 and/or the pharmaceutical composition according to Claim 13, and optionally other drug or means to a subject in need thereof.

**Fig. 1**

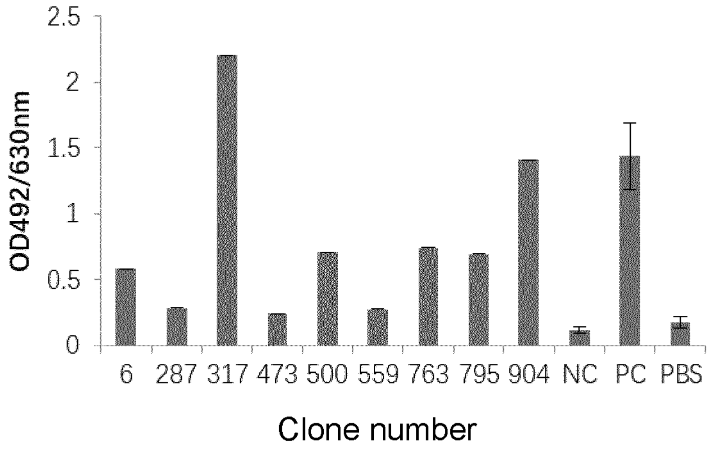
2.5
2
1.5
1
0.5
0
OD492/630nm
6
287
317
473
500
559
763
795
904
NC
PC
PBS
Clone number

1A

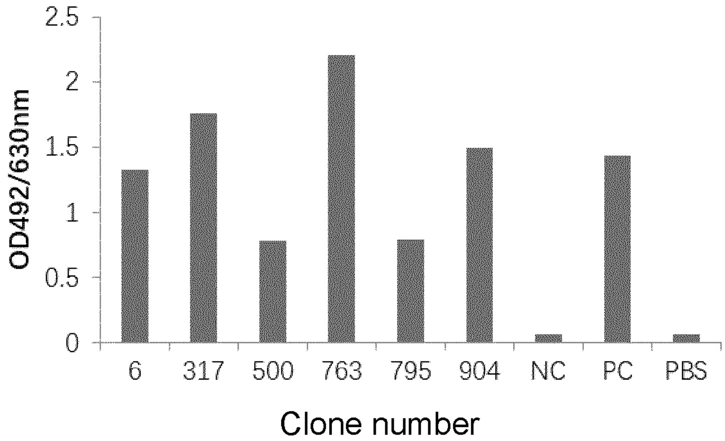
2.5
2
1.5
1
0.5
0
OD492/630nm
6
317
500
763
795
904
NC
PC
PBS
Clone number

1B

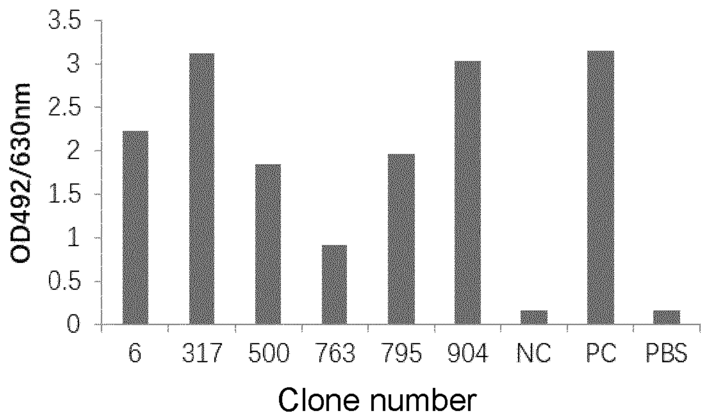
3.5
3
2.5
2
1.5
1
0.5
0
OD492/630nm
6
317
500
763
795
904
NC
PC
PBS
Clone number

1C

Fig.2

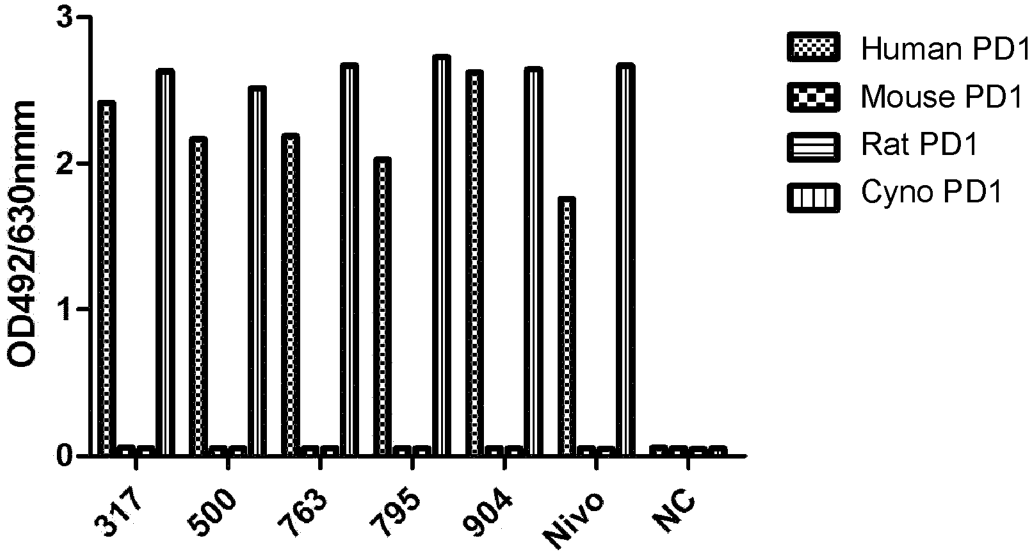
OD492/630nmm
0
1
2
3
317
500
763
795
904
Nivo
NC
Human PD1
Mouse PD1
Rat PD1
Cyno PD1

Fig. 3

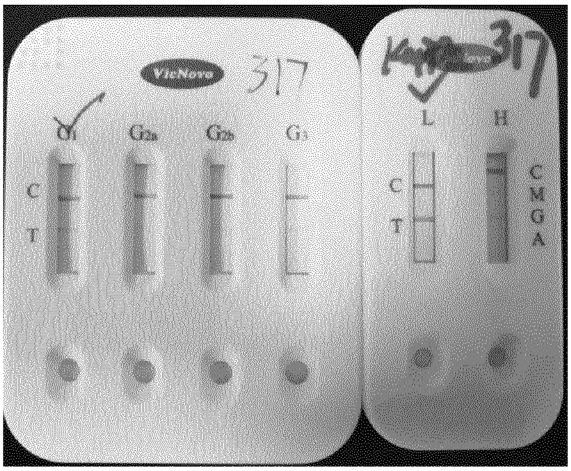
VicNovo
317
G1
G2a
G2b
G3
C
T
L
H
C
M
G
A

**Fig. 4**

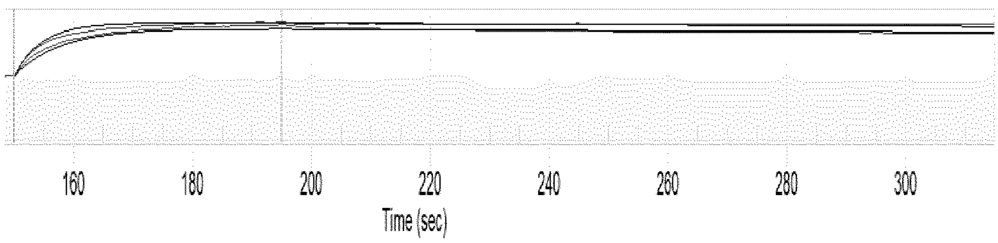
160
180
200
220
240
260
280
300
Time (sec)

4A

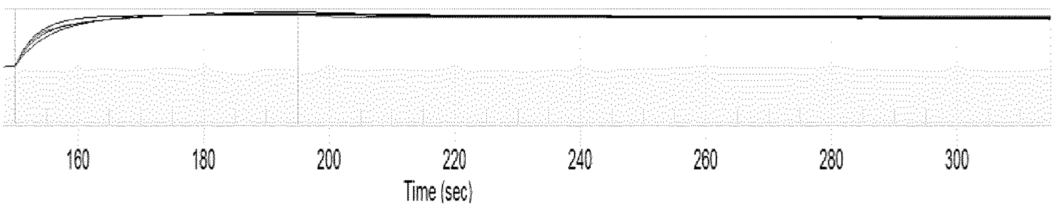
160
180
200
220
240
260
280
300
Time (sec)

4B

**Fig. 5**

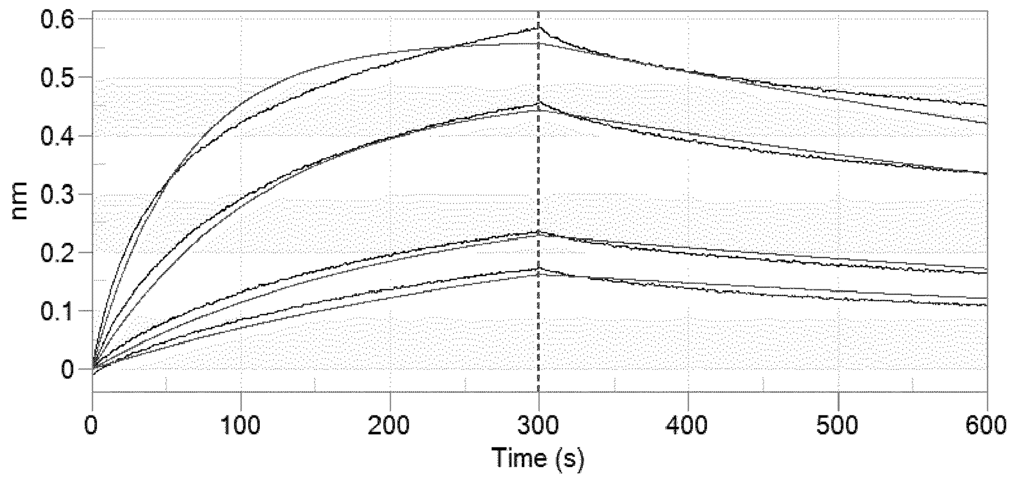
0.6
0.5
0.4
0.3
0.2
0.1
0
nm
0
100
200
300
400
500
600
Time (s)

5A

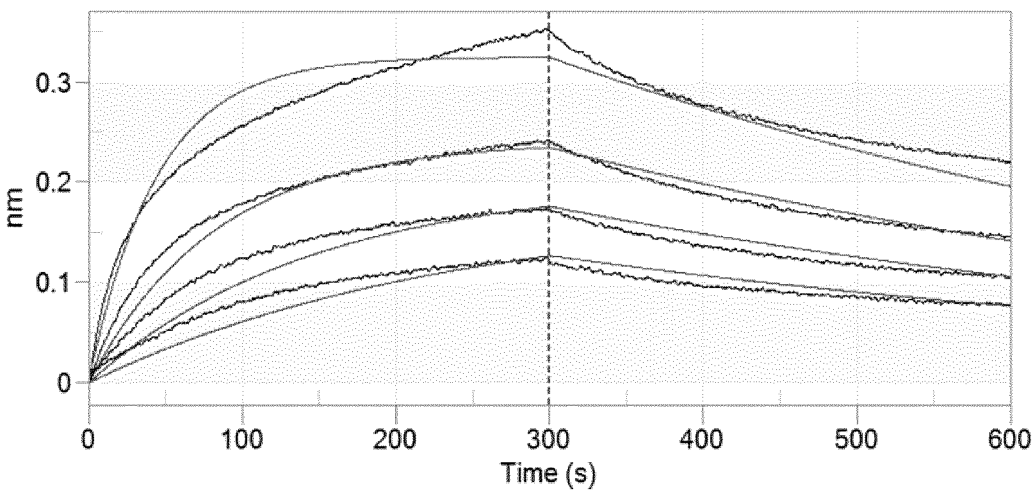
0.3
0.2
0.1
0
nm
0
100
200
300
400
500
600
Time (s)

5B

**Fig. 6**

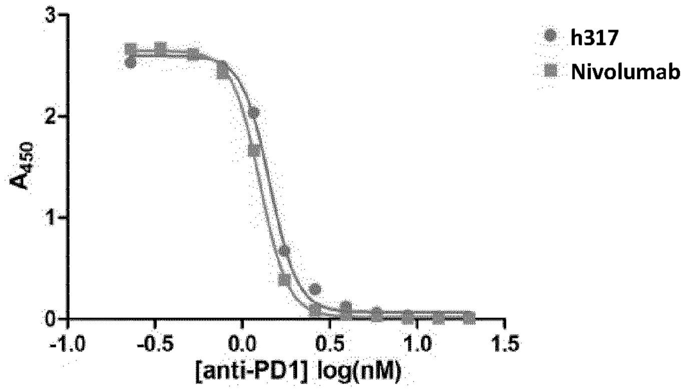
3
2
1
0
A450
-1.0
-0.5
0.0
0.5
1.0
1.5
[anti-PD1] log(nM)
h317
Nivolumab

6A

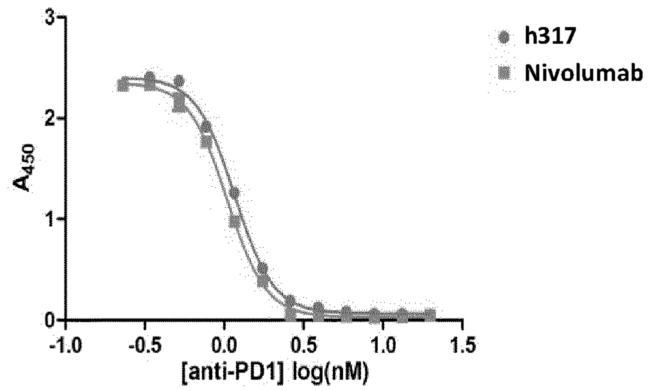
3
2
1
0
A450
-1.0
-0.5
0.0
0.5
1.0
1.5
[anti-PD1] log(nM)
h317
Nivolumab

6B

**Fig. 7**

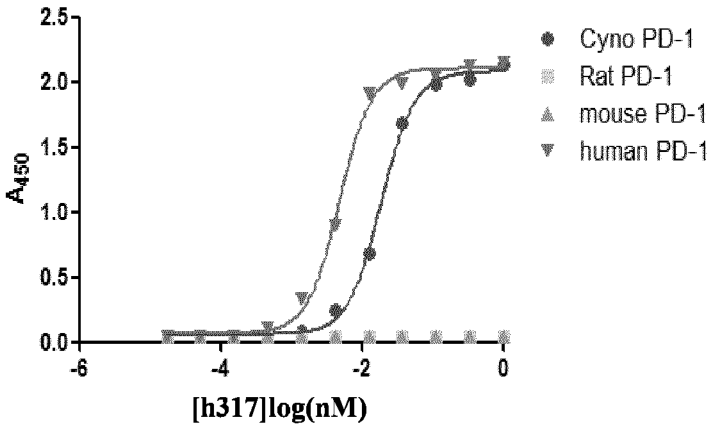
2.5
2.0
1.5
1.0
0.5
0.0
A450
-6
-4
-2
0
[h317]log(nM)
Cyno PD-1
Rat PD-1
mouse PD-1
human PD-1

**Fig. 8**

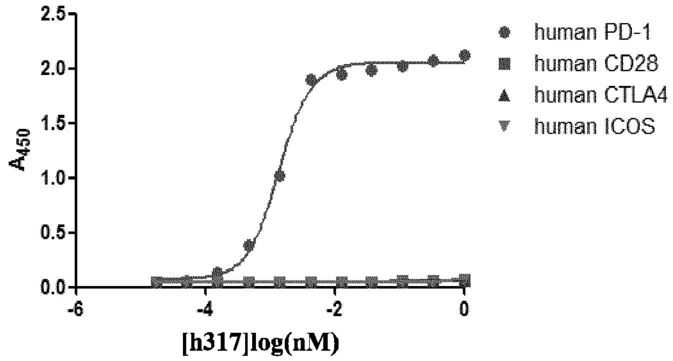
2.5
2.0
1.5
1.0
0.5
0.0
A450
-6
-4
-2
0
[h317]log(nM)
human PD-1
human CD28
human CTLA4
human ICOS

Fig. 9

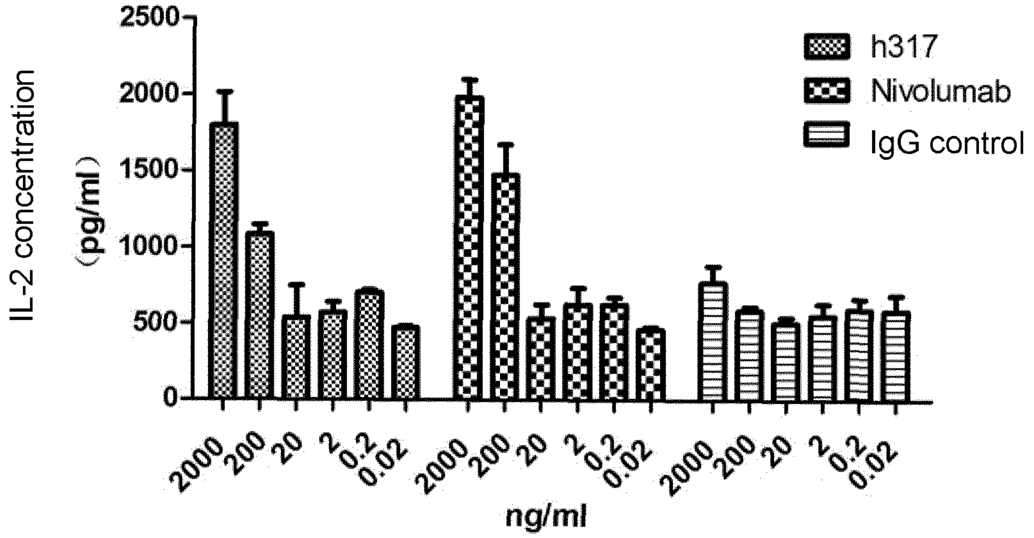

IL-2 concentration (pg/ml)
2500
2000
1500
1000
500
0
h317
Nivolumab
IgG control
2000
200
20
2
0.2
0.02
ng/ml


9A

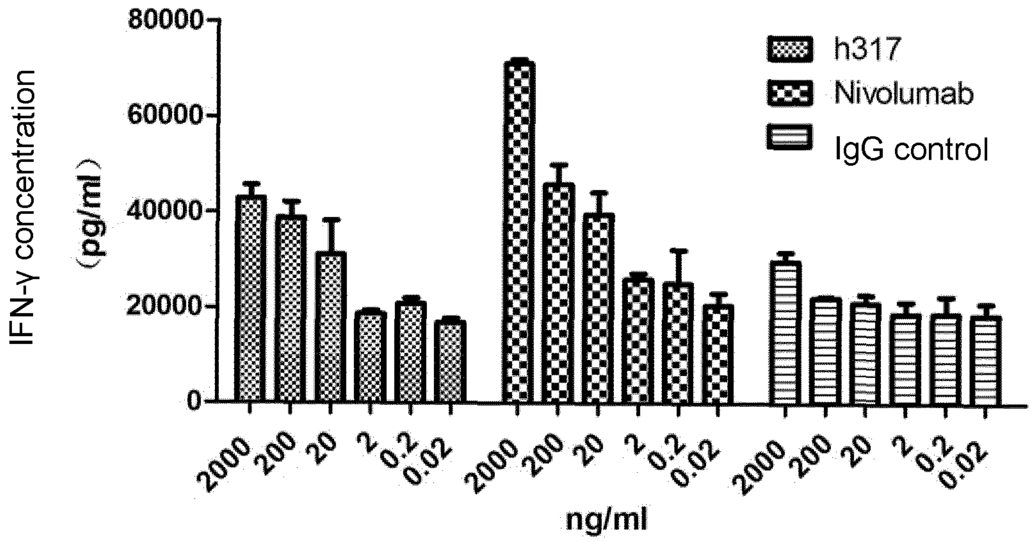

IFN-γ concentration (pg/ml)
80000
60000
40000
20000
0
h317
Nivolumab
IgG control
2000
200
20
2
0.2
0.02
ng/ml


9B

Fig. 10

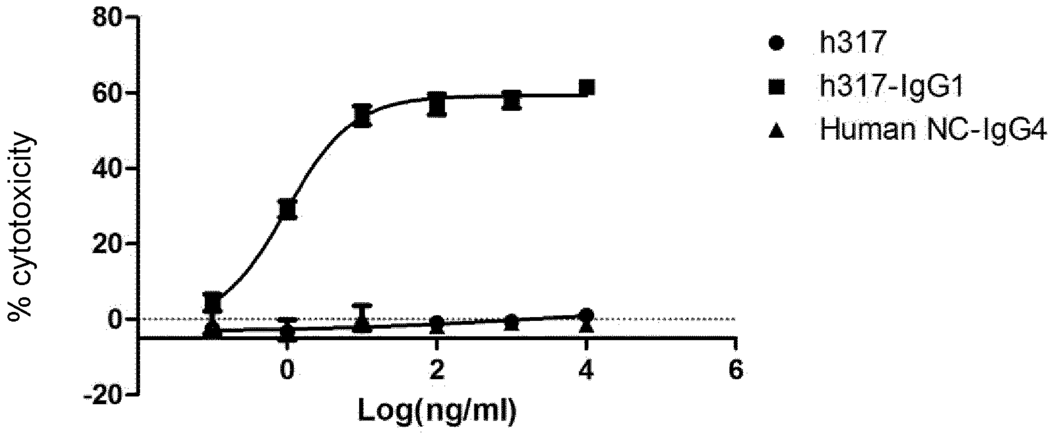
80
60
40
20
0
-20
% cytotoxicity
0
2
4
6
Log(ng/ml)
h317
h317-IgG1
Human NC-IgG4

Fig. 11

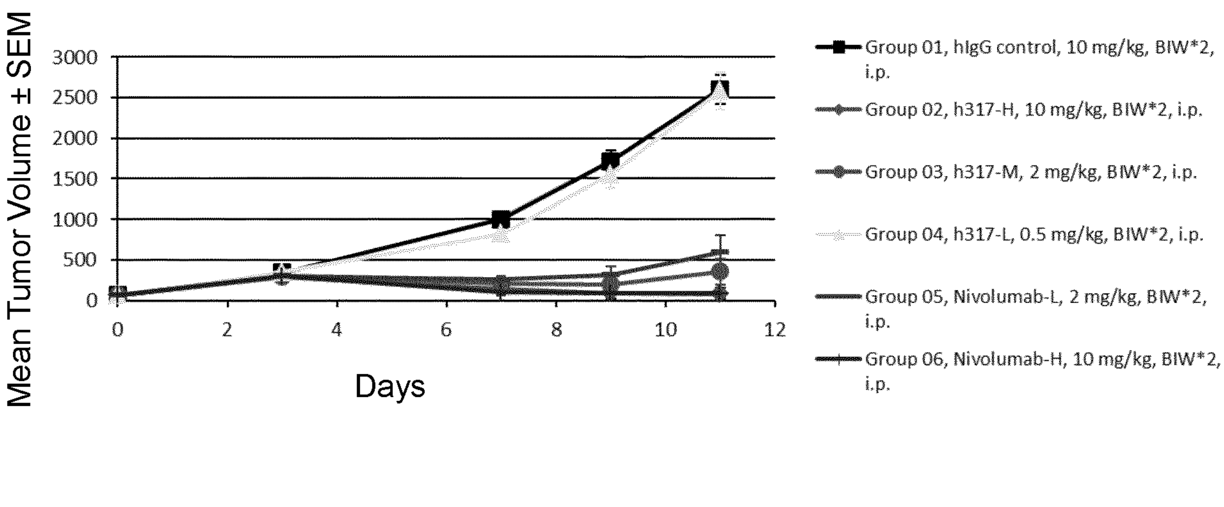
Mean Tumor Volume ± SEM
3000
2500
2000
1500
1000
500
0
0
2
4
6
8
10
12
Days
Group 01, hIgG control, 10 mg/kg, BIW*2, i.p.
Group 02, h317-H, 10 mg/kg, BIW*2, i.p.
Group 03, h317-M, 2 mg/kg, BIW*2, i.p.
Group 04, h317-L, 0.5 mg/kg, BIW*2, i.p.
Group 05, Nivolumab-L, 2 mg/kg, BIW*2, i.p.
Group 06, Nivolumab-H, 10 mg/kg, BIW*2, i.p.

**Fig. 12**

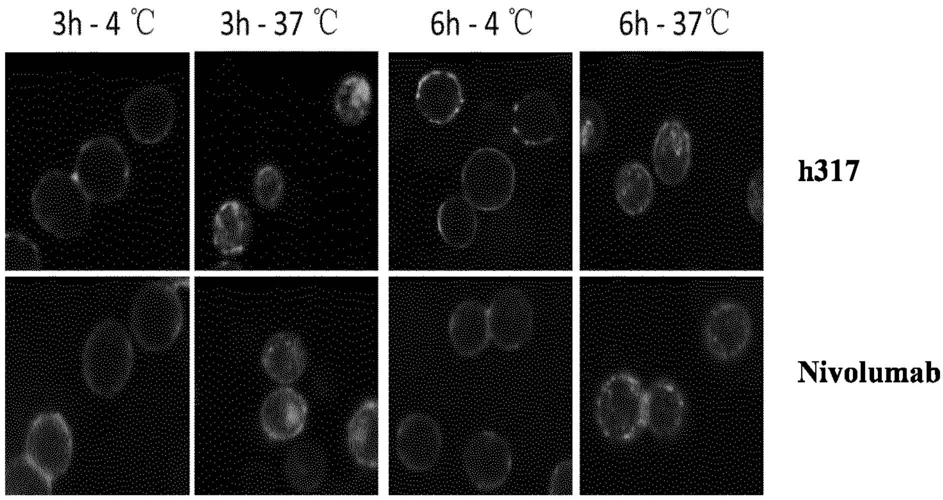


**Fig. 13**

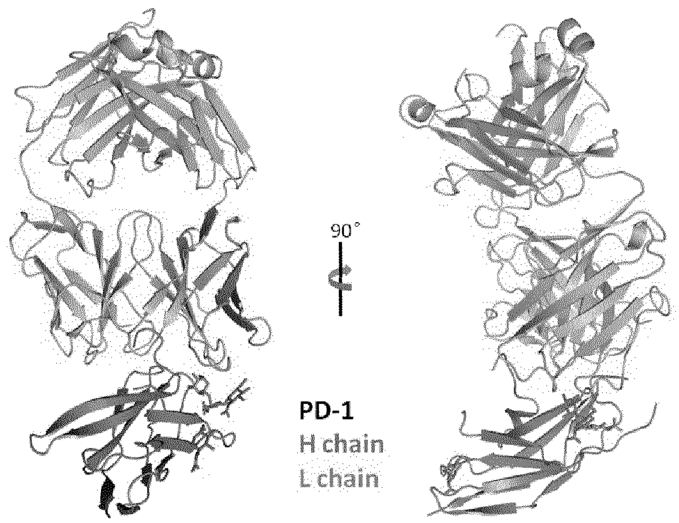

**Fig. 14**

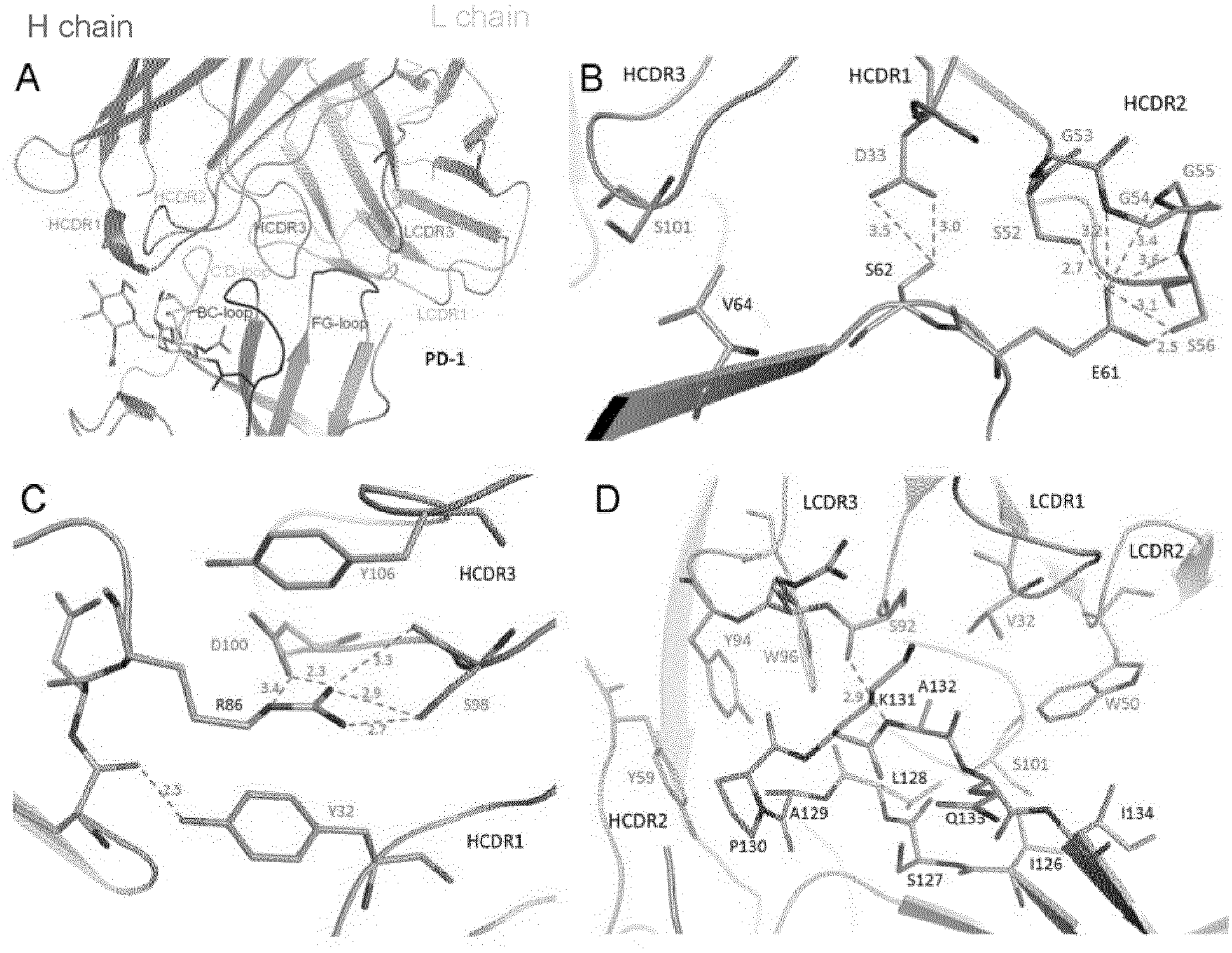
H chain
L chain
A
HCDR1
HCDR2
HCDR3
LCDR3
BC-loop
FG-loop
LCDR1
PD-1
B
HCDR3
HCDR1
HCDR2
S101
D33
G53
G55
G54
S52
V64
S62
E61
S56
C
Y106
HCDR3
D100
R86
S98
Y32
HCDR1
D
LCDR3
LCDR1
LCDR2
S92
V32
Y94
W96
K131
A132
W50
Y59
L128
S101
HCDR2
A129
Q133
I134
P130
I126
S127

**Fig. 15**

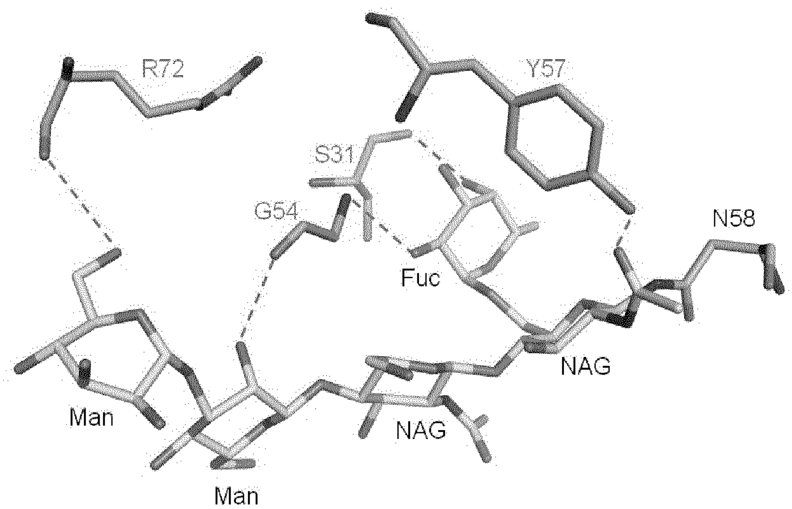
R72
Y57
S31
G54
N58
Fuc
NAG
Man
NAG
Man

Fig. 16

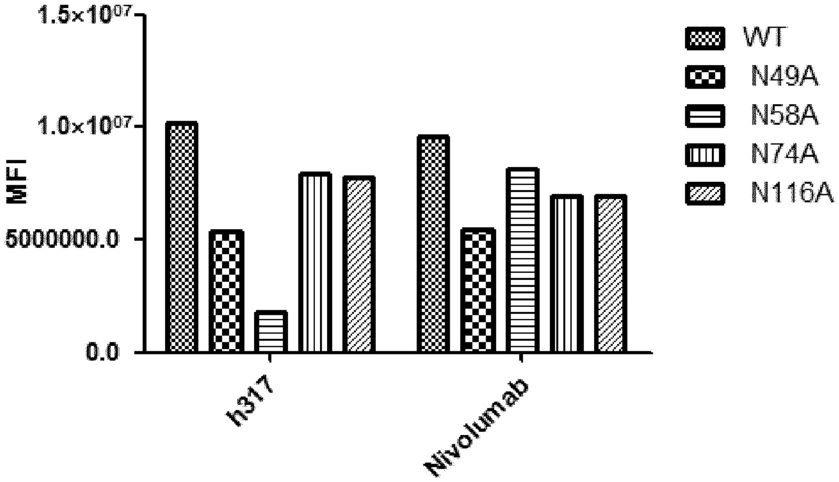
1.5×10^07
1.0×10^07
5000000.0
0.0
MFI
h317
Nivolumab
WT
N49A
N58A
N74A
N116A

Fig. 17

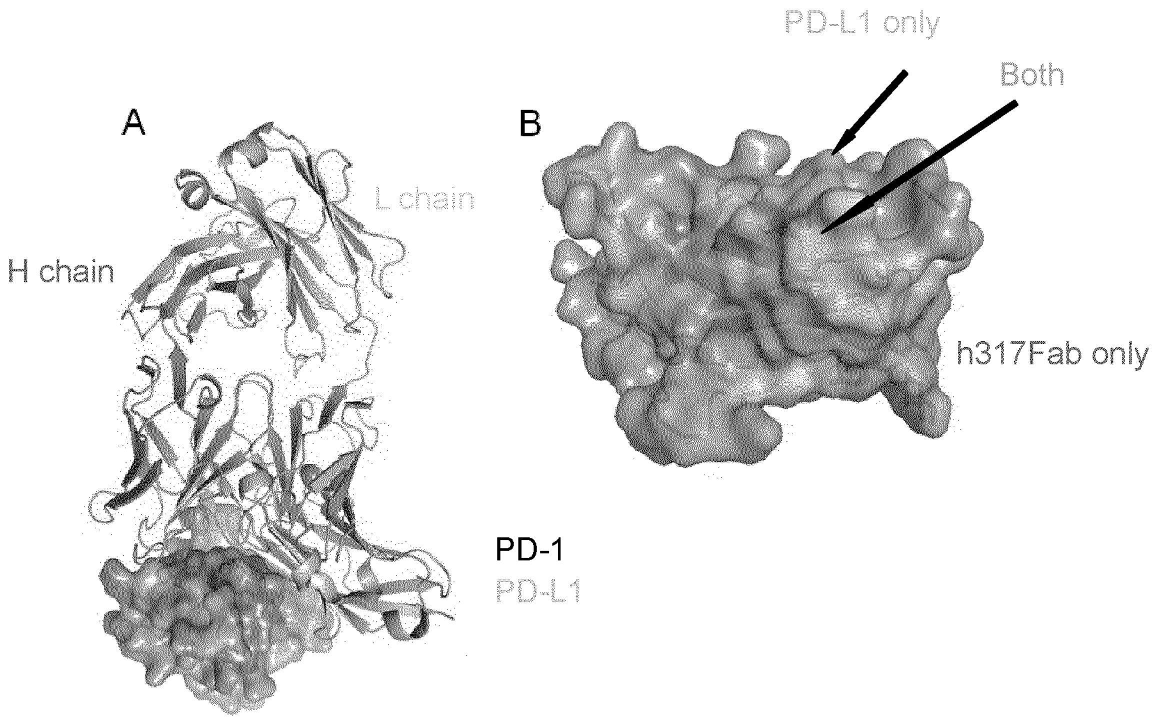
A
H chain
L chain
PD-1
PD-L1
B
PD-L1 only
Both
h317Fab only

# INTERNATIONAL SEARCH REPORT

International application No.
**PCT/CN2020/071353**

**A. CLASSIFICATION OF SUBJECT MATTER**

C07K 16/28(2006.01)i; C12N 15/13(2006.01)i; A61K 39/395(2006.01)i; A61P 35/00(2006.01)i

According to International Patent Classification (IPC) or to both national classification and IPC

**B. FIELDS SEARCHED**

Minimum documentation searched (classification system followed by classification symbols)

C07K, C12N, A61K, A61P

Documentation searched other than minimum documentation to the extent that such documents are included in the fields searched

Electronic data base consulted during the international search (name of data base and, where practicable, search terms used)

CNABS, CPRSABS, DWPI, SIPOABS, CNKI, 万方, ISI Web of Knowledge, BAIDU and search terms: 程序性细胞死亡蛋白1, PD-1, PD1, CD279, 单克隆抗体, 抗体, programmed death-1, antibody, McAb, anti-PD-1; Genbank, EMBL, National Bio-Sequence Database of Chinese Patent, STN and search sequences: SEQ ID NOs: 1-67.

**C. DOCUMENTS CONSIDERED TO BE RELEVANT**

| Category* | Citation of document, with indication, where appropriate, of the relevant passages | Relevant to claim No. |
|---|---|---|
| A | CN 107446048 A (BEIJING HANMI PHARMACEUTICAL CO., LTD.) 08 December 2017 (2017-12-08)<br>entire document | 1-16 |
| A | CN 106632674 A (AKESO BIOPHARMA, INC.) 10 May 2017 (2017-05-10)<br>entire document | 1-16 |
| A | WO 2017058115 A1 (ASIA BIOTECH PTE LTD) 06 April 2017 (2017-04-06)<br>entire document | 1-16 |
| A | WO 2017019846 A1 (MACROGENICS INC.) 02 February 2017 (2017-02-02)<br>entire document | 1-16 |
| A | WO 2016014688 A2 (QIU JUNZHUAN et al.) 28 January 2016 (2016-01-28)<br>entire document | 1-16 |

☐ Further documents are listed in the continuation of Box C. ☑ See patent family annex.

* Special categories of cited documents:
- "A" document defining the general state of the art which is not considered to be of particular relevance
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **16 March 2020** | **14 April 2020** |

| Name and mailing address of the ISA/CN | Authorized officer |
|---|---|
| **China National Intellectual Property Administration (ISA/CN)**<br>**No. 6, Xitucheng Road, Jimenqiao Haidian District, Beijing 100088**<br>**China** | |
| Facsimile No. **(86-10)62019451** | Telephone No. |

Form PCT/ISA/210 (second sheet) (January 2015)

**INTERNATIONAL SEARCH REPORT**

International application No.
**PCT/CN2020/071353**

**Box No. II Observations where certain claims were found unsearchable (Continuation of item 2 of first sheet)**

This international search report has not been established in respect of certain claims under Article 17(2)(a) for the following reasons:

1. [✓] Claims Nos.: **17**
because they relate to subject matter not required to be searched by this Authority, namely:
[1] PCT Rule 39.1(iv) – methods for treatment of the human or animal body by surgery or therapy.

2. [ ] Claims Nos.:
because they relate to parts of the international application that do not comply with the prescribed requirements to such an extent that no meaningful international search can be carried out, specifically:

3. [ ] Claims Nos.:
because they are dependent claims and are not drafted in accordance with the second and third sentences of Rule 6.4(a).

Form PCT/ISA/210 (continuation of first sheet) (January 2015)

**INTERNATIONAL SEARCH REPORT**
**Information on patent family members**

International application No.
**PCT/CN2020/071353**

| Patent document cited in search report | Publication date (day/month/year) | Patent family member(s) | Publication date (day/month/year) |
|---|---|---|---|
| CN 107446048 A | 08 December 2017 | None | |
| CN 106632674 A | 10 May 2017 | CN 106632674 B | 16 November 2018 |
| | | WO 2017071625 A1 | 04 May 2017 |
| WO 2017058115 A1 | 06 April 2017 | EP 3356416 A1 | 08 August 2018 |
| | | JP 2018529368 A | 11 October 2018 |
| | | CN 108368175 A | 03 August 2018 |
| | | EP 3356416 A4 | 10 April 2019 |
| | | KR 20180083318 A | 20 July 2018 |
| | | BR 112018006547 A2 | 11 December 2018 |
| | | US 2018282412 A1 | 04 October 2018 |
| | | CA 3000638 A1 | 06 April 2017 |
| WO 2017019846 A1 | 02 February 2017 | JP 2019055948 A | 11 April 2019 |
| | | AU 2016298227 B9 | 31 October 2019 |
| | | SG 10201906059V A | 27 August 2019 |
| | | CN 108976300 A | 11 December 2018 |
| | | TW 201710292 A | 16 March 2017 |
| | | CN 107847574 A | 27 March 2018 |
| | | US 2019127467 A1 | 02 May 2019 |
| | | AU 2018214151 B2 | 31 October 2019 |
| | | CO 2018000867 A2 | 19 April 2018 |
| | | EP 3456346 A1 | 20 March 2019 |
| | | AU 2016298227 A8 | 08 March 2018 |
| | | MX 2018001227 A | 26 March 2018 |
| | | AU 2016298227 B2 | 03 October 2019 |
| | | WO 2017019846 A8 | 25 January 2018 |
| | | MX 2019001161 A | 04 July 2019 |
| | | AU 2020200054 A1 | 30 January 2020 |
| | | PH 12018500232 A1 | 29 August 2018 |
| | | PE 20181151 A1 | 17 July 2018 |
| | | CA 2993948 A1 | 02 February 2017 |
| | | IL 257216 D0 | 29 March 2018 |
| | | KR 20180034588 A | 04 April 2018 |
| | | EP 3328419 A1 | 06 June 2018 |
| | | KR 20180093127 A | 20 August 2018 |
| | | CL 2018000254 A1 | 20 July 2018 |
| | | AU 2016298227 A1 | 22 February 2018 |
| | | CR 20180062 A | 25 May 2018 |
| | | JP 2018524394 A | 30 August 2018 |
| | | AU 2016298227 A2 | 13 September 2018 |
| | | EP 3328419 A4 | 26 December 2018 |
| | | PE 11512018 A1 | 17 July 2018 |
| | | AU 2018214151 A1 | 30 August 2018 |
| | | EA 201890296 A1 | 31 August 2018 |
| WO 2016014688 A2 | 28 January 2016 | SG 11201700496 W A | 27 February 2017 |
| | | RU 2017105915 A | 26 August 2018 |
| | | US 2020031931 A1 | 30 January 2020 |
| | | EP 3171892 A2 | 31 May 2017 |
| | | KR 20170036016 A | 31 March 2017 |
| | | RU 2017105915 A3 | 30 January 2019 |
| | | BR 112017001385 A2 | 05 June 2018 |

Form PCT/ISA/210 (patent family annex) (January 2015)

**INTERNATIONAL SEARCH REPORT**
**Information on patent family members**

International application No.
**PCT/CN2020/071353**

| Patent document cited in search report | Publication date (day/month/year) | Patent family member(s) | Publication date (day/month/year) |
|---|---|---|---|
| | | AU 2015292678 A1 | 16 February 2017 |
| | | RU 2711141 C2 | 15 January 2020 |
| | | MX 2017000985 A | 08 March 2018 |
| | | WO 2016014688 A3 | 24 March 2016 |
| | | CN 106573052 A | 19 April 2017 |
| | | US 10428146 B2 | 01 October 2019 |
| | | US 2017267762 A1 | 21 September 2017 |
| | | SG 10201900571 Y A | 27 February 2019 |
| | | CA 2955788 A1 | 28 January 2016 |
| | | JP 2017530722 A | 19 October 2017 |
| | | EP 3171892 A4 | 17 January 2018 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 201910022548
- WO 2004004771 A
- WO 2006121168 A
- WO 2008156712 A
- WO 2010029435 A
- WO 2012145493 A
- WO 2015085847 A
- US 20170044260 A
- CN 104250302
- CN 105330740
- CN 105531288


### Non-patent literature cited in the description


- **PARDOLL DM.** The blockade of immune checkpoints in cancer immunotherapy. *Nat. Rev. Cancer,* 2012, vol. 12 (4), 252-264
- **ALSAAB et al.** PD-1 and PD-L1 Checkpoint Signaling Inhibition for Cancer Immunotherapy: Mechanism, Combinations, and Clinical Outcome. *Front. Pharmacol.,* vol. 8, 561
- **MARKHAM, A ; DUGGAN, S.** *Drugs,* 2018
- **TAN S ; ZHANG H ; CHAI Y et al.** An unexpected N-terminal loop in PD-1 dominates binding by nivolumab. *Nat Commun,* 2017, vol. 8, 14369
- **RONGHAO WANG ; RUNCHUAN CHEN ; RUNZHONG LIU.** *Journal of Xiamen University (Natural Science),* 2008, vol. 47 (2), 282-285